# EUROPEAN PATENT APPLICATION

(11) **EP 4 635 949 A1**
(43) Date of publication of application: **22.10.2025**
(21) Application number: 23902591.9
(22) Date of filing: 08.12.2023
(51) Int. Cl.: C07D 401/14, C07D 403/14, C07D 487/00, A61K 31/407, A61K 31/506, A61P 25/00, A61P 25/22, A61P 25/24

(54) **SUBSTITUTED TETRAHYDROCYCLOPENTYL[C]PYRROLE DERIVATIVE, PREPARATION METHOD, INTERMEDIATE AND USE THEREOF**

(30) Priority: 12.12.2022 CN 202211599295; 31.03.2023 CN 202310334144; 28.11.2023 CN 202311606327
(71) Applicant: Jiangsu NHWA Pharmaceutical Co., Ltd, Xuzhou, Jiangsu 221000 (CN)
(72) Inventor: DOU, Fei, Xuzhou, Jiangsu 221000 (CN); DONG, Yingying, Xuzhou, Jiangsu 221000 (CN); HU, Jumeng, Xuzhou, Jiangsu 221000 (CN); PAN, Yongkai, Xuzhou, Jiangsu 221000 (CN); DOU, Pengfei, Xuzhou, Jiangsu 221000 (CN); GE, Meng, Xuzhou, Jiangsu 221000 (CN); WANG, Dongli, Xuzhou, Jiangsu 221000 (CN); GUO, Qiang, Xuzhou, Jiangsu 221000 (CN); XU, Xiangqing, Xuzhou, Jiangsu 221000 (CN); WU, Guosheng, Xuzhou, Jiangsu 221000 (CN); CAI, Yingchun, Xuzhou, Jiangsu 221000 (CN)
(74) Representative: Lavoix
(86) International application number: PCT/CN2023/137347
(87) International publication number: WO 2024/125395

(57) **Abstract**

Provided are a compound as represented by general formula I or a pharmaceutically acceptable salt, stereoisomer or tautomer thereof, a composition containing the compound, a preparation method thereof, an intermediate thereof, and the use thereof in the field of medicine.

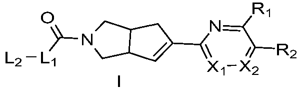

## Description

The present application claims the priority of Chinese application No. 202211599295.X, filed on December 12, 2022, entitled "A substituted tetrahydrocyclopentyl[c]pyrrole methyl ketone derivative, preparation method, and use thereof", and the priority of Chinese application No. 202310334144.X, filed on March 31, 2023, entitled "A substituted tetrahydrocyclopentyl[c]pyrrole derivative, preparation method, intermediate, and use thereof", and the priority of Chinese application No. 202311606327.9, filed on November 28, 2023, entitled "A substituted tetrahydrocyclopentyl[c]pyrrole derivative, preparation method, intermediate, and use thereof", the contents of which are incorporated herein by reference in their entirety.

### TECHNICAL FIELD

The present application belongs to the field of medicine, and particularly relates to a substituted tetrahydrocyclopentyl[c]pyrrole derivative or a pharmaceutically acceptable salt, a stereoisomer, a tautomer thereof, and a composition comprising the compound, a preparation method, an intermediate, and use thereof in the field of medicine.

### BACKGROUND ART

Orexin (also known as hypocretin or orexigenic peptide) exists in two types: orexin-A (hypocretin-1) and orexin-B (hypocretin-2). Orexin signaling is mediated by two receptors and two peptide agonists. Orexin A and orexin B bind to two-high affinity receptors (orexin receptor type 1 (OX1R or OX1) and orexin receptor type 2 (OX2R or OX2)). OX1R tends to prefer orexin A, while OX2R binds to both orexins with similar affinity.

Orexin receptors have important pathological significance and are associated with various diseases, such as sleep disorder, anxiety disorder, panic disorder, obsessive-compulsive disorder, affective neuropathy, depressive neuropathy, anxiety neuropathy, mood disorder, panic attack disorder, behavioral disorder, emotional disturbance, post-traumatic stress disorder, psychosis, schizophrenia, manic depression, delirium, dementia, drug dependence, addiction, cognitive disorder, Parkinson's disease, movement disorder, eating disorder, headache, migraine, pain, insomnia, depression, Alzheimer's disease, sleep apnea, etc.

There are already multiple drugs targeting OX1/2R in clinical stages or already on the market, such as Merck's Suvoraxant and Eisai's Lemborexant. However, existing drugs have antagonistic effects on both OX1R and OX2R receptors, and acting on OX1R can affect rapid eye movement sleep (REM, brain activity is the same as when awake) and non-rapid eye movement sleep (NREM), that is, sacrificing NREM time, prolonging REM time, thereby increasing the risk of drowsiness; and OX1R has no antidepressant effect.

Studies have shown that disorders of the sleep-wake cycle are likely targets for the activity of OX2R receptor regulators. Examples of disorders that can be treated by antagonists or other regulators that downregulate OX2R-mediated processes include insomnia, restless legs syndrome, jet lag (difficulty in sleeping), and sleep disorder secondary to neurological disorders such as manic disorder, schizophrenia, pain syndrome, etc. OX2R is selectively expressed in the tuberomammillary nucleus (TMN), paraventricular nucleus (PVN) of the hypothalamus, and nucleus accumbens (NAc). These brain regions are the main effect sites of orexin neurons in the lateral hypothalamus (LH), which are related to eating, sleep, depression, anxiety, drug addiction, and motivated behaviors, and are more effective in treating sleep disorders (Lu et al., 2020, Neurosci Bull, 4: 432-448).

OX2R antagonists can have antidepressant effects, and OX2R single receptor antagonists can also have sufficient therapeutic effects on insomnia. Therefore, selective OX2R antagonists can avoid various side effects such as drowsiness caused by the action on OX1R. At present, only Seltorexant developed by Janssen is in the clinical stage as an OX2R antagonist, and its main indications are major depressive disorder (MDD), primary and secondary insomnia, etc.

Selective OX2R antagonists have the potential to treat nervous system diseases such as insomnia, depression, and anxiety, and have huge clinical needs. Selective OX2R antagonists have good application prospects as drugs.

### CONTENTS OF THE INVENTION

The following is only a summary of some aspects of the present application and is not limited thereto. When there is a discrepancy between the disclosure of this specification and the referenced literature, the disclosure of this specification shall prevail.

The present application aims to provide a substituted tetrahydrocyclopentyl[c]pyrrole derivative or a pharmaceutically acceptable salt, a stereoisomer, a tautomer, and a pharmaceutical combination thereof, as well as a preparation method and an intermediate thereof, wherein the compound and the pharmaceutical composition can be used for the prevention or treatment of diseases related to orexin receptors.

On the one hand, the present application provides a compound represented by the following formula I or a pharmaceutically acceptable salt, a stereoisomer, or a tautomer thereof, 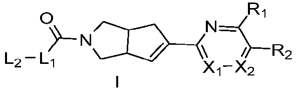 wherein
R₁ and R₂ are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, D, halogen, C₁-C₈ alkoxy, hydroxyl, nitro, and cyano;
or R₁ and R₂ together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, D, halogen, C₁-C₈ alkyl, C₁-C₈ alkoxy, hydroxyl, nitro, and cyano;
Li is selected from the group consisting of 6-14 membered aryl optionally substituted with one or more substituents R_{LA} and 5-14 membered heteroaryl optionally substituted with one or more substituents R_{LA}, preferably selected from the group consisting of 6-10 membered aryl optionally substituted with one or more substituents R_{LA} and 5-10 membered heteroaryl optionally substituted with one or more substituents R_{LA}, further preferably selected from the group consisting of phenyl optionally substituted with one or more substituents R_{LA} and 5-6 membered monocyclic heteroaryl optionally substituted with one or more substituents R_{LA}; the one or more substituents R_{LA} are independently selected from the group consisting of H, D, halogen, C₁-C₈ alkyl optionally substituted with one or more substituents R_{LB}, C₁-C₈ alkoxy optionally substituted with one or more substituents R_{LB}, cyano, C₂-C₈ alkynyl optionally substituted with one or more substituents R_{LB}, C₂-C₈ alkenyl optionally substituted with one or more substituents R_{LB}, hydroxyl, nitro, C₁-C₈ alkylthio optionally substituted with one or more substituents R_{LB}, C₃-C₈ cycloalkyl optionally substituted with one or more substituents R_{LB}, and OR₄, and the one or more substituents R_{LB} are selected from the group consisting of H, D, halogen, and hydroxyl;
R₄ is selected from the group consisting of C₃-C₈ cycloalkyl and 3-8 membered heterocyclyl, preferably C₃-C₆ cycloalkyl;
L₂ is selected from the group consisting of 6-14 membered aryl substituted with 0-4 Rb groups and 5-14 membered heteroaryl substituted with 0-4 Rb groups, preferably selected from the group consisting of 6-10 membered aryl substituted with 0-3 Rb groups and 5-10 membered monocyclic or bicyclic heteroaryl substituted with 0-3 Rb groups, further preferably selected from the group consisting of phenyl substituted with 0-3 Rb groups and 5-6 membered heteroaryl substituted with 0-3 Rb groups;
each Rb group is independently selected from the group consisting of H, D, optionally substituted C₁-C₈ alkyl, optionally substituted C₁-C₈ alkoxy, and halogen; and each substituent is independently selected from the group consisting of H, D, halogen, and hydroxyl;
X₁ is selected from the group consisting of N and CR₁₀;
R₁₀ is selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, D, halogen, C₁-C₈ alkoxy, hydroxyl, nitro, and cyano, preferably selected from the group consisting of H, D, halogen, and hydroxyl;
X₂ is selected from the group consisting of N and CR₁₁;
R₁₁ is selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano; and each substituent is independently selected from the group consisting of H, D, halogen, C₁-C₈ alkoxy, hydroxyl, nitro, and cyano;
and X₁ and X₂ are not N at the same time; and
when X₂ is CR₁₁ and X₁ is N, R₂ is not H or D.

In one aspect, the present application provides a pharmaceutical composition comprising a therapeutically effective amount of a compound represented by formula I as described above or a pharmaceutically acceptable salt, a stereoisomer, or a tautomer thereof, optionally further comprising a pharmaceutically acceptable excipient, carrier, adjuvant, solvent or a combination thereof.

In another aspect, the present application provides a use of a compound as shown in Formula I or a pharmaceutically acceptable salt, a stereoisomer, a tautomer thereof, or a pharmaceutical composition thereof in the preparation of a medicament for treating a disease related to orexin receptors or a method for treating a disease related to orexin receptors in a subject, the method comprising administering a therapeutically effective amount of the compound of the present application to a subject in need thereof, including the compound or specific compound represented by Formula I or a pharmaceutically acceptable salt, a stereoisomer, a tautomer thereof, or a pharmaceutical composition thereof.

In one embodiment, the disease related to orexins is selected from the group consisting of: sleep disorder, anxiety disorder, panic disorder, obsessive-compulsive disorder, affective neuropathy, depressive neuropathy, anxiety neuropathy, mood disorder, panic attack disorder, behavioral disorder, emotional disturbance, post-traumatic stress disorder, psychosis, schizophrenia, manic depression, delirium, dementia, drug dependence, addiction, cognitive disorder, Parkinson's disease, movement disorder, eating disorder, headache, migraine, pain, insomnia, depression, Alzheimer's disease, and sleep apnea; preferably selected from the group consisting of: insomnia, depression, sleep disorder; further preferably selected from the group consisting of: major depressive disorder, primary and secondary insomnia or depression accompanied by insomnia.

In one embodiment, the subject is a mammal. In one embodiment, the subject is a human.

In another aspect, the present application provides a method for preparing a compound represented by formula I, comprising the following steps: reacting a compound represented by formula I-d with a compound represented by formula I-f to obtain a target compound represented by formula I: 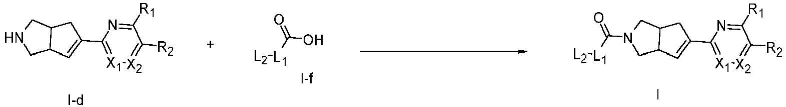 wherein R₁, R₂, X₁, X₂, L₁, and L₂ are as defined above, and preferably the compound represented by formula I-d is a compound represented by formula I-dA and formula I-dB, 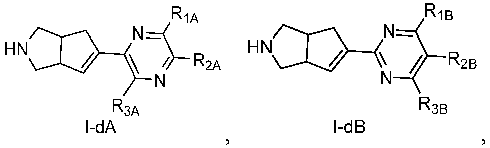 R_{1A}, R_{2A}, and R_{3A} are as defined above; R_{1B}, R_{2B}, and R_{3B} are as defined above.

In another aspect, the present application provides an intermediate as shown in the compound of formula I-d, 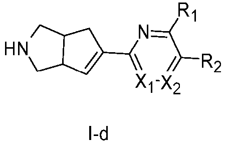 wherein R₁, R₂, X₁, and X₂ are as defined above, and preferably the compound represented by formula I-d is a compound represented by formula I-dA and formula I-dB, 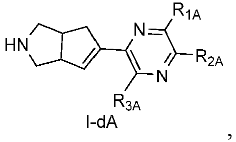 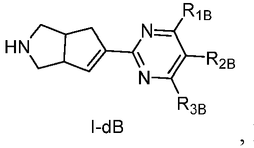 R_{1A}, R_{2A}, and R_{3A} are as defined above; R_{1B}, R_{2B}, and R_{3B} are as defined above.

### DETAILED DESCRIPTION

Unless otherwise specified or there is an obvious conflict in the context, all technical and scientific terms used herein have the same meanings as those generally understood by those of ordinary skill in the art to which the application belongs. If there is a conflict, the definitions provided in the present application shall prevail. When a trade name appears herein, it is intended to refer to the corresponding commercial product or its active ingredient. All patents, published patent applications and publications cited herein are incorporated herein by reference.

### General Terms and Definitions

The term "optional" or "optionally" means that the event or situation described subsequently may but may not necessarily occur, and the description includes instances where the event or situation occurs as well as instances where it does not occur.

The term "optionally substituted" can be used interchangeably with the term "substituted or unsubstituted", i.e., the structure or group is unsubstituted or substituted with one or more substituents described herein, wherein the substitution occurs at any reasonable position allowed by valence on the given structure or group.

Unless otherwise specified, as used herein, the connection point of a substituent may be from any suitable position of the substituent. When a bond of a substituent is shown to pass through a bond connecting two atoms in a ring, then such substituent may be bonded to any one of the ring-forming atoms in the substitutable ring.

In general, the term "substituted" means that one or more hydrogen atoms in a given structure or group are substituted by a specific substituent. Unless otherwise indicated, substitution with one substituent may occur at each reasonable substitutable position of a group. When more than one position in a given structural formula can be substituted with one or more specific substituents selected, substitution with the substituents may occur identically or differently at each reasonable position in the structural formula.

In addition, it should be noted that, unless explicitly stated otherwise, the description "each independently" used in the present application should be understood in a broad sense, and it may mean that specific items expressed by the same symbol in different groups do not affect each other, or that specific items expressed by the same symbol in the same group do not affect each other.

When the lower and upper limits of a range of numerical values are disclosed, any numerical value falling within the range and any included range are specifically disclosed. In particular, each range of values disclosed herein is to be understood as indicating each numerical value and range encompassed within a relatively broad range. When any variable (e.g., R), as well as labeled variables (e.g., R₁, R₂, R₃, R₄, R₅, R₆, R₇, etc.), occurs more than once in a composition or structure of a compound, the variable is independently defined in each case at each occurrence. For example, if a group is substituted with 0, 1, 2, or 3 of R substituents, the group may optionally be substituted with up to three R substituents, and the options for each R substituent in each case are independent of each other.

In each part of the present specification, the substituents of the compounds disclosed in the present application are disclosed according to group types or ranges. It is specifically noted that the present application includes each independent secondary combination of each member of these group types and ranges. For example, the expression m-n used herein refers to the range of m to n as well as to sub-ranges consisting of the point values therein and the point values.

The term "alkyl" refers to a linear or branched saturated aliphatic hydrocarbon group consisting of carbon atoms and hydrogen atoms, which is connected to the rest of the molecule through a single bond. "Alkyl" may have 1-8 carbon atoms, i.e., "C₁-C₈ alkyl", e.g., C₁₋₄ alkyl, C₁₋₃ alkyl, C₁₋₂ alkyl, C₃ alkyl, C₄ alkyl, C₈ alkyl, C₁₋₈ alkyl, or C₃₋₈ alkyl. It may also have 1 to 3 carbon atoms, i.e., "C₁-C₃ alkyl", e.g., C₁₋₃ alkyl, C₁₋₂ alkyl, or C₃ alkyl. The term "C₁-C₅ alkyl" particularly refers to independently disclosed methyl, ethyl, C₃ alkyl, C₄ alkyl, or C₅ alkyl. Examples of alkyl groups include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, etc. For example, the expression "C₁-C₈" or "C₁₋₈" encompasses a range of 1-8 carbon atoms and should be understood as also encompassing any sub-range and each point value therein, e.g., C₁-C₅, C₃-C₄, C₂-C₆, C₃-C₆, C₄-C₆, C₄-C₇, C₄-C₈, C₂-C₄, and the like, as well as C₁, C₂, C₃, C₄, C₅, C₆, C₇, C₈, etc. As another example, the expression "C₁-C₅" or "C₁₋₅" encompasses a range of 1-5 carbon atoms and should be understood as also encompassing any sub-range and each point value therein, e.g., C₂-C₅, C₃-C₄, C₁-C₂, C₁-C₃, C₁-C₄, C₁-C₅, and the like, as well as C₁, C₂, C₃, C₄, C₅, etc. As another example, the expression "C₂-C₅" or "C₂₋₅" encompasses a range of 2-5 carbon atoms and should be understood as also encompassing any sub-range and each point value therein, e.g., C₂-C₅, C₃-C₄, C₂-C₃, C₂-C₄, C₃-C₅, C₄-C₅, and the like, as well as C₂, C₃, C₄, C₅, etc. As another example, the expression "C₁-C₈" or "C₁₋₈" encompasses a range of 1-8 carbon atoms and should be understood as also encompassing any sub-range and each point value therein, e.g., C₂-C₅, C₃-C₄, C₂-C₆, C₃-C₆, C₄-C₆, C₄-C₇, C₄-C₈, C₂-C₄, and the like, as well as C₁, C₂, C₃, C₄, C₅, C₆, C₇, C₈, etc. As another example, the expression "three to eight membered" should be understood as encompassing any sub-range and each point value therein, e.g., three to five membered, three to six membered, three to seven membered, three to eight membered, four to five membered, four to six membered, four to seven membered, four to eight membered, five to seven membered, five to eight membered, six to seven membered, six to eight membered, and the like, as well as three membered, four membered, five membered, six membered, seven membered, eight membered, etc. Other similar expressions herein should also be understood in a similar manner.

The term "one or more" or the similar expression "at least one" may indicate, for example, 1, 2, 3, 4, 5, 6, 7, 8, 9, 10 or more.

The term "selected from the group consisting of..." refers to independent selection of one or more elements from the group listed afterward, and may include a combination of two or more elements.

The terms "comprise", "comprises", "comprising", "include", "includes", and "including" are open-ended, i.e., including what is indicated in the present application, but not excluding other aspects.

When it is stated that each carbon atom in a group may optionally be replaced by a heteroatom, the proviso is that the normal valences of all atoms in the group in the present case are not exceeded, and that a stable compound is formed.

The term "heteroatom" means one or more oxygen (O), sulfur (S), or nitrogen (N) atoms, including any form of nitrogen (N) and sulfur (S) in an oxidation state; forms of primary, secondary, and tertiary amines and quaternary ammonium salts; or forms in which hydrogen on a nitrogen atom in a heterocycle is substituted, such as: N, NH, and NR.

The term "aryl" refers to a 6-14 membered all-carbon monocyclic or fused polycyclic (i.e., rings that share adjacent carbon atom pairs) group with a conjugated electron system. The term "aryl" can be used interchangeably with the term "aromatic ring". Examples of aryl groups include 6-14 membered aryl, 6-10 membered aryl, specifically including phenyl, naphthyl, etc. The aryl group is optionally substituted with one or more substituents described in the present application.

The term "heteroaryl" refers to a heteroaromatic system containing 1 to 4 heteroatoms, and 5 to 14 ring atoms, wherein the heteroatoms are one or more selected from the group consisting of: oxygen, sulfur, and nitrogen. The term "heteroaryl" can be used interchangeably with the term "heteroaromatic ring" or "heteroaromatic compound". Examples of heteroaryl groups include 5-14 membered heteroaryl, 5-10 membered heteroaryl, 5-10 membered monocyclic or polycyclic heteroaryl, 5-6 membered monocyclic heteroaryl, specifically including pyrimidinyl, pyridyl, pyrazinyl, thiazolyl, oxazolyl, imidazolyl, pyrazolyl, pyrrolyl, 1,2,3-triazolyl, thiadiazolyl, thienyl, furyl, pyridazinyl, triazinyl, oxadiazolyl, isoxazolyl, pyranyl, 1,3,4-triazolyl, furanopyrimidinyl, thienopyrimidinyl, pyrrolopyridyl, pyranopyrimidinyl, benzothiazolyl, benzoxazolyl, thienopyrimidinyl, indolyl, etc. The heteroaryl group is optionally substituted with one or more substituents described herein.

The terms "heterocyclic ring" and "heterocyclyl" can be used interchangeably to refer to monovalent or multivalent monocyclic, bicyclic, or tricyclic systems comprising 3-14 ring atoms, or 3-8 ring atoms, or 5-6 ring atoms, wherein one or more atoms on the ring are independently replaced by heteroatoms, which have the meaning described in the present application, and the ring can be fully saturated or comprise one or more unsaturation degrees. Unless otherwise specified, the -CH₂- group on the heterocyclyl can be optionally replaced by -C(=O)-. The sulfur atom of the ring can be optionally oxidized to S-oxide. The nitrogen atom of the ring can be optionally oxidized to N-oxide. Examples of heterocyclyls include 3-14 membered heterocyclyl, 3-8 membered heterocyclyl, 5-10 membered heterocyclyl, 5-10 membered monocyclic or bicyclic heterocyclyl, specifically including: oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuryl, dihydrofuryl, tetrahydrothiophenyl, dihydrothiophenyl, 1,3-dioxolanyl, dithiolanyl, tetrahydropyranyl, dihydropyranyl, 2H-pyranyl, 4H-pyranyl, tetrahydrothiopyranyl, dihydrothiopyranyl, piperidinyl, morpholinyl, thiomorpholinyl, piperazinyl, cyclopentylpyrimidinyl, dihydrofuranopyrimidinyl, dihydropyranopyrimidinyl, dihydropyridyl, 2-pyridonyl, 2-oxopyrrolidinyl, oxo-1,3-thiazolidinyl, etc. Examples of heterocyclyls in which the -CH₂- group is replaced by -C(=O)- include, but are not limited to, 2-pyridone, 2-oxopyrrolidinyl, oxo-1,3-thiazolidinyl, 2-piperidonyl, 3,5-dioxopiperidinyl, and pyrimidinedionyl. Examples of heterocyclyl groups in which the sulfur atom is oxidized include, but are not limited to, sulfolanyl, 1,1-dioxothiomorpholinyl. The heterocyclyl groups may be optionally substituted with one or more substituents described herein.

The term "alkenyl" refers to a general term for hydrocarbons comprising a carbon-carbon double bond in molecules, and is an unsaturated aliphatic hydrocarbon. For example, alkenyl (CH₂=CH-). The "alkenyl" may have 2-8 carbon atoms, i.e., "C₂-C₈" alkenyl, e.g., C₂₋₈ alkenyl, C₂₋₄ alkenyl, C₂₋₅ alkenyl, C₃ alkenyl, C₄ alkenyl, C₆ alkenyl, C₂₋₆ alkenyl, C₃₋₈ alkenyl, C₃₋₆ alkenyl, etc. It can also have 2-5 carbon atoms, i.e., "C₂-C₅ alkynyl", e.g., C₂₋₅ alkynyl, C₂₋₃ alkynyl, C₃ alkynyl, C₅ alkynyl, etc. Examples of alkenyl groups include, but are not limited to ethenyl (CH₂=CH-), propenyl (-CH=CH-CH₃), butenyl (-CH=CH-CH₂-CH₃, -CH₂-CH=CH-CH₃), pentenyl (-CH=CH-CH₂-CH₂-CH₃, -CH₂-CH₂-CH=CH-CH₃, -CH(CH₃)-CH=CH-CH₃), hexenyl, heptenyl, octenyl, etc.

The term "alkynyl" refers to a general term for hydrocarbons comprising a carbon-carbon triple bonds in molecules, and is an unsaturated aliphatic hydrocarbon. For example, ethynyl (-C≡CH). The "alkyne" may have 2-8 carbon atoms, i.e., "C₂-C₈" alkynyl, e.g., C₂₋₈ alkynyl, C₂₋₄ alkynyl, C₂₋₅ alkynyl, C₃ alkynyl, C₄ alkynyl, C₆ alkynyl, C₂₋₆ alkynyl, C₃₋₈ alkynyl, C₃₋₆ alkynyl, etc. It can also have 2-5 carbon atoms, i.e., "C₂-C₅ alkynyl", e.g., C₂₋₅ alkynyl, C₂₋₃ alkynyl, C₃ alkynyl, C₅ alkynyl, etc. Examples of alkynyl groups include, but are not limited to ethynyl, propynyl, butynyl, pentynyl, hexynyl, heptynyl, octynyl, etc.

The term "hydrogen (H)" refers to a single hydrogen atom, which can be connected to other groups such as oxygen atoms to form a hydroxyl group.

The term "deuterium (D or 2H)" is a stable isotope of hydrogen, which exists at a natural abundance of 0.015 mol%. The term "deuterated" refers to the replacement of one or more hydrogen atoms H by D in a group or compound.

The term "halogen" or "halo" should be understood to mean fluorine (F), chlorine (Cl), bromine (Br), or iodine (I), preferably a fluorine, chlorine, or bromine atom, and more preferably a fluorine atom.

The term "alkoxy" means that an alkyl group is connected to the rest of a molecule through an oxygen atom, wherein the alkyl group has the meaning described in the present application. In one embodiment, the alkoxy group comprises 1 to 8 carbon atoms. In one embodiment, the alkoxy group comprises 1 to 5 carbon atoms; in another embodiment, the alkoxy group comprises 1 to 3 carbon atoms. The alkoxy group may optionally be substituted with one or more substituents described in the present application. Examples of alkoxy groups include, but are not limited to, methoxy (MeO, -OCH₃), ethoxy (EtO, -OCH₂CH₃), 1-propoxy (n-PrO, n-propoxy, -OCH₂CH₂CH₃), 2-propoxy (i-PrO, i-propoxy, -OCH(CH₃)₂), 1-butoxy (n-BuO, n-butoxy, -OCH₂CH₂CH₂CH₃), 2-methyl-l-propoxy (i-BuO, i-butoxy, -OCH₂CH(CH₃)₂), 2-butoxy (s-BuO, s-butoxy, -OCH(CH₃)CH₂CH₃), 2-methyl-2-propoxy (t-BuO, t-butoxy, -OC(CH₃)₃), etc.

The term "alkylthio" means that an alkyl group is connected to the rest of the molecule through a sulfur atom, wherein the alkyl group has the meaning described in the present application. In one embodiment, the alkylthio group comprises 1-8 carbon atoms. In one embodiment, the alkylthio group comprises 1-5 carbon atoms. In another embodiment, the alkylthio group comprises 1-3 carbon atoms. The alkylthio group can be optionally substituted with one or more substituents described in the present application. Examples of alkylthio groups include, but are not limited to, methylthio (-SCH₃), ethylthio (-SCH₂CH₃), propylthio (-SCH₂CH₂CH₃, -SCH(CH₃)₂), butylthio (-SCH₂CH₂CH₂CH₃, -SCH₂CH₂CH(CH₃)₂, -SCH(CH₃)CH₂CH₃, -SC(CH₃)₃), etc.

The term "cycloalkyl" refers to a cyclic hydrocarbyl or cyclic alkenyl consisting of carbon atoms and hydrogen atoms, preferably comprising 1 or 2 rings. The cycloalkyl can be a monocyclic ring, a fused polycyclic ring, a bridged ring or a spirocyclic structure. Cycloalkyl can have 3-8 carbon atoms, i.e., "C₃-C₈ alkyl", or 3-6 carbon atoms, i.e., "C₃-C₆ alkyl", e.g., C₆ cycloalkyl, C₅ cycloalkyl, C₄ cycloalkyl, C₃ cycloalkyl. Examples of cycloalkyl groups include C₃-C₈ cycloalkyl, C₃-C₆ cycloalkyl, specifically including cyclopropyl, cyclobutyl, cyclopentyl, cyclohexyl, cyclobutenyl, cyclopentenyl, cyclohexenyl, etc. The term also encompasses situations in which the C atom can be substituted with oxo (=O).

The terms "hydroxyalkyl" and "hydroxy substituted alkyl" can be used interchangeably, referring to an alkyl group substituted with one or more hydroxyl groups. Examples include, but are not limited to, hydroxymethyl (-CH₂OH), hydroxyethyl (-CH₂CH₂OH, -CHOHCH₃), hydroxypropyl (-CH₂CH₂CH₂OH, -CH₂CHOHCH₃, -CHOHCH₂CH₃, -COHCH₃CH₃), hydroxybutyl (-CH₂CH₂CH₂CH₂OH, -CH₂CH₂CHOHCH₃, -CHOHCH₂CH₂CH₃, -COHCH₃CH₂CH₃), and the above hydroxyl substitutions can be mono- or poly-substituted.

The terms "hydroxyalkoxy" and "hydroxy substituted alkoxy" can be used interchangeably, referring to an alkoxy group substituted with one or more hydroxyl groups.

The terms "haloalkyl" and "halogen substituted alkyl" can be used interchangeably, referring to an alkyl group substituted with one or more halogens. Examples include, but are not limited to, halomethyl, haloethyl, halopropyl, halobutyl, and halopentyl, which may be monohalogenated, dihalogenated, or trihalogenated; when it is trihalogenated methyl, it may further preferably be trifluoromethyl.

The terms "haloalkoxy" and "halogen substituted alkoxy" can be used interchangeably, referring to an alkoxy group substituted with one or more halogens. Examples include, but are not limited to, halomethoxy, haloethoxy, halopropoxy, halobutoxy, and halopentoxy, which may be monohalogenated, dihalogenated, or trihalogenated.

The terms "haloalkenyl" and "halogen substituted alkenyl" can be used interchangeably, referring to an alkenyl group substituted with one or more halogens. Examples include, but are not limited to, haloalkenyl, halopropenyl, halobutenyl, and halopentenyl, which may be monohalogenated, dihalogenated, or trihalogenated.

The terms "haloalkynyl" and "halogen substituted alkynyl" can be used interchangeably, referring to an alkynyl group substituted with one or more halogens. Examples include, but are not limited to, haloethynyl, halopropynyl, halobutynyl, and halopentynyl, which may be monohalogenated, dihalogenated, or trihalogenated.

The term "hydroxyl" refers to the -OH group.

"R₁ and R₂ together with the carbon atom to which they are connected form a 3-8 membered heterocyclyl, a 5-8 membered heteroaryl or a C₃-C₈ cycloalkyl" or "R₁ and R₂ together form a 3-8 membered heterocyclyl, a 5-8 membered heteroaryl or a C₃-C₈ cycloalkyl" means that R₁ and R₂ together with the carbon atom to which they are connected form a fused polycyclic group.

Examples include, but are not limited to, 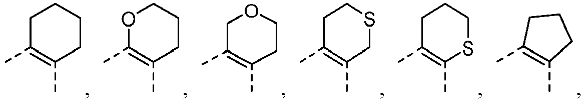 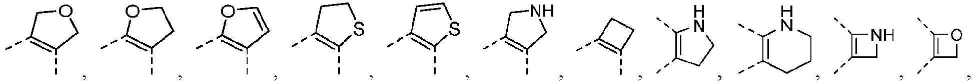 and 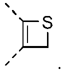 The group is optionally substituted with one or more substituents.

The term "stereoisomer" refers to a compound having the same chemical structure, but having a different arrangement of atoms or groups in space. Stereoisomers include enantiomers, diastereomers, conformational isomers (rotamers), geometric isomers (cis/trans) isomers, atropisomers, etc.

The term "tautomer" refers to a structural isomer having different energies that are interconvertible via a low energy barrier. If tautomerism is possible (e.g., in a solution), a chemical equilibrium of tautomers can be achieved. For example, proton tautomers (also known as prototropic tautomers) include interconversion via migration of a proton, such as keto-enol isomerization and imine-enamine isomerization.

The term "pharmaceutically acceptable salt" refers to an organic salt or an inorganic salt of the compound of the present application.

The term "pharmaceutically acceptable carrier" refers to those substances that do not have a significant irritating effect on organisms and do not impair the biological activity and properties of the active compound. The "pharmaceutically acceptable carrier" includes, but is not limited to, a glidant, a sweetener, a diluent, a preservative, a dye/colorant, a flavoring agent, a surfactant, a wetting agent, a dispersant, a disintegrant, a stabilizer, a solvent, or an emulsifier.

The following detailed description of the present invention is intended to illustrate non-limiting embodiments and to enable others skilled in the art to more fully understand the technical solutions of the present application, its principles, and its practical applications, so that others skilled in the art may modify and implement the present application in various forms to allow it to be optimally adapted to the requirements of particular use.

### Compound of formula I

The present application provides a compound of formula I or a pharmaceutically acceptable salt, a stereoisomer, or a tautomer thereof, 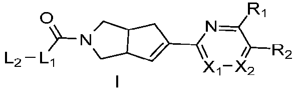 wherein
R₁ and R₂ are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, D, halogen, C₁-C₈ alkoxy, hydroxyl, nitro, and cyano;
or R₁ and R₂ together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, D, halogen, C₁-C₈ alkyl, C₁-C₈ alkoxy, hydroxyl, nitro, and cyano;
L₁ is selected from the group consisting of 6-14 membered aryl optionally substituted with one or more substituents R_{LA} and 5-14 membered heteroaryl optionally substituted with one or more substituents R_{LA}, preferably selected from the group consisting of 6-10 membered aryl optionally substituted with one or more substituents R_{LA} and 5-10 membered heteroaryl optionally substituted with one or more substituents R_{LA}, further preferably selected from the group consisting of phenyl optionally substituted with one or more substituents R_{LA} and 5-6 membered monocyclic heteroaryl optionally substituted with one or more substituents R_{LA}; the one or more substituents R_{LA} are independently selected from the group consisting of H, D, halogen, C₁-C₈ alkyl optionally substituted with one or more substituents R_{LB}, C₁-C₈ alkoxy optionally substituted with one or more substituents R_{LB}, cyano, C₂-C₈ alkynyl optionally substituted with one or more substituents R_{LB}, C₂-C₈ alkenyl optionally substituted with one or more substituents R_{LB}, hydroxyl, nitro, C₁-C₈ alkylthio optionally substituted with one or more substituents R_{LB}, C₃-C₈ cycloalkyl optionally substituted with one or more substituents R_{LB}, or OR₄, the one or more substituents R_{LB} are selected from the group consisting of H, D, halogen and hydroxyl;
R₄ is selected from the group consisting of C₃-C₈ cycloalkyl and 3-8 membered heterocyclyl, preferably C₃-C₆ cycloalkyl;
L₂ is selected from the group consisting of 6-14 membered aryl substituted with 0-4 Rb groups and 5-14 membered heteroaryl substituted with 0-4 Rb groups, preferably selected from the group consisting of 6-10 membered aryl substituted with 0-3 Rb groups and 5-10 membered monocyclic or bicyclic heteroaryl substituted with 0-3 Rb groups, further preferably selected from the group consisting of phenyl substituted with 0-3 Rb groups and 5-6 membered heteroaryl substituted with 0-3 Rb groups;
each Rb group is independently selected from the group consisting of H, D, optionally substituted C₁-C₈ alkyl, optionally substituted C₁-C₈ alkoxy, and halogen; and each substituent is independently selected from the group consisting of H, D, halogen and hydroxyl;
X₁ is selected from the group consisting of N and CR₁₀;
R₁₀ is selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, D, halogen, C₁-C₈ alkoxy, hydroxyl, nitro, and cyano, preferably selected from the group consisting of H, D, halogen and hydroxyl;
X₂ is selected from the group consisting of N and CR₁₁;
R₁₁ is selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano; and each substituent is independently selected from the group consisting of H, D, halogen, C₁-C₈ alkoxy, hydroxyl, nitro, and cyano;
and X₁ and X₂ are not N at the same time; and
when X₂ is CR₁₁ and X₁ is N, R₂ is not H or D.

In one embodiment, the heteroaryl is selected from the group consisting of pyrimidinyl, pyridyl, pyrazinyl, thiazolyl, oxazolyl, imidazolyl, pyrazolyl, pyrrolyl, 1,2,3-triazolyl, thiadiazolyl, thienyl, furyl, 1,3,4-triazolyl, and pyranyl. In one preferred embodiment, the heteroaryl is selected from the group consisting of imidazolyl, pyrazolyl, thienyl, furyl, 1,2,3-triazolyl, pyranyl, and thiazolyl. In one more preferred embodiment, the heteroaryl is selected from the group consisting of thienyl, 1,2,3-triazolyl, pyranyl, thiazolyl, and furyl.

In one embodiment, the heterocyclyl is preferably selected from the group consisting of oxiranyl, tetrahydrothiopyranyl, tetrahydrofuryl, dihydrofuryl, tetrahydropyranyl, dihydropyranyl, dihydrothiopyranyl, tetrahydrothiophenyl, dihydrothiophenyl, azetidinyl, oxetanyl, thietanyl, piperidinyl, and pyrrolidinyl. In one preferred embodiment, the heterocyclyl is selected from the group consisting of tetrahydrofuryl, dihydrofuryl, tetrahydropyranyl, and dihydropyranyl. In one particularly preferred embodiment, the heterocyclyl is selected from the group consisting of tetrahydrofuryl, dihydrofuryl, tetrahydropyranyl, and dihydropyranyl.

In one embodiment, the C₁-C₈ alkyl is selected from the group consisting of C₁-C₈ alkyl and C₁-C₃ alkyl. In one specific embodiment, the C₁-C₈ alkyl and C₁-C₈ alkyl are each independently selected from the group consisting of methyl, ethyl, propyl, isopropyl, butyl, isobutyl, pentyl, and isopentyl. In one more specific embodiment, the C₁-C₈ alkyl, C₁-C₈ alkyl, and C₁-C₃ alkyl are each independently selected from the group consisting of methyl, ethyl, propyl, and isopropyl.

In one embodiment, the propyl includes, but is not limited to, n-propyl (n-Pr, -CH₂CH₂CH₃) or isopropyl (i-Pr, -CH(CH₃)₂). The butyl includes, but is not limited to, n-butyl (n-Bu, -CH₂CH₂CH₂CH₃), isobutyl (i-Bu, -CH₂CH(CH₃)₂), sec-butyl (s-Bu, -CH(CH₃)CH₂CH₃), or tert-butyl (t-Bu, -C(CH₃)₃). The pentyl includes, but is not limited to, n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), or 2-methyl-1-butyl (-CH₂CH(CH₃)CH₂CH₃).

In one embodiment, the C₁-C₈ alkoxy is selected from the group consisting of C₁-C₈ alkoxy and C₁-C₃ alkoxy. In one specific embodiment, the C₁-C₈ alkoxy and the C₁-C₈ alkoxy are each independently selected from the group consisting of methoxy, ethoxy, propoxy, butoxy, and pentyloxy. In one more specific embodiment, the C₁-C₈ alkoxy, the C₁-C₈ alkoxy, and the C₁-C₃ alkoxy are each independently selected from the group consisting of methoxy, ethoxy, and propoxy.

In one embodiment, the C₃-C₈ cycloalkyl is preferably C₃-C₆ cycloalkyl. In one preferred embodiment, the C₃-C₈ cycloalkyl and C₃-C₆ cycloalkyl are each independently selected from the group consisting of cyclopropyl, cyclobutyl, cyclobutenyl, cyclopentyl, cyclohexyl, cyclopentenyl, and cyclohexenyl. In one more preferred embodiment, the C₃-C₈ cycloalkyl and C₃-C₆ cycloalkyl are each independently selected from the group consisting of cyclopropyl, cyclopentyl, cyclohexyl, cyclopentenyl, and cyclohexenyl. In one particularly preferred embodiment, the C₃-C₈ cycloalkyl and C₃-C₆ cycloalkyl are each independently selected from the group consisting of cyclopentyl and cyclopentenyl.

In one embodiment, the halogen is selected from the group consisting of fluorine, chlorine, bromine, and iodine. In one preferred embodiment, the halogen is selected from the group consisting of fluorine, chlorine, and bromine. In one more preferred embodiment, the halogen is selected from the group consisting of fluorine and chlorine. In one particularly preferred embodiment, the halogen is fluorine.

In one embodiment, the compound of formula I is represented by formula I-A, 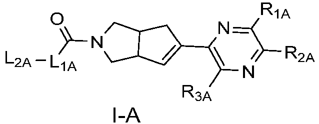
wherein R_{1A}, R_{2A}, and R_{3A} are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, halogen, C₁-C₈ alkoxy, hydroxyl, nitro, and cyano, preferably selected from the group consisting of H, D, halogen and hydroxyl;
or R_{1A} and R_{2A} together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, halogen, C₁-C₈ alkyl, C₁-C₈ alkoxy, hydroxyl, nitro, and cyano;
L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; the one or more substituents are independently selected from the group consisting of H, D, halogen, optionally substituted C₁-C₈ alkyl, optionally substituted C₁-C₈ alkoxy, cyano, optionally substituted C₂-C₈ alkynyl, optionally substituted C₂-C₈ alkenyl, hydroxyl, nitro, optionally substituted C₁-C₈ alkylthio, optionally substituted C₃-C₈ cycloalkyl, and OR_{4A}, preferably independently selected from the group consisting of H, halogen, optionally substituted C₁-C₈ alkyl, optionally substituted C₁-C₈ alkoxy, hydroxyl, optionally substituted C₃-C₆ cycloalkyl, and OR_{4A}, further preferably independently selected from the group consisting of H, halogen, optionally substituted C₁-C₈ alkyl, optionally substituted C₁-C₈ alkoxy, optionally substituted C₃-C₆ cycloalkyl, and OR_{4A}, and each substituent is selected from the group consisting of H, D, halogen and hydroxyl, and the substituent is preferably selected from the group consisting of D, halogen, and hydroxyl;
R_{4A} is selected from the group consisting of C₃-C₈ cycloalkyl and 3-8 membered heterocyclyl, preferably C₃-C₆ cycloalkyl;
L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, further preferably selected from the group consisting of phenyl substituted with 0-3 Rbbb groups and 5-6 membered heteroaryl substituted with 0-3 Rbbb groups;
each Rbbb group is independently selected from the group consisting of H, D, optionally substituted C₁-C₈ alkyl, optionally substituted C₁-C₈ alkoxy, and halogen; and each substituent is independently selected from the group consisting of H, D, halogen, and hydroxyl.

In one embodiment, the compound of formula I is represented by formula I-A, 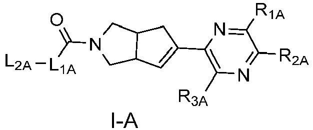
wherein R_{1A}, R_{2A}, and R_{3A} are independently selected from the group consisting of **H,** optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of **H,** optionally substituted C₁-C₈ alkyl, optionally substituted C₁-C₈ alkoxy, optionally substituted C₃-C₈ cycloalkyl, and halogen; and each substituent is independently selected from the group consisting of **H,** halogen, C₁-C₈ alkoxy, hydroxyl, nitro, and cyano, preferably selected from the group consisting of **H,** halogen, and hydroxyl;
or R_{1A} and R_{2A} together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, halogen, C₁-C₈ alkyl, C₁-C₈ alkoxy, hydroxyl, nitro, and cyano;
L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; the one or more substituents are independently selected from the group consisting of **H,** halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}, preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₆ cycloalkyl, and OR_{4A}, further preferably independently selected from the group consisting of H, halogen, C₁-C₈ alkyl, C₁-C₈ alkoxy, C₁-C₈ haloalkyl, C₃-C₆ cycloalkyl, and OR_{4A};
R_{4A} is selected from the group consisting of C₃-C₈ cycloalkyl and 3-8 membered heterocyclyl, preferably C₃-C₆ cycloalkyl;
L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, further preferably selected from the group consisting of phenyl substituted with 0-3 Rbbb groups and 5-6 membered heteroaryl substituted with 0-3 Rbbb groups;
each Rbbb group is independently selected from the group consisting of H, optionally substituted C₁-C₈ alkyl, optionally substituted C₁-C₈ alkoxy, and halogen; and each substituent is independently selected from the group consisting of H, halogen, and hydroxyl.

In one embodiment, R_{1A}, R_{2A}, and R_{3A} are independently selected from the group consisting of H, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, halogen, C₁-C₈ alkoxy, hydroxyl, nitro, and cyano, preferably selected from the group consisting of H, halogen, and hydroxyl. In one embodiment, the C₃-C₈ cycloalkyl is selected from the group consisting of cyclopropyl, cyclobutyl, cyclopentyl, and cyclohexyl, further preferably cyclopropyl;

In one embodiment, R_{1A} and R_{2A} together with the carbon atom to which they are connected form a 3-8 membered heterocyclyl, a 5-8 membered heteroaryl, or a C₃-C₈ cycloalkyl optionally substituted with one or more substituents, the substituents are independently selected from the group consisting of H, halogen, C₁-C₈ alkyl, C₁-C₈ alkoxy, hydroxyl, nitro, and cyano. In one embodiment, the 3-8 membered heterocyclyl is selected from the group consisting of oxiranyl, tetrahydrofuryl, dihydrofuryl, tetrahydropyranyl, dihydropyranyl, tetrahydrothiopyranyl, dihydrothiopyranyl, tetrahydrothiophenyl, dihydrothiophenyl, azetidinyl, oxetanyl, thietanyl, piperidinyl, and pyrrolidinyl. In one preferred embodiment, the 3-8 membered heterocyclyl is selected from the group consisting of tetrahydropyranyl and dihydropyranyl. In one embodiment, the 5-8 membered heteroaryl is selected from the group consisting of furyl, pyranyl, and thienyl. In one embodiment, the C₃-C₈ cycloalkyl is selected from the group consisting of cyclopropyl, cyclobutyl, cyclopentyl, cyclohexyl, cyclopentenyl, cyclobutenyl, and cyclohexenyl. In one preferred embodiment, the C₃-C₈ cycloalkyl is selected from the group consisting of cyclopentyl and cyclopentenyl.

In one specific embodiment, R_{1A} and R_{2A} together with the carbon atom to which they are connected form the following substituent: 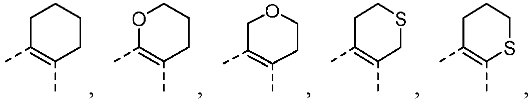 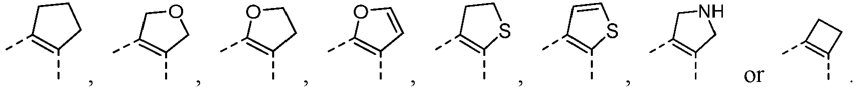 In one more specific embodiment, R_{1A} and R_{2A} together with the carbon atom to which they are connected form the following substituent: 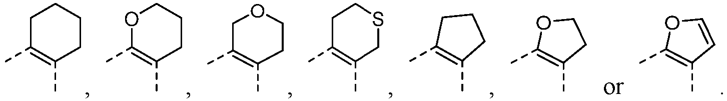 In one particularly specific embodiment, R_{1A} and R_{2A} together with the carbon atom to which they are connected form the following substituent: 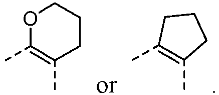 Particularly, the above groups are optionally substituted with one or more substituents, the substituents are independently selected from the group consisting of H, halogen, C₁-C₈ alkyl, C₁-C₈ alkoxy, hydroxyl, nitro, and cyano, preferably selected from the group consisting of H, halogen, C₁-C₈ alkyl, and C₁-C₈ alkoxy, and further preferably selected from the group consisting of H and halogen.

In one embodiment, L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; and the one or more substituents are independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}, preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₆ cycloalkyl, and OR_{4A}, and further preferably independently selected from the group consisting of H, halogen, C₁-C₈ alkyl, C₁-C₈ alkoxy, C₁-C₈ haloalkyl, C₃-C₆ cycloalkyl, and OR_{4A}; R_{4A} is selected from the group consisting of C₃-C₈ cycloalkyl and 3-8 membered heterocyclyl, preferably C₃-C₆ cycloalkyl.

In one preferred embodiment, L_{1A} is selected from phenyl, pyrimidinyl, pyridyl, pyrazinyl, thiazolyl, oxazolyl, imidazolyl, pyrazolyl, pyrrolyl, triazolyl, thiadiazolyl, thienyl, and furyl. In one more preferred embodiment, L_{1A} is selected from phenyl, pyridyl, pyrazolyl, thiazolyl, and thienyl. In one particularly preferred embodiment, L_{1A} is selected from phenyl, pyridyl, and thiazolyl. The above groups are optionally substituted with one or more substituents, which are independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}, preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₆ cycloalkyl, and OR_{4A}, further preferably independently selected from the group consisting of H, halogen, C₁-C₈ alkyl, C₁-C₈ alkoxy, C₁-C₈ haloalkyl, C₃-C₆ cycloalkyl, and OR_{4A};
R_{4A} is selected from the group consisting of C₃-C₈ cycloalkyl and 3-8 membered heterocyclyl, preferably C₃-C₆ cycloalkyl.

In one embodiment, the compound of formula I is represented by formula I-A, 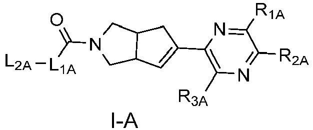
wherein R_{1A}, R_{2A}, and R_{3A} are independently selected from the group consisting of H, D, C₁-C₈ alkyl, C₁-C₈ alkoxy, halogen, haloalkyl, deuterated C₁-C₈ alkyl, and deuterated C₁-C₈ alkoxy, preferably selected from the group consisting of H, D, C₁-C₈ alkyl, C₁-C₈ alkoxy, and deuterated C₁-C₈ alkyl;
L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; the one or more substituents are independently selected from the group consisting of H, D, C₁-C₈ alkyl, C₁-C₈ alkoxy, halogen, haloalkyl, deuterated C₁-C₈ alkyl, and deuterated C₁-C₈ alkoxy, preferably independently selected from the group consisting of H, D, C₁-C₈ alkyl, C₁-C₈ alkoxy, and deuterated C₁-C₈ alkyl;
L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, further preferably selected from the group consisting of phenyl substituted with 0-3 Rbbb groups and 5-6 membered heteroaryl substituted with 0-3 Rbbb groups;
each Rbbb group is independently selected from the group consisting of H, D, C₁-C₈ alkyl, C₁-C₈ alkoxy, halogen, haloalkyl, deuterated C₁-C₈ alkyl, and deuterated C₁-C₈ alkoxy; preferably selected from the group consisting of H, D, C₁-C₈ alkyl, C₁-C₈ alkoxy, deuterated C₁-C₈ alkyl, and deuterated C₁-C₈ alkoxy.

In one embodiment, R_{1A}, R_{2A}, and R_{3A} are independently selected from the group consisting of H, D, C₁-C₈ alkyl, C₁-C₈ alkoxy, halogen, haloalkyl, deuterated C₁-C₈ alkyl, and deuterated C₁-C₈ alkoxy, preferably selected from the group consisting of H, D, C₁-C₈ alkyl, C₁-C₈ alkoxy, and deuterated C₁-C₈ alkyl.

In one preferred embodiment, the L_{2A}-L_{1A} structure is selected from the group consisting of 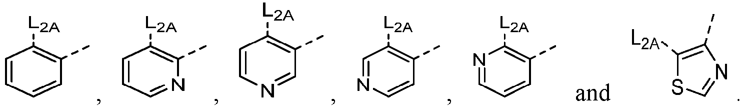 In one more preferred embodiment, the L_{2A}-L_{1A} structure is selected from the group consisting of 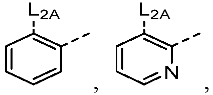 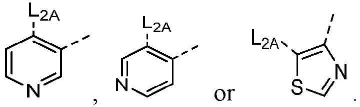 In one particularly preferred embodiment, the L_{2A}-L_{1A} structure is selected from the group consisting of

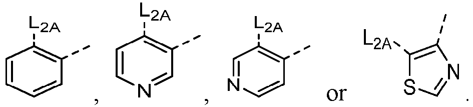

In one embodiment, the L_{1A} group in the above L_{2A}-L_{1A} structure is optionally substituted by one or more substituents, and the one or more substituents are independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}; preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₆ cycloalkyl, and OR_{4A}; further preferably independently selected from the group consisting of H, halogen, C₁-C₈ alkyl, C₁-C₈ alkoxy, C₁-C₈ haloalkyl, C₃-C₆ cycloalkyl, and OR_{4A}; R_{4A} is selected from the group consisting of C₃-C₈ cycloalkyl and 3-8 membered heterocyclyl, preferably C₃-C₆ cycloalkyl.

In one embodiment, the L_{1A} group in the above L_{2A}-L_{1A} structure is optionally substituted with one or more substituents, and the one or more substituents are independently selected from the group consisting of H, D, C₁-C₈ alkyl, C₁-C₈ alkoxy, halogen, haloalkyl, deuterated C₁-C₈ alkyl, and deuterated C₁-C₈ alkoxy, preferably independently selected from the group consisting of H, D, C₁-C₈ alkyl, C₁-C₈ alkoxy, and deuterated C₁-C₈ alkyl.

In one embodiment, L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, and further preferably selected from the group consisting of phenyl substituted with 0-3 Rbbb groups and 5-6 membered heteroaryl substituted with 0-3 Rbbb groups.

In one embodiment, L_{2A} is selected from the group consisting of phenyl, pyrimidinyl, pyridyl, pyrazinyl, oxazolyl, imidazolyl, pyrazolyl, pyrrolyl, 1,2,3-triazolyl, thienyl, furyl, and 1,3,4-triazolyl, preferably selected from the group consisting of phenyl, pyrimidinyl, pyridyl, pyrazolyl, thienyl, imidazolyl, and 1,2,3-triazolyl, further preferably selected from the group consisting of phenyl, pyridyl, pyrazolyl, thienyl, and 1,2,3-triazolyl; wherein the above groups are optionally substituted with 0-4 Rbbb groups.

In one embodiment, each Rbbb group is independently selected from the group consisting of H, optionally substituted C₁-C₈ alkyl, optionally substituted C₁-C₈ alkoxy, and halogen; and each substituent is independently selected from the group consisting of H, halogen, and hydroxyl.

In one embodiment, each Rbbb group is independently selected from the group consisting of H, D, C₁-C₈ alkyl, C₁-C₈ alkoxy, halogen, haloalkyl, deuterated C₁-C₈ alkyl, and deuterated C₁-C₈ alkoxy; preferably selected from the group consisting of H, D, C₁-C₈ alkyl, C₁-C₈ alkoxy, deuterated C₁-C₈ alkyl, and deuterated C₁-C₈ alkoxy.

In one specific embodiment, the compound of formula I-A is represented by formula II-A: 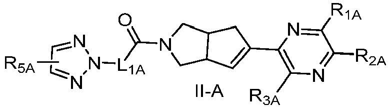 wherein R_{5A} is selected from the group consisting of H, optionally substituted C₁-C₈ alkyl, optionally substituted C₁-C₈ alkoxy, and halogen; and each substituent is independently selected from the group consisting of H, halogen, and hydroxyl.

In one specific embodiment, the compound of formula I-A is represented by formula III-A: 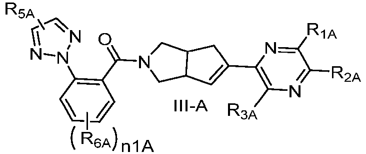
wherein R_{5A} is selected from the group consisting of H, optionally substituted C₁-C₈ alkyl, optionally substituted C₁-C₈ alkoxy, and halogen; and each substituent is independently selected from the group consisting of H, halogen, and hydroxyl;
n1A is an integer selected from 0-4, preferably 1;
R_{6A} is independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}; preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₈ cycloalkyl, and OR_{4A}; further preferably independently selected from the group consisting of H, halogen, C₁-C₈ alkyl, C₁-C₈ alkoxy, C₁-C₈ haloalkyl, C₃-C₈ cycloalkyl, and OR_{4A};
R_{4A} is selected from the group consisting of C₃-C₈ cycloalkyl and 3-8 membered heterocyclyl, preferably C₃-C₆ cycloalkyl.

In some embodiments, n1A is 1, and R_{6A} is located at position 2 of phenyl, or at position 3 of phenyl, or at position 4 of phenyl, or at position 5 of phenyl, or at position 6 of phenyl. In some embodiments, n1A is 1, and R_{6A} is located at position 4 of phenyl. In some embodiments, n1A is 1, and R_{6A} is located at position 6 of phenyl.

In one more preferred embodiment, R_{6A} is independently selected from the group consisting of H, D, halogen, and C₁-C₈ alkoxy. In some embodiments, R_{6A} is C₁-C₄ alkoxy, preferably methoxy. In some embodiments, R_{6A} is halogen, preferably F.

In one specific embodiment, the compound of formula I-A is represented by formula IV-A: 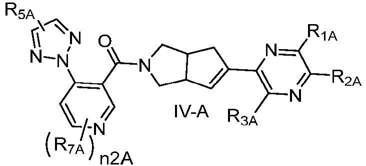
wherein R_{5A} is selected from the group consisting of H, optionally substituted C₁-C₈ alkyl, optionally substituted C₁-C₈ alkoxy, and halogen; and each substituent is independently selected from the group consisting of H, halogen, and hydroxyl;
n2A is an integer selected from 0-3, preferably 1;
R_{7A} is independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}; preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₈ cycloalkyl, and OR_{4A}; further preferably independently selected from the group consisting of H, halogen, C₁-C₈ alkyl, C₁-C₈ alkoxy, C₁-C₈ haloalkyl, C₃-C₈ cycloalkyl, and OR_{4A};
R_{4A} is selected from the group consisting of C₃-C₈ cycloalkyl and 3-8 membered heterocyclyl, preferably C₃-C₆ cycloalkyl.

In some embodiments, n2A is 1, and R_{7A} is located at position 2 of pyridyl, or at position 5 of pyridyl, or at position 6 of pyridyl. In some embodiments, n2A is 1, and R_{7A} is located at position 5 of pyridyl. In some embodiments, n2A is 1, and R_{7A} is located at position 6 of pyridyl.

In one more preferred embodiment, R_{7A} is independently selected from the group consisting of H, D, halogen, and C₁-C₈ alkoxy. In some embodiments, R_{7A} is a C₁-C₄ alkoxy, preferably methoxy. In some embodiments, R_{7A} is halogen, preferably F.

In one specific embodiment, the compound of formula I-A is represented by formula V-A: 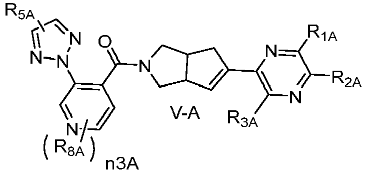
wherein R_{5A} is selected from the group consisting of H, optionally substituted C₁-C₈ alkyl, optionally substituted C₁-C₈ alkoxy, and halogen; and each substituent is independently selected from the group consisting of H, halogen, and hydroxyl;
n3A is an integer selected from 0-3, preferably 1;
R_{8A} is independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}; preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₈ cycloalkyl, and OR_{4A}; further preferably independently selected from the group consisting of H, halogen, C₁-C₈ alkyl, C₁-C₈ alkoxy, C₁-C₈ haloalkyl, C₃-C₈ cycloalkyl, and OR_{4A};
R_{4A} is selected from the group consisting of C₃-C₈ cycloalkyl and 3-8 membered heterocyclyl, preferably C₃-C₆ cycloalkyl.

In some embodiments, n3A is 1, and R_{8A} is located at position 2 of pyridyl, or at position 5 of pyridyl, or at position 6 of pyridyl. In some embodiments, n3A is 1, and R_{7A} is located at position 5 of pyridyl. In some embodiments, n3A is 1, and R_{8A} is located at position 6 of pyridyl.

In one more preferred embodiment, R_{8A} is independently selected from the group consisting of H, D, halogen, and C₁-C₈ alkoxy. In some embodiments, R_{8A} is C₁-C₄ alkoxy, preferably methoxy. In some embodiments, R_{8A} is halogen, preferably F.

In one embodiment, the compound of formula I is represented by formula I-B, 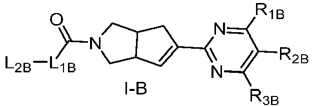
wherein R_{1B} and R_{3B} are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of halogen, D, C₁-C₈ alkoxy, hydroxyl, nitro, and cyano;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F;
or R_{1B} and R_{2B} together form a 3-8 membered heterocyclyl, a 5-8 membered heteroaryl, or a C₃-C₈ cycloalkyl optionally substituted with one or more substituents, and each substituent is independently selected from the group consisting of halogen, D, C₁-C₈ alkyl, C₁-C₈ alkoxy, hydroxyl, nitro, and cyano;
L_{1B} is selected from the group consisting of optionally substituted phenyl and optionally substituted pyridyl, each substituent is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxy alkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
L_{2B} is 5-14 membered heteroaryl substituted with 0-4 Rbb groups, preferably 5-10 membered monocyclic or bicyclic heteroaryl substituted with 0-3 Rbb groups, and further preferably 5-6 membered heteroaryl substituted with 0-3 Rbb groups;
each Rbb group is independently selected from the group consisting of H, D, optionally substituted C₁-C₈ alkyl, optionally substituted C₁-C₈ alkoxy, and halogen, preferably selected from the group consisting of H and C₁-C₈ alkyl; and each substituent is independently selected from the group consisting of D and halogen.

In one embodiment, R_{1B} and R_{3B} are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably independently selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of halogen, D, C₁-C₈ alkoxy, hydroxyl, nitro, and cyano; the 3-8 membered heterocyclyl is preferably selected from the group consisting of oxiranyl, azetidinyl, oxetanyl, thietanyl, tetrahydrofuryl, tetrahydrothiopyranyl, piperidinyl, and pyrrolidinyl; the C₃-C₈ cycloalkyl is preferably selected from the group consisting of cyclopropyl, cyclobutyl, cyclopentyl, and cyclohexyl, more preferably cyclopropyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

In one embodiment, R_{1B} and R_{2B} together form a 3-8 membered heterocyclyl, a 5-8 membered heteroaryl, or a C₃-C₈ cycloalkyl optionally substituted with one or more substituents; the 3-8 membered heterocyclyl is preferably selected from the group consisting of tetrahydrofuryl, dihydrofuryl, tetrahydropyranyl, dihydropyranyl, azetidinyl, oxetanyl, thietanyl, piperidinyl, and pyrrolidinyl, and further preferably selected from the group consisting of tetrahydrofuryl, tetrahydropyranyl, and dihydropyranyl; the 5-8 membered heteroaryl is preferably selected from the group consisting of furyl, pyranyl, and thienyl; the C₃-C₈ cycloalkyl is preferably selected from the group consisting of cyclopropyl, cyclobutyl, cyclopentyl, cyclohexyl, cyclopentenyl and cyclohexenyl, and further preferably selected from the group consisting of cyclopentyl, cyclohexyl, cyclopentenyl and cyclohexenyl; and each substituent is independently selected from the group consisting of halogen, D, C₁-C₈ alkyl, C₁-C₈ alkoxy, hydroxyl, nitro, and cyano.

In one specific embodiment, R_{1B} and R_{2B} together with the pyrimidinyl to which they are connected form the following substituent: 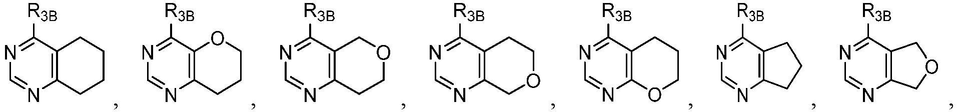 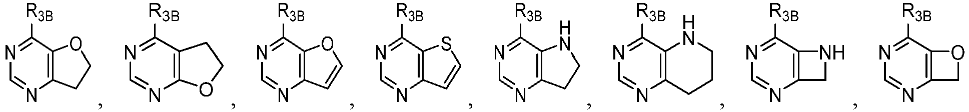 or 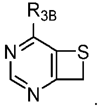 In one more specific embodiment, R_{1B} and R_{2B} together with the pyrimidinyl to which they are connected form the following substituent: 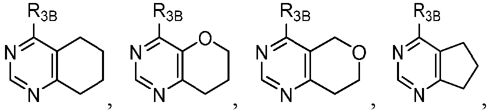 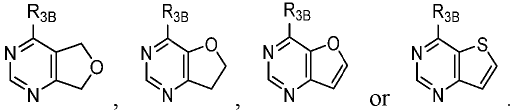 Wherein, the above groups are optionally substituted with one or more substituents, which are independently selected from the group consisting of halogen, D, C₁-C₈ alkyl, C₁-C₈ alkoxy, hydroxyl, nitro, and cyano.

In one preferred embodiment, R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, and nitro, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

In one embodiment, L_{1B} is selected from the group consisting of optionally substituted phenyl and optionally substituted pyridyl, and each substituent is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably independently selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio; R_{4B} is selected from the group consisting of C₃-C₈ cycloalkyl and 3-8 membered heterocyclyl, preferably C₃-C₆ cycloalkyl.

In one preferred embodiment, L_{1B} is selected from the group consisting of 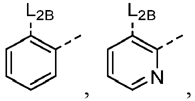 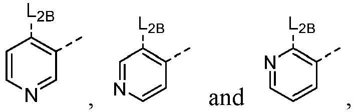 preferably selected from the group consisting of 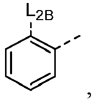 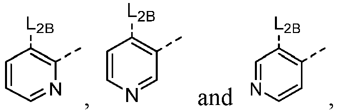 further preferably selected from the group consisting of 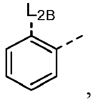 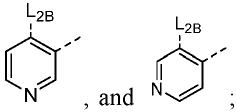 particularly, the above groups are optionally substituted with one or more substituents, which are independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably independently selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio; R_{4B} is selected from the group consisting of C₃-C₈ cycloalkyl and 3-8 membered heterocyclyl, preferably C₃-C₆ cycloalkyl.

In one embodiment, L_{2B} is selected from the group consisting of pyrimidinyl, pyridyl, pyrazinyl, thiazolyl, oxazolyl, imidazolyl, pyrazolyl, pyrrolyl, 1,2,3-triazolyl, thiadiazole, thienyl, furyl, and 1,3,4-triazolyl, preferably selected from the group consisting of pyrazolyl, imidazolyl, and 1,2,3-triazolyl; further preferably selected from 1,2,3-triazolyl; wherein, the above groups are optionally substituted with 0-4 Rbb groups.

In one embodiment, each Rbb group is independently selected from the group consisting of H, D, optionally substituted C₁-C₈ alkyl, optionally substituted C₁-C₈ alkoxy, and halogen, preferably selected from the group consisting of H and C₁-C₈ alkyl; and each substituent is independently selected from the group consisting of D and halogen.

In one embodiment, the heteroaryl is selected from the group consisting of pyrimidinyl, pyridyl, pyrazinyl, thiazolyl, oxazolyl, imidazolyl, pyrazolyl, pyrrolyl, 1,2,3-triazolyl, thiadiazole, thienyl, furyl, 1,3,4-triazolyl, and pyranyl. In one preferred embodiment, the heteroaryl is selected from the group consisting of imidazolyl, pyrazolyl, thienyl, furyl, 1,2,3-triazolyl, and pyranyl. In one more preferred embodiment, the heteroaryl is selected from the group consisting of thienyl, furyl, 1,2,3-triazolyl, and pyranyl.

In one embodiment, the heterocyclyl is preferably selected from the group consisting of oxiranyl, tetrahydrothiopyranyl, tetrahydrofuryl, dihydrofuryl, tetrahydropyranyl, dihydropyranyl, azetidinyl, oxetanyl, thietanyl, piperidinyl, and pyrrolidinyl. In one preferred embodiment, the heterocyclyl is selected from the group consisting of tetrahydrofuryl, dihydrofuryl, tetrahydropyranyl, and dihydropyranyl. In one particularly preferred embodiment, the heterocyclyl is selected from the group consisting of tetrahydrofuryl, dihydrofuryl, and tetrahydropyranyl.

In one embodiment, the C₃-C₈ cycloalkyl is C₃-C₆ cycloalkyl. In one preferred embodiment, the C₃-C₈ cycloalkyl and C₃-C₆ cycloalkyl are independently selected from the group consisting of cyclopropyl, cyclobutyl, cyclopentyl, cyclohexyl, cyclopentenyl, and cyclohexenyl. In one more preferred embodiment, the C₃-C₈ cycloalkyl and C₃-C₆ cycloalkyl are independently selected from the group consisting of cyclopropyl, cyclopentyl, cyclohexyl, cyclopentenyl, and cyclohexenyl. In one particularly preferred embodiment, the C₃-C₈ cycloalkyl and C₃-C₆ cycloalkyl are independently cyclopentyl or cyclopentenyl.

In one specific embodiment, the compound of formula I-B is represented by formula II-B: 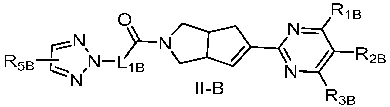
wherein R_{5B} is selected from the group consisting of H, D, optionally substituted C₁-C₈ alkyl, optionally substituted C₁-C₈ alkoxy, and halogen, and each substituent is independently selected from the group consisting of D and halogen;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

In one specific embodiment, the compound of formula I-B is represented by formula III-B: 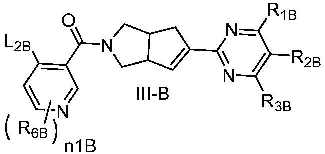
wherein n1B is an integer selected from 0-3;
R_{6B} is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, or halogen, and even further preferably F.

In some embodiments, n1B is 1, and R_{6B} is located at position 2 of pyridyl, or at position 5 of pyridyl, or at position 6 of pyridyl. In some embodiments, n1B is 1, and R_{6B} is located at position 5 of pyridyl. In some embodiments, n1B is 1, and R_{6B} is located at position 6 of pyridyl.

In one more preferred embodiment, R_{6B} is independently selected from the group consisting of H, D, halogen, and C₁-C₈ alkoxy. In some embodiments, R_{6B} is C₁-C₄ alkoxy, preferably methoxy. In some embodiments, R_{6B} is halogen, preferably F.

In one specific embodiment, the compound of formula I-B is represented by formula IV-B: 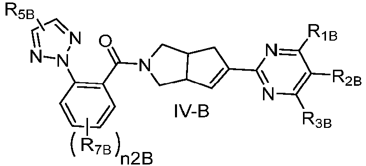
wherein R_{5B} is selected from the group consisting of H, D, optionally substituted C₁-C₈ alkyl, optionally substituted C₁-C₈ alkoxy, and halogen, and each substituent is independently selected from the group consisting of D and halogen;
n2B is an integer selected from 0-4, preferably 1;
R_{7B} is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, halogen, further preferably selected from the group consisting of C₁-C₈ alkyl, C₁-C₈ haloalkyl, and halogen, and even further preferably F.

In some embodiments, n2B is 1, and R_{7B} is located at position 2 of phenyl, or at position 3 of phenyl, or at position 4 of phenyl, or at position 5 of phenyl, or at position 6 of phenyl. In some embodiments, n2B is 1, and R_{7B} is located at position 4 of phenyl. In some embodiments, n2B is 1, and R_{7B} is located at position 6 of phenyl.

In one more preferred embodiment, R_{7B} is independently selected from the group consisting of H, D, halogen, and C₁-C₈ alkoxy. In some embodiments, R_{7B} is C₁-C₄ alkoxy, preferably methoxy. In some embodiments, R_{7B} is halogen, preferably F.

In one specific embodiment, the compound of formula I-B is represented by formula V-B: 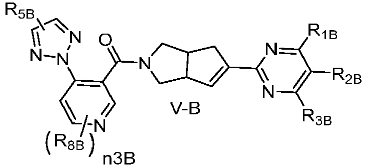
wherein R_{5B} is selected from the group consisting of H, D, optionally substituted C₁-C₈ alkyl, optionally substituted C₁-C₈ alkoxy, and halogen, and each substituent is independently selected from the group consisting of D and halogen;
n3B is an integer selected from 0-3, preferably 1,
R_{8B} is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B} and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B} and C₁-C₈ alkylthio; R_{4B} is selected from the group consisting of C₃-C₈ cycloalkyl and 3-8 membered heterocyclyl, preferably C₃-C₆ cycloalkyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, and nitro, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

In some embodiments, n3B is 1, and R_{8B} is located at position 2 of pyridyl, or at position 5 of pyridyl, or at position 6 of pyridyl. In some embodiments, n3B is 1, and R_{8B} is located at position 5 of pyridyl. In some embodiments, n3B is 1, and R_{8B} is located at position 6 of pyridyl.

In one more preferred embodiment, R_{8B} is independently selected from the group consisting of H, D, halogen, and C₁-C₈ alkoxy. In some embodiments, R_{8B} is C₁-C₄ alkoxy, preferably methoxy. In some embodiments, R_{8B} is halogen, preferably F.

In one specific embodiment, the compound of formula I is selected from any one of the following compounds:

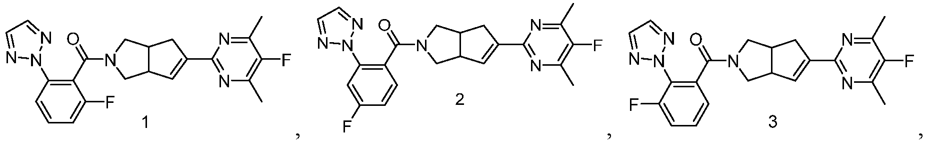

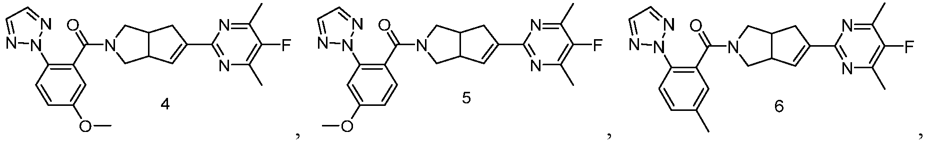

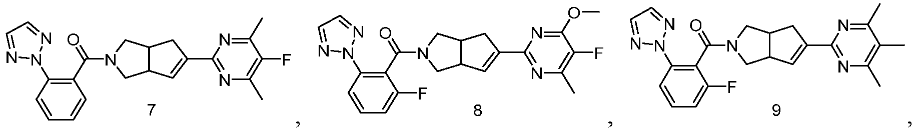

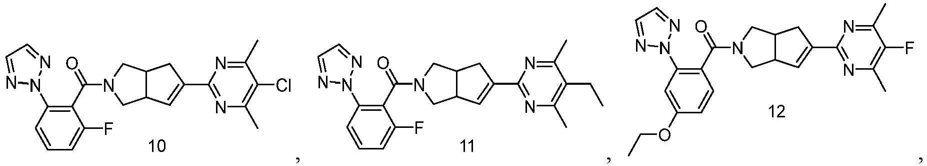

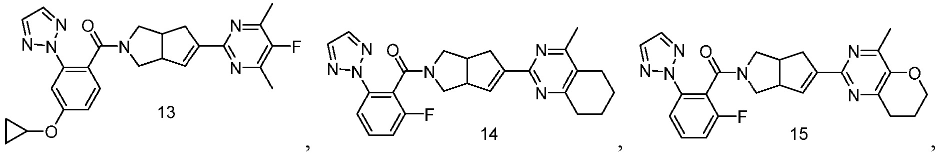

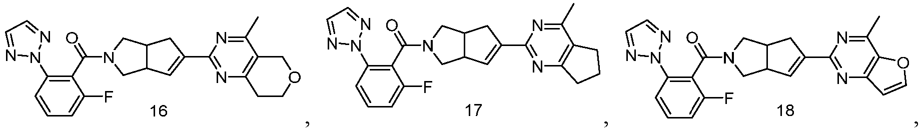

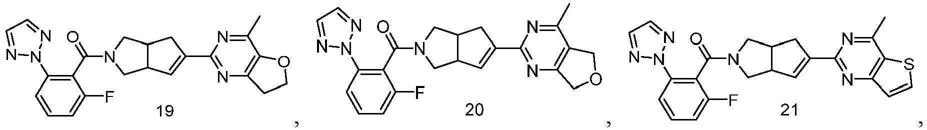

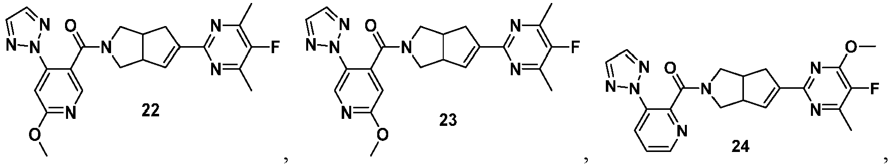

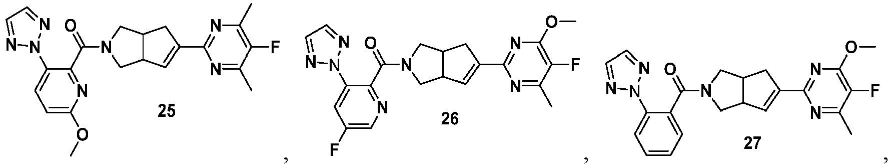

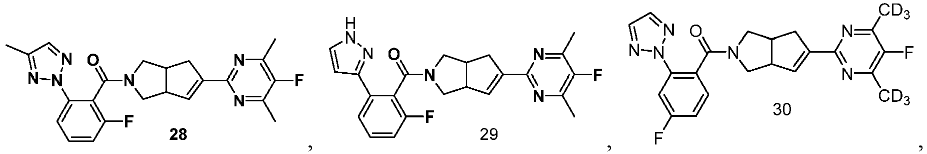

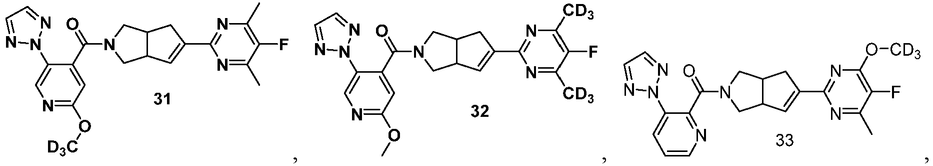

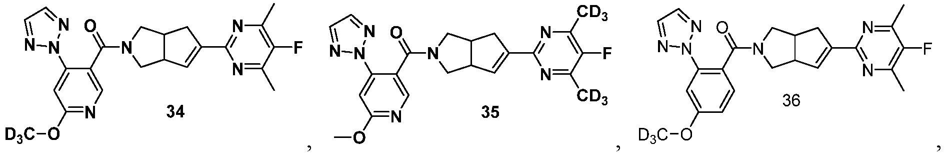

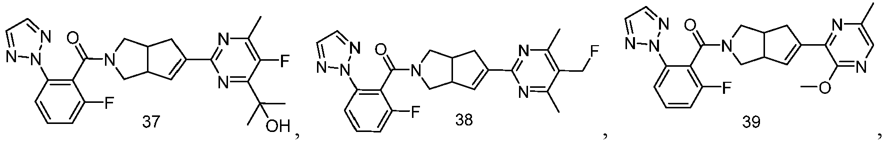

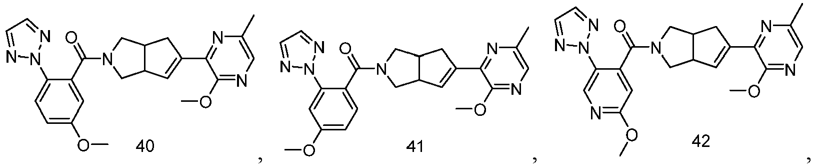

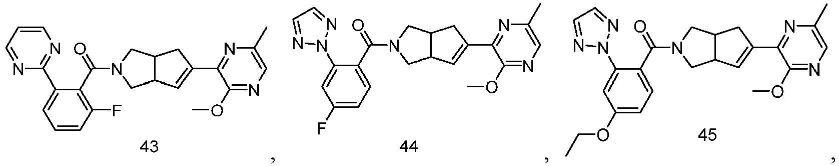

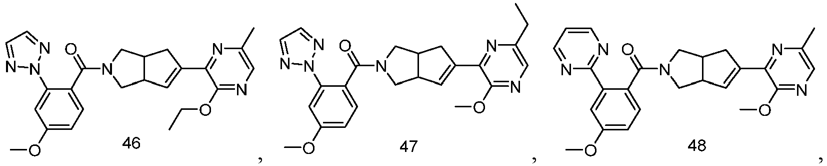

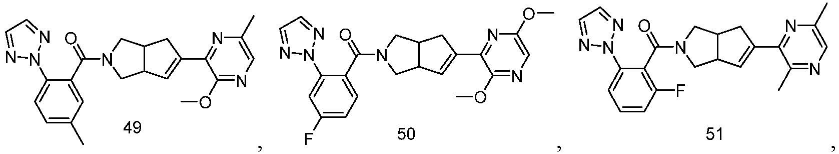

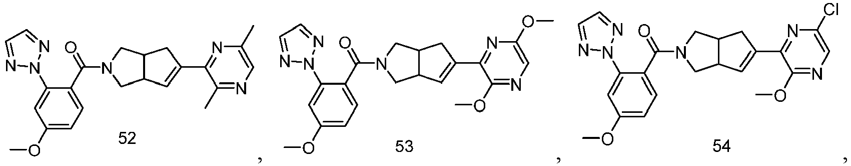

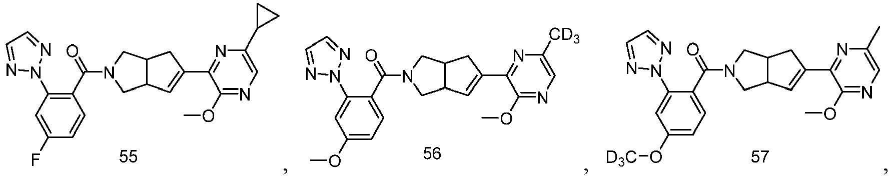

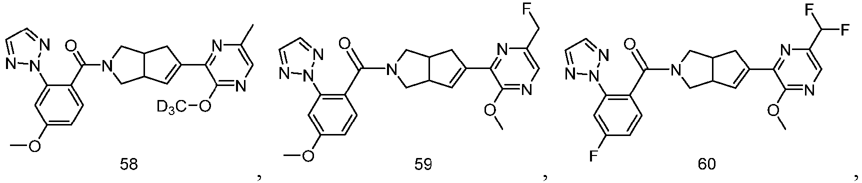

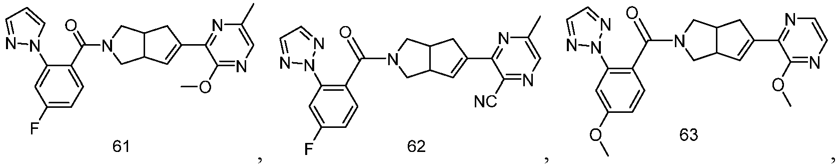

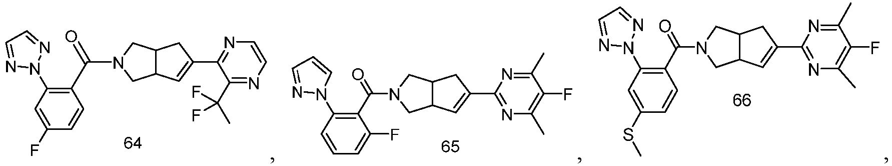

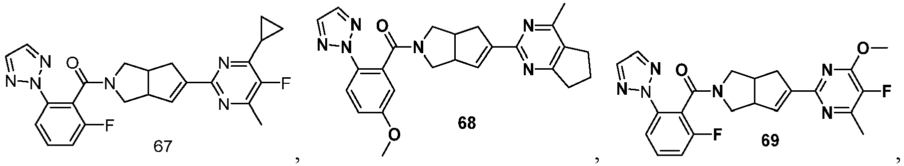

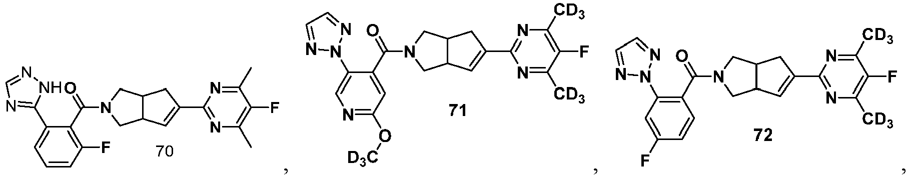

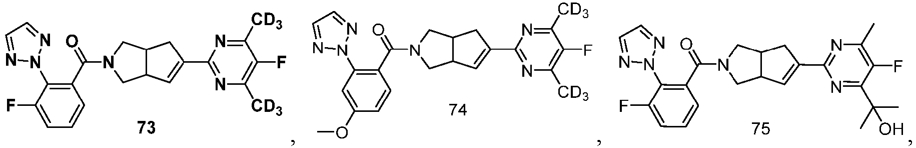

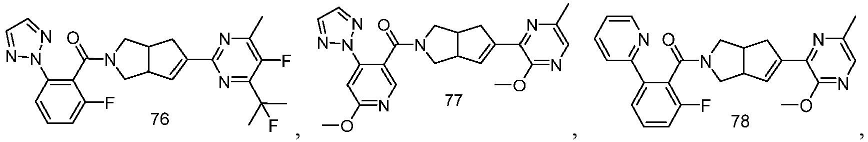

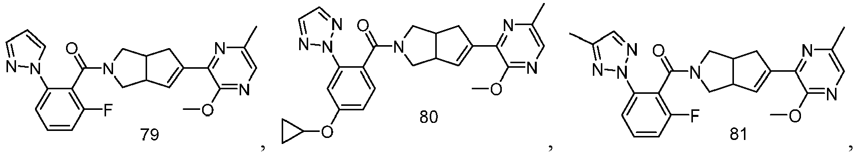

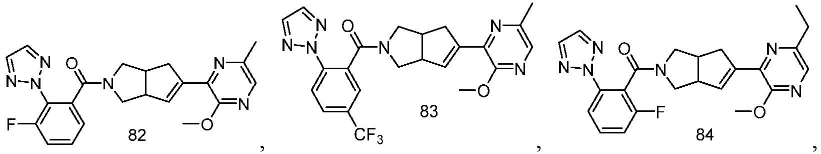

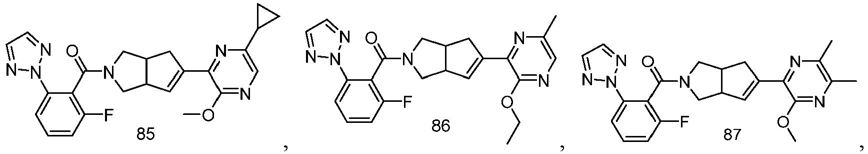

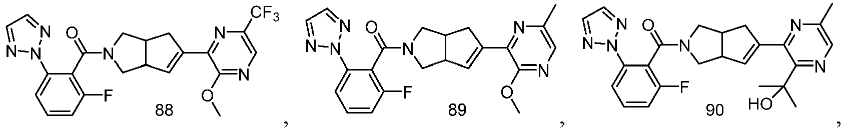

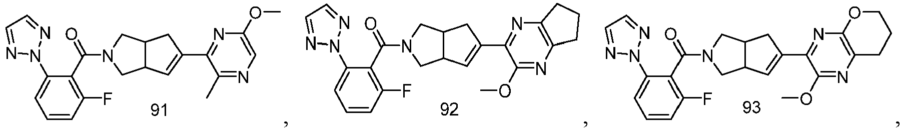

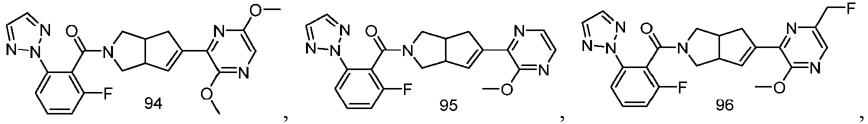

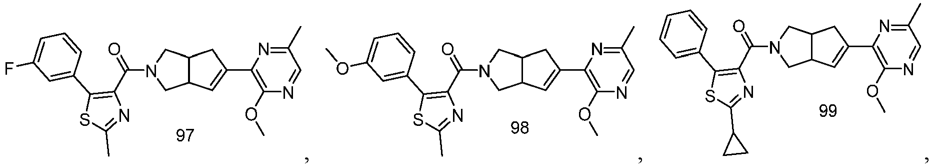

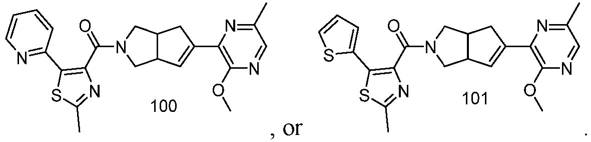

### Beneficial Technical Effects of the Invention

Compared with the prior art, the technical solution of the present application has the following advantages:
the present application relates to a class of novel structural compounds, which are selective OX2R antagonists and can be used for the prevention, treatment and/or alleviation of diseases related to orexin receptors, preferably for the treatment of insomnia, depression, Alzheimer's disease, and sleep apnea. The compounds exhibit good selectivity and pharmacodynamic activity as OX2 receptor antagonists, as well as good pharmacokinetic properties, improves the oral bioavailability of subjects, significantly reduces the autonomous activity of subjects, and has good prospects for clinical application.

### Brief Description of the Drawings

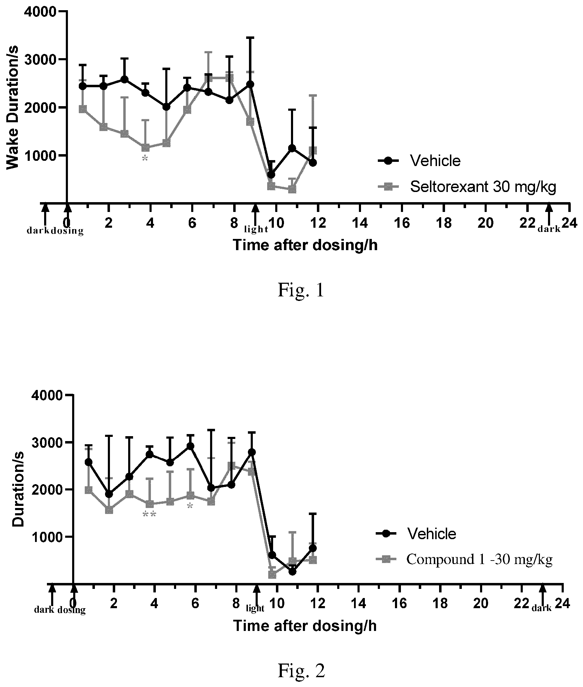

Fig. 1 shows the wake duration of rats in each time period for 12 h after administration of 30 mg/kg of Seltorexant;
Fig. 2 shows the wake duration of rats in each time period for 12 h after administration of 30 mg/kg of compound 1;

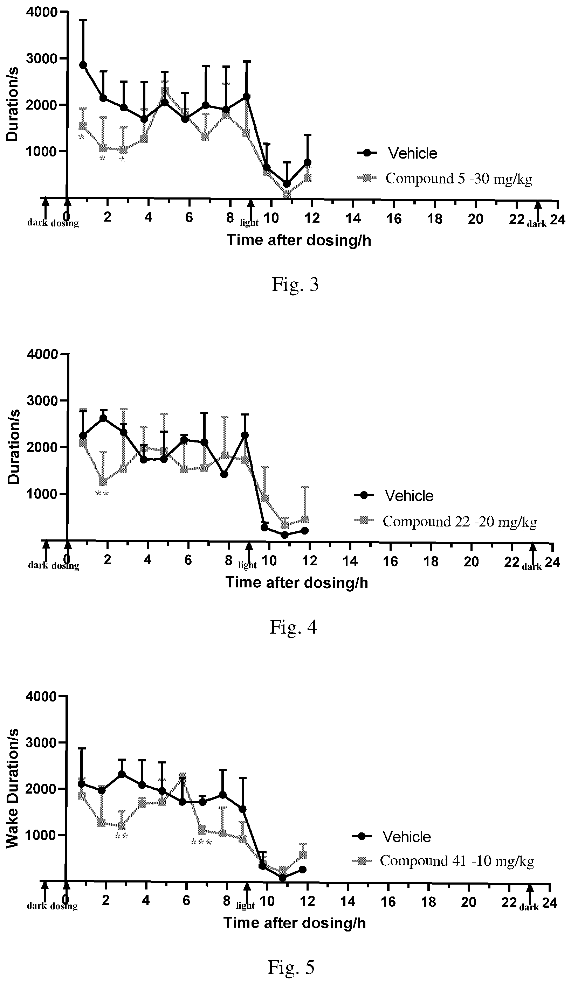

Fig. 3 shows the wake duration of rats in each time period for 12 h after administration of 30 mg/kg of compound 5;
Fig. 4 shows the wake duration of rats in each time period for 12 h after administration of 30 mg/kg of compound 22;
Fig. 5 shows the wake duration of rats in each time period for 12 h after administration of 30 mg/kg of compound 41.

### Examples

The examples of the present application are described in detail below. The examples described below are exemplary and are only used to explain the present application, and cannot be understood as limiting the present application. Unless otherwise specified, the ratios, percentages, etc. referred to herein are all by weight.

The following examples are prepared using the cis structure as the starting material, that is, tert-butyl cis-5-oxohexahydrocyclopenta[c]pyrrole-2(1H)-carboxylate is used as the starting material to obtain the example compounds that are all cis isomers.

### Synthesis Example

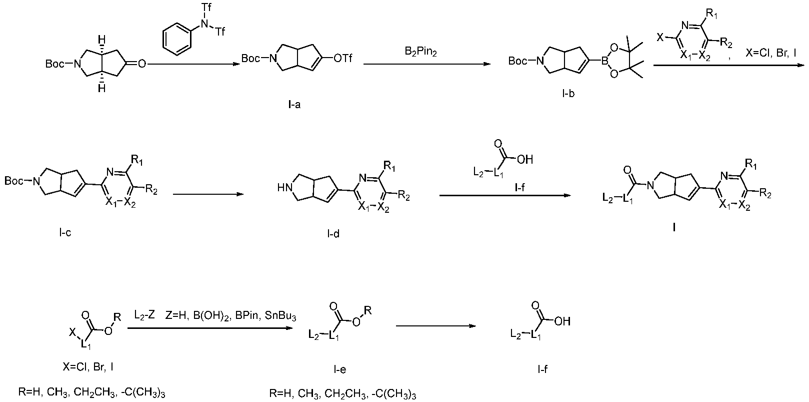

Tert-butyl cis-5-oxohexahydrocyclopenta[c]pyrrole-2(1H)-carboxylate was subjected to trifluoromethanesulfonylation reaction with N-phenylbistrifluoromethylsulfonimide to obtain intermediate I-a, I-a was then subjected to boronate esterification reaction with bis(pinacolato)diboron to obtain intermediate I-b, I-b was subjected to coupling reaction with 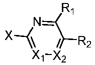 to obtain intermediate I-c, I-c was removed the Boc protecting group to obtain intermediate I-d; 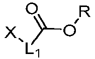 and L₂-Z are subjected to coupling reaction to obtain intermediate I-e, I-e was subjected to ester hydrolysis reaction to obtain intermediate I-f, and finally I-d and I-f were subjected to condensation reaction to obtain the compound represented by formula I.

### Example 1: Preparation of (5-(5-fluoro-4,6-dimethylpyrimidin-2-yl)-3,3a,4,6a-tetrahydrocyclopenta[c]pyrrol-2(1H)-yl)(2-fluoro-6-(2H-1,2,3-triazol-2-yl) phenyl)methanone (1)

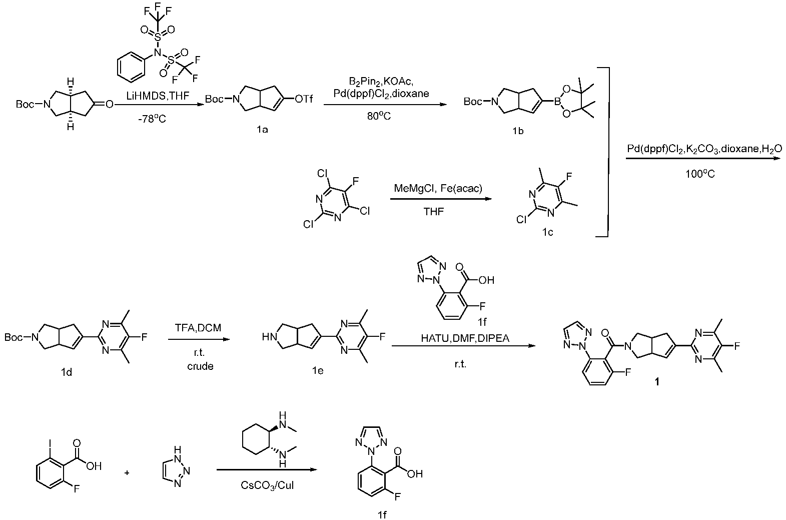

1.1 A solution of tert-butyl cis-5-oxo-hexahydrocyclopenta[c]pyrrole-2-carboxylate (5 g, 22.19 mmol) in THF (20 mL) was treated with LiHMDS (5.2 g, 110.95 mmol) at -78 °C for 2 hours, followed by the dropwise addition of N-phenyl bis(trifluoromethanesulfonyl)imide (9.51 g, 26.63 mmol) at -78 °C. The resulting mixture was stirred overnight at room temperature under nitrogen atmosphere. The reaction was quenched by the addition of water/ice (10 mL) at room temperature. The resulting mixture was extracted with EtOAc (3 x 50 mL). The combined organic layers were washed with brine (3 x 20 mL) and dried over anhydrous Na₂SO₄. After filtration, the filtrate was concentrated under reduced pressure to obtain a residue, which was purified by silica gel column chromatography and eluted with PE/EA (1:1) to obtain tert-butyl 5-[[(trifluoromethyl)sulfonyl]oxy]-3,3a,4,6a-tetrahydrocyclopenta[c]pyrrole-2(1H)-carboxylate (Compound 1a) (4.8 g).

1.2 1a (4.5 g, 12.59 mmol), bis(pinacolato)diboron (4.8 g, 18.90 mmol), KOAc (3.5 g, 25.36 mmol) and Pd(dppf)Cl₂ (0.9 g, 1.24 mmol) were added to a solution of dioxane (20 mL), heated to 80 °C and stirred overnight under nitrogen atmosphere. The mixture was cooled to room temperature. The resulting mixture was diluted with water (20 ml). The resulting mixture was extracted with EtOAc (3 x 50 mL). The combined organic layers were washed with brine (2 x 20 ml) and dried over anhydrous Na₂SO₄. After filtration, the filtrate was concentrated under reduced pressure to obtain 3.6 g of crude product of tert-butyl 5-(4,4,5,5-tetramethyl-1,3,2-dioxaborolan-2-yl)-3,3a,4,6a-tetrahydrocyclopenta[c]pyrrole-2(1H)-car boxylate (Compound 1b). The crude product was used directly in the next step without further purification.

1.3 2,4,6-Trichloro-5-fluoropyrimidine (5 g, 24.87 mmol) and iron triacetylacetonate (0.9 g, 2.55 mmol) were dissolved in 20 mL THF solution, cooled to -78 °C, and MeMgCl (3.0M solution in THF, 19 mL) was added dropwise under nitrogen protection. After the addition was complete, the mixture was reacted at -78 °C for 1 hour, and then placed at room temperature for 3 hours. After the reaction was complete, the reaction solution was poured into NH₄Cl solution to quench, extracted with EtOAc (3 x 50 mL), and the combined organic layers were washed with brine (2 x 20 ml) and dried over anhydrous Na₂SO₄. After filtration, the filtrate was concentrated under reduced pressure and eluted with PE/EA (20:1) to obtain 3.4 g of 2-chloro-5-fluoro-4,6-dimethylpyrimidine (1c).

1.4 1b (3.5 g, 10.44 mmol), 1c (1.8 g, 11.48 mmol), K₂CO₃ (2.9 g, 21.01 mmol), and Pd(dppf)Cl₂ (0.76 g, 1.05 mmol) were dissolved in a mixed solution of dioxane (20 mL)/H₂O (5 mL) and heated to 100 °C under nitrogen protection for reaction. After the reaction was completed, the reaction solution was cooled to room temperature, diluted with 30 mL of water, and then extracted with ethyl acetate (3 x 50 mL). The organic phase was washed with saturated brine (2 x 20 mL), dried over anhydrous Na₂SO₄, filtered, and the filtrate was concentrated under reduced pressure, and then separated and purified by column chromatography (PE/EA=1:1) to obtain tert-butyl 5-(5-fluoro-4,6-dimethylpyrimidin-2-yl)-3,3a,4,6a-tetrahydrocyclopenta[c]pyrrole-2(1H)-carboxylat e (Compound 1d) (2.8 g).

1.5 Compound 1d (2.8 g, 8.40 mmol) and trifluoroacetic acid (10.00 mL) were added to dichloromethane (30.00 mL) and stirred at room temperature for 2 hours under nitrogen protection. After the reaction was completed, the reaction solution was concentrated under reduced pressure to obtain a crude product of 5-(5-fluoro-4,6-dimethylpyrimidin-2-yl)-1,2,3,3a,4,6a-hexahydrocyclopenta[c]pyrrole (Compound 1e). The crude product was used directly in the next step without further purification.

1.6 2-Fluoro-6-iodobenzoic acid (10.0 g, 37.59 mmol), 1,2,3-triazole (5.2 g, 75.29 mmol), cuprous iodide (0.35 g, 1.2 mmol), cesium carbonate (24.5 g, 75.15 mmol), (1R,2R)-N,N-dimethyl-1,2-diaminocyclohexane (1.1 g, 7.73 mmol) and dioxane (100 mL) were placed in a 250 mL round-bottom flask and stirred at 85 °C overnight under nitrogen protection. After the reaction was completed, the reaction solution was cooled to room temperature, 50 mL of tert-butyl methyl ether and 50 mL of water were added, and stirring was continued for half an hour before separation. After discarding the organic phase, the aqueous phase was adjusted to an acidic pH with 2N hydrochloric acid, and then extracted with ethyl acetate (50 mL × 3). The organic phases were combined, dried over anhydrous sodium sulfate, concentrated, and separated by column chromatography (petroleum ether/ethyl acetate = 1:1) to obtain 2-fluoro-6-(2H-1,2,3-triazol-2-yl) benzoic acid (Compound 1f) (4.6 g).

1.7 1e (100 mg, 0.43 mmol) and 1f (101 mg, 0.49 mmol) were added to DMF (5 mL), and HATU (262 mg, 0.69 mmol) were added and DIPEA (119 mg, 0.92 mmol) were added dropwise at room temperature under nitrogen protection, stirring at room temperature to react for 1 hour. After the reaction was completed, the reaction solution was extracted with ethyl acetate (3 x 15 mL), and the organic phase was washed with water (3x10 mL), dried over anhydrous Na₂SO₄, filtered, concentrated under reduced pressure, and separated by column chromatography (petroleum ether/ethyl acetate = 1:2) to obtain 68 mg of (5-(5-fluoro-4,6-dimethylpyrimidin-2-yl)-3,3a,4,6a-tetrahydrocyclopenta[c]pyrrol-2(1H)-yl)(2-fluoro-6-(2H-1,2,3-triazol-2-yl)phenyl)methanone (Compound 1). ¹H NMR (400 MHz, Methanol-*d*₄) δ 7.99 - 7.78 (m, 2H), 7.75 - 7.55 (m, 2H), 7.35 - 7.18 (m, 1H), 6.77 - 6.45 (m, 1H), 3.99 - 3.74 (m, 2H), 3.64 (ddd, *J =* 24.2, 18.9, 10.8 Hz, 2H), 3.51 - 3.35 (m, 1H), 3.26 - 3.00 (m, 2H), 3.00 - 2.82 (m, 1H), 2.71 - 2.39 (m, 6H). LCMS (ES, m/z): 423 [M+H]⁺.

### Example 2: Preparation of (4-fluoro-2-(2H-1,2,3-triazol-2-yl)phenyl)(5-(5-fluoro-4,6-dimethylpyrimidin-2-yl)-3,3a,4,6a-te trahydrocyclopenta[c]pyrrol-2(1H)-yl)methanone (2)

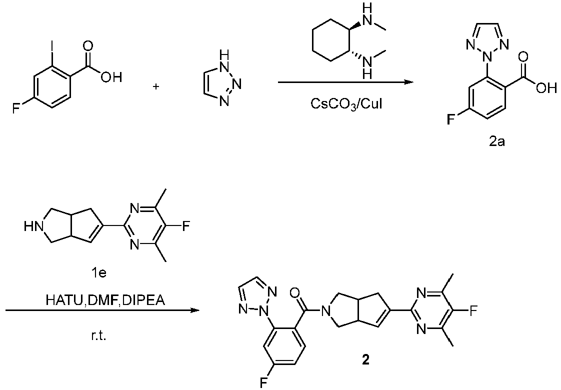

2.1 The reaction raw material 2-fluoro-6-iodobenzoic acid was replaced with 4-fluoro-6-iodobenzoic acid, and 4-fluoro-6-(2H-1,2,3-triazol-2-yl) benzoic acid (Compound 2a) was prepared according to the method of Example 1-1.6.

2.2 The reaction raw material 1f was replaced with 2a, and (4-fluoro-2-(2H-1,2,3-triazol-2-yl)phenyl)(5-(5-fluoro-4,6-dimethylpyrimidin-2-yl)-3,3 a,4,6a-tetrahydrocyclopenta[c]pyrrol-2(1H)-yl)methanone (Compound 2) was prepared according to the method of Example 1-1.7. ¹H NMR (400 MHz, DMSO-*d*₆) δ 8.13 - 7.91 (m, 2H), 7.77 - 7.72 (m, 1H), 7.53 - 7.48 (m, 1H), 7.43 - 7.35 (m, 1H), 6.58 (d, *J =* 80.4 Hz, 1H), 3.80 - 3.45 (m, 3H), 3.26 - 2.90 (m, 3H), 2.84 - 2.59 (m, 2H), 2.44 (s, 6H). LCMS (ES, m/z): 423 [M+H]⁺.

### Example 3: Preparation of (3-fluoro-2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (3)

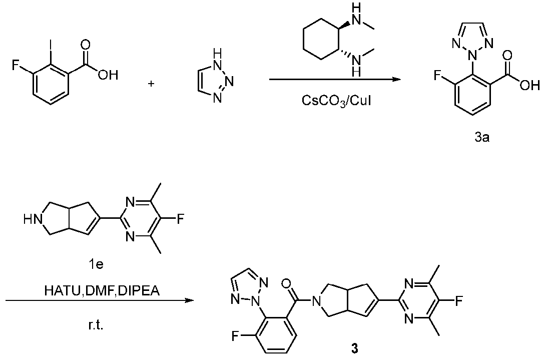

3.1 The reaction raw material 2-fluoro-6-iodobenzoic acid was replaced with 3-fluoro-2-iodobenzoic acid, and 3-fluoro-2-(2H-1,2,3-triazol-2-yl) benzoic acid (Compound 3a) was prepared according to the method of Example 1-1.6.

3.2 The reaction raw material 1f was replaced with 3a, and (3-fluoro-2-(2H-1,2,3-triazol-2-yl)phenyl)(5-(5-fluoro-4,6-dimethylpyrimidin-2-yl)-3,3 a,4,6a-tetra-hydrocyclopenta[c]pyrrol-2(1H)-yl)methanone (Compound 3) was prepared according to the method of Example 1-1.7. ¹H NMR (400 MHz, DMSO-*d*₆) δ 7.95 (d, *J =* 22.4 Hz, 2H), 7.70 - 7.56 (m, 2H), 7.34 (t, *J =* 6.2 Hz, 1H), 6.60 (d, *J =* 20.0 Hz, 1H), 3.65 - 3.36 (m, 3H), 3.10 - 2.81 (m, 3H), 2.67 - 2.53 (m, 2H), 2.43 (s, 6H). LCMS (ES, m/z): 423 [M+H]⁺.

### Example 4: Preparation of 5-(5-fluoro-4,6-dimethylpyrimidin-2-yl)-3,3a,4,6a-tetrahydrocyclopenta[c]pyrrol-2(1H)-yl)(5-methoxy-2-(2H-1,2,3-triazol-2-yl)phenyl)methanone (4)

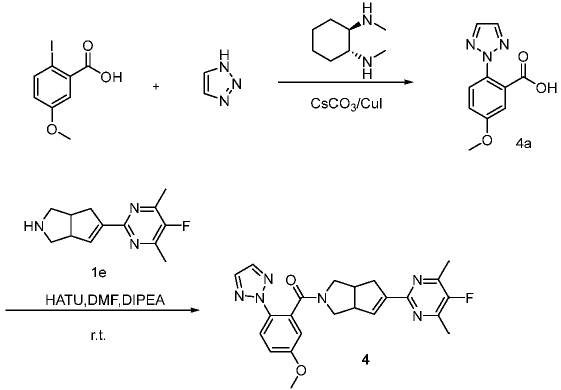

4.1 The reaction raw material 2-fluoro-6-iodobenzoic acid was replaced with 2-iodo-5-methoxybenzoic acid, and 5-methoxy-2-(2H-1,2,3-triazol-2-yl)benzoic acid (Compound 4a) was prepared according to the method of Example 1-1.6.

4.2 The reaction raw material 1f was replaced with 4a, and (5-(5-fluoro-4, 6-dimethylpyrimidin-2-yl)-3,3 a,4,6a-tetrahydrocyclopenta[c]pyrrol-2(1H)-yl)(5-methoxy-2-(2H-1,2,3-triazol-2-yl)phenyl)methanone (Compound 4) was prepared according to the method of Example 1-1.7. ¹H NMR (400 MHz, DMSO-*d*₆) δ 7.91 (d, *J =* 23.2 Hz, 2H), 7.68 - 7.51 (m, 2H), 7.14 - 7.07 (m, 1H), 6.51 (d, *J =* 41.7 Hz, 1H), 3.81 (d, *J =* 5.6 Hz, 3H), 3.69 - 3.44 (m, 3H), 3.29 - 3.17 (m, 2H), 3.02 - 2.68 (m, 3H), 2.40 (d, *J =* 5.6 Hz, 6H). LCMS (ES, m/z): 435 [M+H]⁺.

### Example 5: Preparation of (5-(5-fluoro-4,6-dimethylpyrimidin-2-yl)-3,3a,4,6a-tetrahydrocyclopenta[c]pyrrol-2(1H)-yl)(4-methoxy-2-(2H-1,2,3-triazol-2-yl)phenyl)methanone (5)

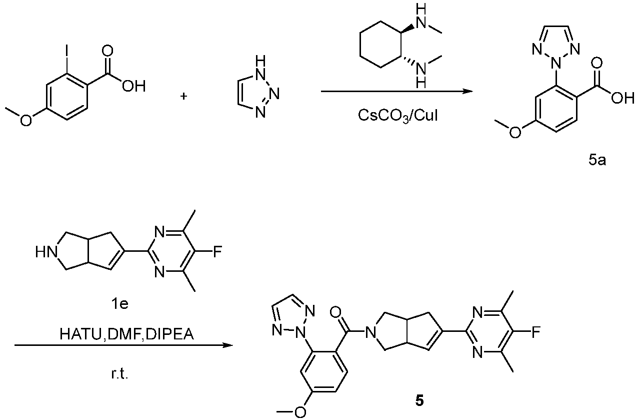

5.1 The reaction raw material 2-fluoro-6-iodobenzoic acid was replaced with 2-iodo-4-methoxybenzoic acid, and 4-methoxy-2-(2H-1,2,3-triazol-2-yl)benzoic acid (Compound 5a) was prepared according to the method of Example 1-1.6.

5.2 The reaction raw material 1f was replaced with 5a, and the (5-(5-fluoro-4,6-dimethylpyrimidin-2-yl)-3,3a,4,6a-tetrahydrocyclopenta[c]pyrrol-2(1H)-yl)(4-methoxy-2-(2H-1,2,3-triazol-2-yl)phenyl)methanone (Compound 5) was prepared according to the method of Example 1-1.7. ¹H NMR (400 MHz, DMSO-*d*₆) δ 7.95 (d, *J =* 26.3 Hz, 2H), 7.38 - 7.33 (m, 2H), 7.07 (td, *J =* 8.7, 2.5 Hz, 1H), 6.57 (d, *J =* 78.2 Hz, 1H), 3.87 (d, *J =* 5.6 Hz, 3H), 3.76 - 3.41 (m, 3H), 3.30 - 3.09 (m, 2H), 3.01 - 2.59 (m, 3H), 2.43 (s, 6H). LCMS (ES, m/z): 435 [M+H]⁺.

### Example 6: Preparation of (5-(5-fluoro-4,6-dimethylpyrimidin-2-yl)-3,3a,4,6a-tetrahydrocyclopenta[c]pyrrol-2(1H)-yl)(5-methyl-2-(2H-1,2,3-triazol-2-yl)phenyl)methanone (6)

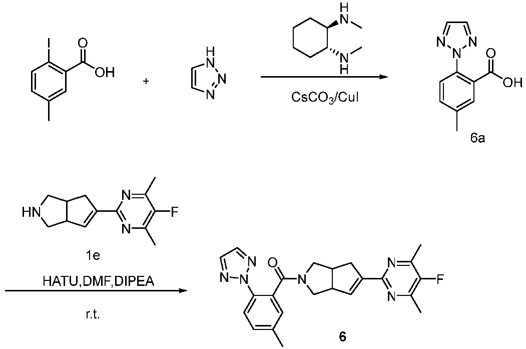

6.1 The reaction raw material 2-fluoro-6-iodobenzoic acid was replaced with 2-iodo-5-methylbenzoic acid, and 5-methyl-2-(2H-1,2,3-triazol-2-yl)benzoic acid (Compound 6a) was prepared according to the method of Example 1-1.6.

6.2 The reaction raw material 1f was replaced with 6a, and (5-(5-fluoro-4,6-dimethylpyrimidin-2-yl)-3,3a,4,6a-tetrahydrocyclopenta[c]pyrrol-2(1H)-yl)(5-methyl-2-(2H-1,2,3-triazol-2-yl)phenyl)methanone (Compound 6) was prepared according to the method of Example 1-1.7. ¹H NMR (400 MHz, DMSO-*d*₆) δ 7.90 (d, *J =* 24.5 Hz, 2H), 7.53 - 7.42 (m, 2H), 7.23 - 7.11 (m, 1H), 6.53 (d, *J =* 46.1 Hz, 1H), 3.63 - 3.41 (m, 3H), 3.34 - 3.22 (m, 2H), 3.13 - 2.64 (m, 3H), 2.56 (s, 3H), 2.38 (s 6H). LCMS (ES, m/z): 419 [M+H]⁺.

### Example 7: Preparation of (2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (7)

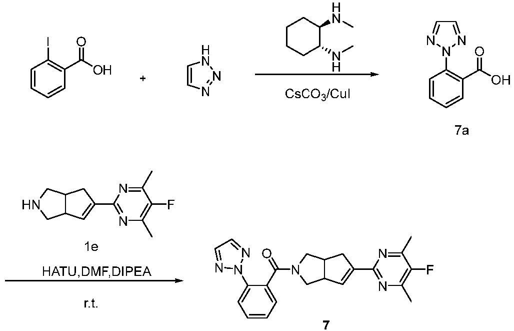

7.1 The reaction raw material 2-fluoro-6-iodobenzoic acid was replaced with 2-iodobenzoic acid, and 2-(2H-1,2,3-triazol-2-yl)benzoic acid (Compound 7a) was prepared according to the method of Example 1-1.6.

7.2 The reaction raw material 1f was replaced with 7a, and (2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (Compound 7) was prepared according to the method of Example 1-1.7. ¹H NMR (400 MHz, Chloroform-*d*) δ 8.04 - 8.01 (m, 1H), 7.83 - 7.75 (m, 2H), 7.59 - 7.53 (m, 1H), 7.50 - 7.45 (m, 2H), 6.67 (d, *J =* 114.0 Hz, 1H), 4.06 - 3.69 (m, 3H), 3.56 - 3.25 (m, 2H), 3.17 - 2.86 (m, 3H), 2.53 (s, 6H). LCMS (ES, m/z): 405 [M+H]⁺.

### Example 8: Preparation of (5-(5-fluoro-4-methoxy-6-methylpyrimidin-2-yl)-3,3a,4,6a-tetrahydrocyclopenta[c]pyrrol-2(1 H)-yl)(2-fluoro-6-(2H-1,2,3-triazol-2-yl)phenyl)methanone (8)

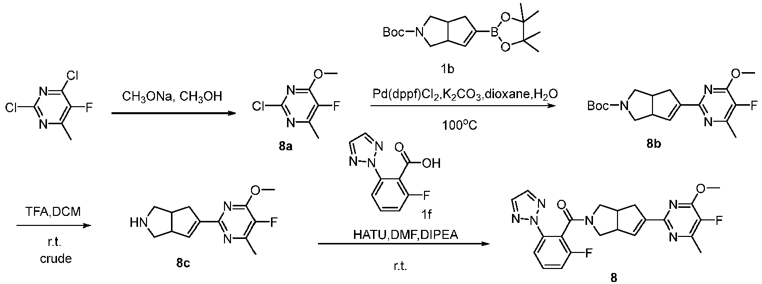

8.1 2,4-Dichloro-5-fluoro-6-methylpyrimidine (3 g, 16.58 mmol) and sodium methoxide (1.0 g, 18.51 mmol) were added to 10 mL of methanol and reacted at room temperature for 1 hour. After the reaction was completed, the reaction solution was concentrated under reduced pressure, separated and purified by column chromatography, and eluted with PE/EA (1:1) to obtain 2.1 g of 2-chloro-5-fluoro-4-methoxy-6-methylpyrimidine (Compound 8a).

8.2 The reaction raw material 1c was replaced with 8a, and tert-butyl 5-(5-fluoro-4-methoxy-6-methylpyrimidin-2-yl)-3,3a,4,6a-tetrahydrocyclopenta[c]pyrrol-2(1H)-car boxylate (Compound 8b) was prepared according to the method of Example 1-1.4.

8.3 The reaction raw material 1d was replaced with 8b, and 5-(5-fluoro-4-methoxy-6-methylpyrimidin-2-yl)-1,2,3,3 a,4,6a-hexahydrocyclopenta[c]pyrrole (Compound 8c) was prepared according to the method of Example 1-1.5.

8.4 The reaction raw material 1e was replaced with 8c, and (5-(5-fluoro-4-methoxy-6-methylpyrimidin-2-yl)-3,3a,4,6a-tetrahydrocyclopenta[c]pyrrol-2(1H)-yl)(2-fluoro-6-(2H-1,2,3-triazol-2-yl)phenyl)methanone (Compound 8) was prepared according to the method of Example 1-1.7. ¹H NMR (400 MHz, Chloroform-*d*) δ 8.03 - 7.78 (m, 2H), 7.68 - 7.49 (m, 2H), 7.31 - 7.14 (m, 1H), 6.71 - 6.62 (m, 1H), 4.04 (s, 3H), 3.98 - 3.85 (m, 1H), 3.73 - 3.32 (m, 3H), 3.19 - 2.83 (m, 3H), 2.58 (d, *J =* 16.7 Hz, 1H), 2.43 (dd, *J =* 11.0, 3.0 Hz, 3H). LCMS (ES, m/z): 439 [M+H]⁺.

### Example 9: Preparation of 2-fluoro-6-(2H-1,2,3-triazol-2-yl)phenyl)(5-(4,5,6-trimethylpyrimidin-2-yl)-3,3a,4,6a-tetrahydrocyclopenta[c]pyrrol-2(1H)-yl)methanone (9)

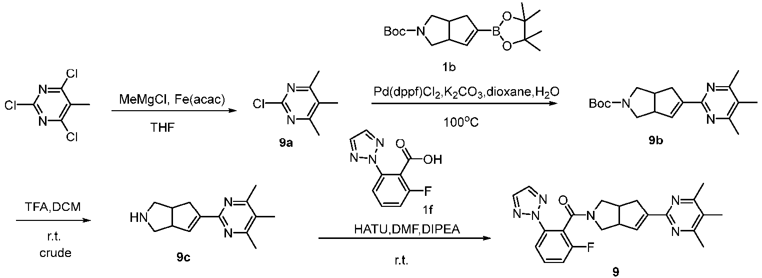

9.1 The reaction raw material 2,4,6-trichloro-5-fluoropyrimidine was replaced with 2,4,6-trichloro-5-methylpyrimidine, and 2-chloro-4,5,6-trimethylpyrimidine (Compound 9a) was prepared according to the method of Example 1-1.3.

9.2 The reaction raw material 1c was replaced with 9a, and tert-butyl 5-(4,5,6-trimethylpyrimidin-2-yl)-3,3a,4,6a-tetrahydrocyclopenta[c]pyrrole-2(1H)-carboxylate (Compound 9b) was prepared according to the method of Example 1-1.4.

9.3 The reaction raw material 1d was replaced with 9b, and 5-(4,5,6-trimethylpyrimidin-2-yl)-1,2,3,3a,4,6a-hexahydrocyclopenta[c]pyrrole (Compound 9c) was prepared according to the method of Example 1-1.5.

9.4 The reaction raw material 1e was replaced with 9c, and (2-fluoro-6-(2H-1,2,3-triazol-2-yl)phenyl)(5-(4,5,6-trimethylpyrimidin-2-yl)-3,3a,4,6a-tetrahydrocyclopenta[c]pyrrol-2(1H)-yl)methanone (Compound 9) was prepared according to the method of Example 1-1.7. ¹H NMR (400 MHz, Chloroform-*d*) δ 7.87 - 7.76 (m, 2H), 7.64 (d, *J =* 26.4 Hz, 1H), 7.48 - 7.42 (m, 1H), 7.23 - 6.94 (m, 1H), 6.88 - 6.43(m, 1H), 3.77 - 3.30 (m, 3H), 3.23 - 2.92 (m, 2H), 2.81 - 2.58 (m, 3H), 2.57 - 2.31 (m, 9H). LCMS (ES, m/z): 419 [M+H]⁺.

### Example 10: Preparation of (5-(5-chloro-4,6-dimethylpyrimidin-2-yl)-3,3a,4,6a-tetrahydrocyclopenta[c]pyrrol-2(1H)-yl)(2-fluoro-6-(2H-1,2,3-triazol-2-yl)phenyl)methanone (10)

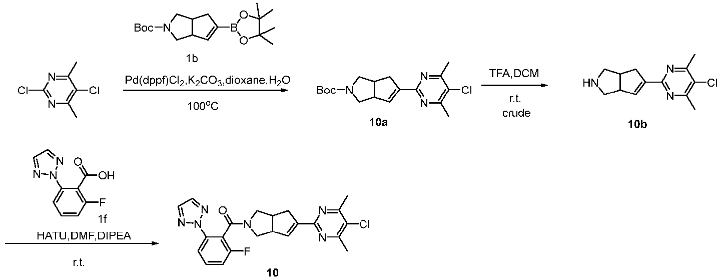

10.1 The reaction raw material 1c was replaced with 2,5-dichloro-4,6-dimethylpyrimidine, and tert-butyl 5-(5-chloro-4,6-dimethylpyrimidin-2-yl)-3,3a,4,6a-tetrahydrocyclopenta[c]pyrrole-2(1H)-carboxylate (Compound 10a) was prepared according to the method of Example 1-1.4.

10.2 The reaction raw material 1d was replaced with 10a, and 5-(5-chloro-4,6-dimethylpyrimidin-2-yl)-1,2,3,3a,4,6a-hexahydrocyclopenta[c]pyrrole (Compound 10b) was prepared according to the method of Example 1-1.5.

10.3 The reaction raw material 1e was replaced with 9c, and (5-(5-chloro-4,6-dimethylpyrimidin-2-yl)-3,3a,4,6a-tetrahydrocyclopenta[c]pyrrol-2(1H)-yl)(2-fluoro-6-(2H-1,2,3-triazol-2-yl)phenyl)methanone (Compound 10) was prepared according to the method of Example 1-1.7. ¹H NMR (400 MHz, Chloroform-*d*) δ 7.94 - 7.72 (m, 2H), 7.61 (d, *J =* 25.4 Hz, 1H), 7.46 (h, *J =* 6.0, 5.4 Hz, 1H), 7.12 (dt, *J =* 16.2, 8.5 Hz, 1H), 6.92 - 6.43 (m, 1H), 4.18 - 3.78 (m, 2H), 3.75 - 3.50 (m, 2H), 3.50 - 3.32 (m, 1H), 3.23 - 2.83 (m, 3H), 2.74 - 2.41 (m, 6H). LCMS (ES, m/z): 439 [M+H]⁺.

### Example 11: Preparation of (5-(5-ethyl-4,6-dimethylpyrimidin-2-yl)-3,3a,4,6a-tetrahydrocyclopenta[c]pyrrol-2(1H)-yl)(2-fluoro-6-(2H-1,2,3-triazol-2-yl)phenyl)methanone (11)

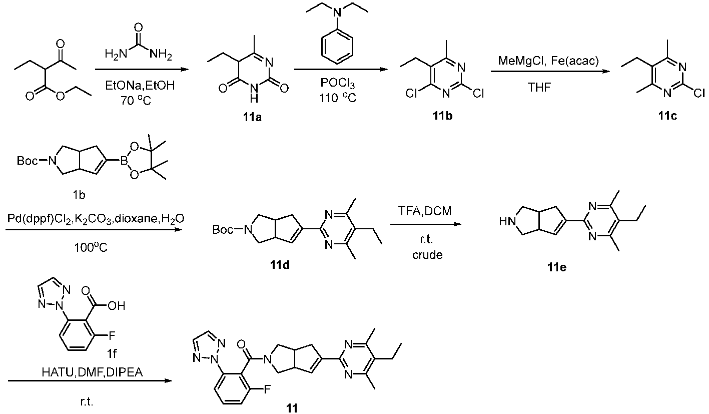

11.1 Ethyl 2-ethyl-3-oxobutyrate (5.0 g, 31.61 mmol) and urea (1.9 g, 31.67 mmol) were added to 60 mL of ethanol, and EtONa (4.3 g, 63.24 mmol) was added under nitrogen protection. The temperature was raised to 70 °C and the reaction was carried out for 7 hours. After the reaction was completed, the reaction solution was cooled to room temperature, and extracted with EtOAc (3 × 50 mL). The organic phases were combined, washed with water (2 × 20 mL), dried with anhydrous sodium sulfate, filtered, and the filtrate was concentrated under reduced pressure, separated and purified by column chromatography and eluted with PE/EA (10:1) to obtain 2.1 g of 5-ethyl-6-methylpyrimidine-2,4(3H,5H)-dione (Compound 11a).

11.2 11a (1.8 g, 11.68 mmol) and diethylaniline (2 mL) were added to phosphorus oxychloride (20 mL), and the mixture was reacted at 110 °C for 3 hours under nitrogen protection. After the reaction was completed, the reaction solution was cooled to room temperature, adjusted to pH=7 with saturated Na₂CO₃ solution, and then extracted with EtOAc (3 × 10 mL). The organic phases were combined, washed with water (2 × 5 mL), dried over anhydrous sodium sulfate, filtered, and the filtrate was concentrated, separated and purified by column chromatography and eluted with PE/EA (3:1) to obtain 1.2 g of 2,4-dichloro-5-ethyl-6-methylpyrimidine (Compound 11b).

11.3 The reaction raw material 2,4,6-trichloro-5-fluoropyrimidine was replaced with 11b, and 2-chloro-5-ethyl-4,6-dimethylpyrimidine (Compound 11c) was prepared according to the method of Example 1-1.3.

11.4 The reaction raw material 1c was replaced with 11c, and tert-butyl 5-(5-ethyl-4,6-dimethylpyrimidin-2-yl)-3,3a,4,6a-tetrahydrocyclopenta[c]pyrrole-2(1H)-carboxylate (Compound 11d) was prepared according to the method of Example 1-1.4.

11.5 The reaction raw material 1d was replaced with 11d, and 5-(5-ethyl-4,6-dimethylpyrimidin-2-yl)-1,2,3,3a,4,6a-hexahydrocyclopenta[c]pyrrole (Compound 11e) was prepared according to the method of Example 1-1.5.

11.6 The reaction raw material 1e was replaced with 11e, and (5-(5-ethyl-4,6-dimethylpyrimidin-2-yl)-3,3a,4,6a-tetrahydrocyclopenta[c]pyrrol-2(1H)-yl)(2-fluoro -6-(2H-1,2,3-triazol-2-yl)phenyl)methanone (Compound 11) was prepared according to the method of Example 1-1.7. ¹H NMR (400 MHz, Chloroform-*d*) δ 7.92 - 7.74 (m, 2H), 7.61 (d, *J =* 31.4 Hz, 1H), 7.46 (ttd, *J =* 8.4, 6.0, 3.1 Hz, 1H), 7.21 - 6.97 (m, 1H), 6.90 - 6.46 (m, 1H), 4.20 - 3.77 (m, 1H), 3.75 - 3.30 (m, 3H), 3.27 - 2.83 (m, 3H), 2.81 - 2.58 (m, 3H), 2.57 - 2.42 (m, 6H), 1.23 - 1.06 (m, 3H).LCMS (ES, m/z): 433 [M+H]⁺.

### Example 12: Preparation of (4-ethoxy-2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (12)

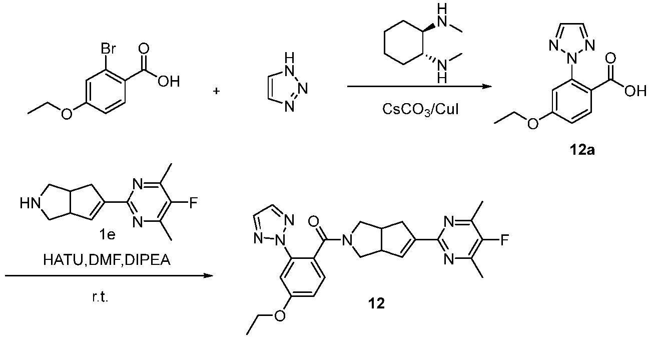

12.1 The reaction raw material 2-fluoro-6-iodobenzoic acid was replaced with 2-bromo-4-ethoxybenzoic acid, and 4-ethoxy-2-(2H-1,2,3-triazol-2-yl)benzoic acid (Compound 12a) was prepared according to the method of Example 1-1.6.

12.2 The reaction raw material 1f was replaced with 12a, and (4-ethoxy-2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (Compound 12) was prepared according to the method of Example 1-1.7. ¹H NMR (400 MHz, DMSO-*d*₆) δ 7.91 (d, *J =* 24.8 Hz, 2H), 7.33 - 7.28 (m, 2H), 7.09 - 7.05 (m, 1H), 6.52 (d, *J =* 76.4 Hz, 1H), 3.87 - 3.78 (m, 2H), 3.73 - 3.39 (m, 3H), 3.31 - 3.07 (m, 2H), 3.01 - 2.59 (m, 3H), 2.38 (s, 6H), 1.19 - 1.04 (m, 3H). LCMS (ES, m/z): 449 [M+H]⁺.

### Example 13: Preparation of (4-cyclopropyloxy-2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (13)

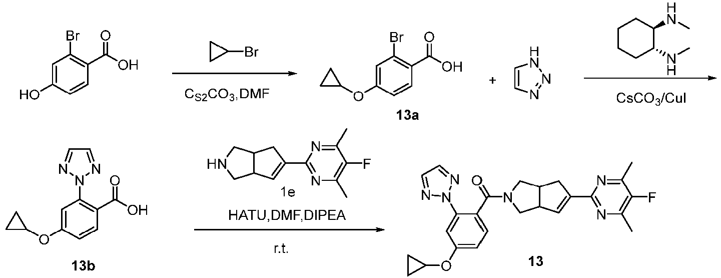

13.1 2-Bromo-4-hydroxybenzoic acid (5.0 g, 23.04 mmol), bromocyclopropane (8.4 g, 69.43 mmol) and cesium carbonate (15.0 g, 46.01 mmol) were added to 30 mL of DMF, and the tube was sealed for reaction. The temperature was raised to 100 °C and the reaction was carried out for 10 hours. After the reaction was completed, the reaction solution was cooled to room temperature, followed by the addition of 150 mL of water, and then extracted with EtOAc (3 × 50 mL). The organic phases were combined, washed with water (2 × 30 mL), dried over anhydrous sodium sulfate, filtered, and the filtrate was concentrated under reduced pressure, separated and purified by column chromatography, and eluted with PE/EA (1:1) to obtain 1.1 g of 2-bromo-4-cyclopropyloxybenzoic acid (Compound 13a).

13.2 The reaction raw material 2-fluoro-6-iodobenzoic acid was replaced with 13a, and 4-cyclopropyloxy-2-(2H-1,2,3-triazol-2-yl)benzoic acid (Compound 13b) was prepared according to the method of Example 1-1.6.

13.3 The reaction raw material 1f was replaced with 13b, and (4-cyclopropyloxy-2-(2H-1,2,3-triazol-2-yl)phenyl)(5-(5-fluoro-4,6-dimethylpyrimidin-2-yl)-3,3a,4,6a-tetrahydrocyclopenta[c]pyrrol-2(1H)-yl)methanone (Compound 13) was prepared according to the method of Example 1-1.7. ¹H NMR (400 MHz, DMSO-*d*₆) δ 7.99 - 7.93 (m, 2H), 7.40 - 7.31 (m, 2H), 7.14 - 7.07 (m, 1H), 6.46 (d, *J =* 76.4 Hz, 1H), 4.13 - 4.06 (m, 1H), 3.68 - 3.35 (m, 3H), 3.21 - 3.04 (m, 2H), 2.99 - 2.56 (m, 3H), 2.34 (s, 6H), 1.18 - 1.04 (m, 4H). LCMS (ES, m/z): 449 [M+H]⁺.

### Example 14: (2-fluoro-6-(2H-1,2,3-triazol-2-yl)phenyl)(5-(4-methyl-5,6,7,8-tetrahydroquinazolin-2-yl)-3,3a, 4,6a-tetrahydrocyclopenta[c]pyrrol-2(1H)-yl)methanone (14)

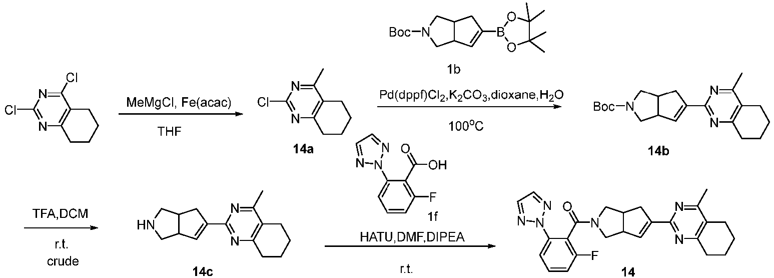

14.1 The reaction raw material 2,4,6-trichloro-5-fluoropyrimidine was replaced with 2,4-dichloro-5,6,7,8-tetrahydroquinazoline, and 2-chloro-4-methyl-5,6,7,8-tetrahydroquinazoline (Compound 14a) was prepared according to the method of Example 1-1.3.

14.2 The reaction raw material 1c was replaced with 14a, and tert-butyl 5-(4-methyl-5,6,7,8-tetrahydroquinazolin-2-yl)-3,3a,4,6a-tetrahydrocyclopenta[c]pyrrole-2(1H)-car boxylate (Compound 14b) was prepared according to the method of Example 1-1.4.

14.3 The reaction raw material 1d was replaced with 14b, and 5-(4-methyl-5,6,7,8-tetrahydroquinazolin-2-yl)-1,2,3,3a,4,6a-hexahydrocyclopenta[c]pyrrole (Compound 14c) was prepared according to the method of Example 1-1.5.

14.4 The reaction raw material 1e was replaced with 14c, and (2-fluoro-6-(2H-1,2,3-triazol-2-yl)phenyl)(5-(4-methyl-5,6,7,8-tetrahydroquinazolin-2-yl)-3,3a,4,6a -tetrahydrocyclopenta[c]pyrrol-2(1H)-yl)methanone (Compound 14) was prepared according to the method of Example 1-1.7. ¹H NMR (400 MHz, Methanol-*d*₄) δ 7.92 (d, *J* = 2.0 Hz, 1H), 7.88 - 7.74 (m, 1H), 7.71 - 7.56 (m, 2H), 7.32 - 7.23 (m, 1H), 6.77 - 6.48 (m, 1H), 4.00 - 3.53 (m, 3H), 3.46 - 3.42 (m, 2H), 3.22 - 2.96 (m, 2H), 3.02 - 2.84 (m, 5H), 2.41 - 2.33 (m, 3H), 2.03 (m, 4H). LCMS (ES, m/z): 445 [M+H]⁺.

### Example 15: Preparation of (2-fluoro-6-(2H-1,2,3-triazol-2-yl)phenyl)(5-(4-methyl-7,8-dihydro-6H-pyrano[3,2-d]pyrimidin -2-yl)-3,3a,4,6a-tetrahydrocyclopenta[c]pyrrol-2(1H)-yl)methanone (15)

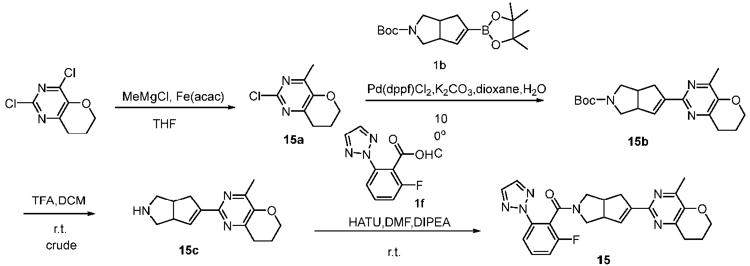

15.1 The reaction raw material 2,4,6-trichloro-5-fluoropyrimidine was replaced with 2,4-dichloro-7,8-dihydro-6H-pyrano[3,2-d]pyrimidine, and 2-chloro-4-methyl-7,8-dihydro-6H-pyrano[3,2-d]pyrimidine (Compound 15a) was prepared according to the method of Example 1-1.3.

15.2 The reaction raw material 1c was replaced with 15a, and tert-butyl 5-(4-methyl-7,8-dihydro-6H-pyrano[3,2-d]pyrimidin-2-yl)-3,3a,4,6a-tetrahydrocyclopenta[c] pyrrole-2(1H)-carboxylate (Compound 15b) was prepared according to the method of Example 1-1.4.

15.3 The reaction raw material 1d was replaced with 15b, and 5-(4-methyl-7,8-dihydro-6H-pyrano[3,2-d]pyrimidin-2-yl)-1,2,3,3a,4,6a-hexahydrocyclopenta[c] pyrrole (Compound 15c) was prepared according to the method of Example 1-1.5.

15.4 The reaction raw material 1e was replaced with 15c, and (2-fluoro-6-(2H-1,2,3-triazol-2-yl)phenyl)(5-(4-methyl-7,8-dihydro-6H-pyrano[3,2-d]pyrimidin-2-yl)-3,3a,4,6a-tetrahydrocyclopenta[c]pyrrol-2(1H)-yl)methanone (Compound 15) was prepared according to the method of Example 1-1.7. ¹H NMR (400 MHz, Chloroform-*d*) δ 7.91 - 7.78 (m, 2H), 7.54 (d, *J =* 25.6 Hz, 1H), 7.49 - 7.42 (m, 1H), 7.15 - 7.13 (m, 1H), 6.89 - 6.42 (m, 1H), 4.53 - 4.48 (m, 2H), 3.89 - 3.58 (m, 3H), 3.50 - 3.37 (m, 3H), 3.26 - 2.91 (m, 4H), 2.26 (s, 3H), 1.21 - 1.18 (m, 2H). LCMS (ES, m/z): 447 [M+H]⁺.

### Example 16: Preparation of (2-fluoro-6-(2H-1,2,3-triazol-2-yl)phenyl)(5-(4-methyl-7,8-dihydro-5H-pyrano[4,3-d]pyrimidin -2-yl)-3,3a,4,6a-tetrahydrocyclopenta[c]pyrrol-2(1H)-yl)methanone (16)

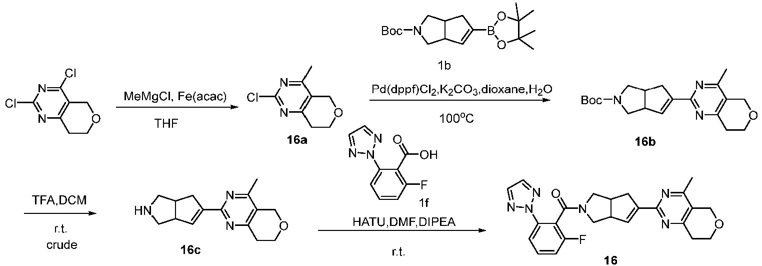

16.1 The reaction raw material 2,4,6-trichloro-5-fluoropyrimidine was replaced with 2,4-dichloro-7,8-dihydro-5H-pyrano[4,3-d]pyrimidine, and 2-chloro-4-methyl-7,8-dihydro-5H-pyrano[4,3-d]pyrimidine (Compound 16a) was prepared according to the method of Example 1-1.3.

16.2 The reaction raw material 1c was replaced with 16a, and tert-butyl 5-(4-methyl-7,8-dihydro-5H-pyrano[4,3-d]pyrimidin-2-yl)-3,3a,4,6a-tetrahydrocyclopenta[c] pyrrole-2(1H)-carboxylate (Compound 16b) was prepared according to the method of Example 1-1.4.

16.3 The reaction raw material 1d was replaced with 16b, and 5-(4-methyl-7,8-dihydro-5H-pyrano[4,3-d]pyrimidin-2-yl)-1,2,3,3a,4,6a-hexahydrocyclopenta[c] pyrrole (Compound 16c) was prepared according to the method of Example 1-1.5.

16.4 The reaction raw material 1e was replaced with 16c, and (2-fluoro-6-(2H-1,2,3-triazol-2-yl)phenyl)(5-(4-methyl-7,8-dihydro-5H-pyrano[4,3-d]pyrimidin-2-yl)-3,3a,4,6a-tetrahydrocyclopenta[c]pyrrol-2(1H)-yl)methanone (Compound 16) was prepared according to the method of Example 1-1.7. ¹H NMR (400 MHz, Chloroform-*d*) δ 7.96 - 7.69 (m, 2H), 7.61 (dd, *J =* 23.9, 1.8 Hz, 1H), 7.56 - 7.38 (m, 1H), 7.12 (dt, *J =* 16.1, 8.5 Hz, 1H), 6.93 - 6.42 (m, 1H), 4.73 (d, *J =* 8.7 Hz, 2H), 4.29 - 3.77 (m, 4H), 3.78 - 3.33 (m, 3H), 3.27 - 2.79 (m, 5H), 2.51 - 2.22 (m, 3H). LCMS (ES, m/z): 447 [M+H]⁺.

### Example 17: Preparation of (2-fluoro-6-(2H-1,2,3-triazol-2-yl)phenyl)(5-(4-methyl-6,7-dihydro-5H-cyclopenta[d]pyrimidin -2-yl)-3,3a,4,6a-tetrahydrocyclopenta[c]pyrrol-2(1H)-yl)methanone (17)

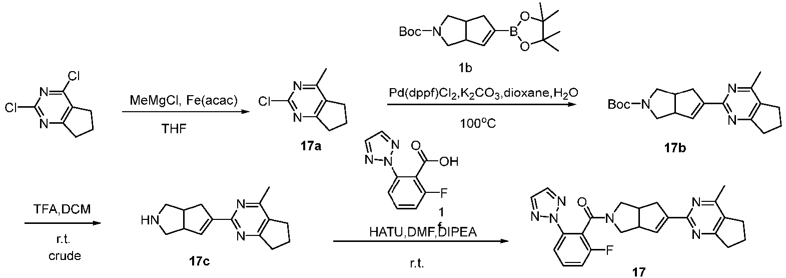

17.1 The reaction raw material 2,4,6-trichloro-5-fluoropyrimidine was replaced with 2,4-dichloro-6,7-dihydro-5H-cyclopenta[d]pyrimidine, and 2-chloro-4-methyl-6,7-dihydro-5H-cyclopenta[d]pyrimidine (Compound 17a) was prepared according to the method of Example 1-1.3.

17.2 The reaction raw material 1c was replaced with 17a, and tert-butyl 5-(4-methyl-6,7-dihydro-5H-cyclopenta[d]pyrimidin-2-yl)-3,3a,4,6a-tetrahydrocyclopenta[c]pyrrole -2(1H)-carboxylate (Compound 17b) was prepared according to the method of Example 1-1.4.

17.3 The reaction raw material 1d was replaced with 17b, and 5-(4-methyl-6,7-dihydro-5H-cyclopenta[d]pyrimidin-2-yl)-1,2,3,3a,4,6a-hexahydrocyclopenta[c] pyrrole (Compound 17c) was prepared according to the method of Example 1-1.5.

17.4 The reaction raw material 1e was replaced with 17c, and (2-fluoro-6-(2H-1,2,3-triazol-2-yl)phenyl)(5-(4-methyl-6,7-dihydro-5H-cyclopenta[d]pyrimidin-2-yl)-3,3a,4,6a-tetrahydrocyclopenta[c]pyrrol-2(1H)-yl)methanone (Compound 17) was prepared according to the method of Example 1-1.7. ¹H NMR (400 MHz, Methanol-*d*₄) δ 7.96 (d, *J =* 1.4 Hz, 1H), 7.92 - 7.79 (m, 1H), 7.73 - 7.56 (m, 2H), 7.33 - 7.21 (m, 1H), 6.81 - 6.51 (m, 1H), 4.00 - 3.53 (m, 3H), 3.44 (tdd, *J =* 12.1, 8.4, 3.7 Hz, 2H), 3.24 - 3.03 (m, 2H), 3.02 - 2.84 (m, 5H), 2.46 - 2.39 (m, 3H), 2.14 (h, *J* = 7.3 Hz, 2H). LCMS (ES, m/z): 431 [M+H]⁺.

### Example 18: Preparation of (2-fluoro-6-(2H-1,2,3-triazol-2-yl)phenyl)(5-(4-methylfuro[3,2-d]pyrimidin-2-yl)-3,3a,4,6a-tetrahydrocyclopenta[c]pyrrol-2(1H)-yl)methanone (18)

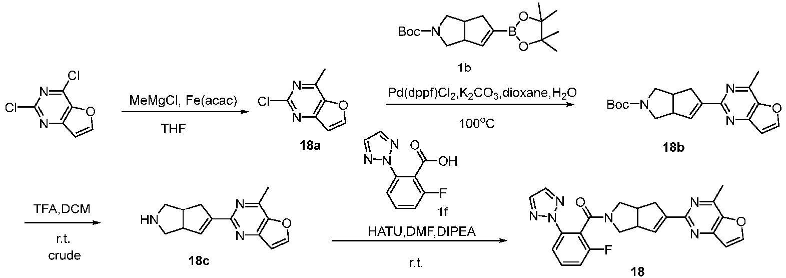

18.1 The reaction raw material 2,4,6-trichloro-5-fluoropyrimidine was replaced with 2,4-dichlorofuro[3,2-d]pyrimidine, and 2-chloro-4-methylfuro[3,2-d]pyrimidine (Compound 18a) was prepared according to the method of Example 1-1.3.

18.2 The reaction raw material 1c was replaced with 18a, and tert-butyl 5-(4-methylfuro[3,2-d]pyrimidin-2-yl)-3,3a,4,6a-tetrahydrocyclopenta[c]pyrrole-2(1H)-carboxylate (Compound 18b) was prepared according to the method of Example 1-1.4.

18.3 The reaction raw material 1d was replaced with 18b, and 5-(4-methylfuro[3,2-d]pyrimidin-2-yl)-1,2,3,3a,4,6a-hexahydrocyclopenta[c]pyrrole (Compound 18c) was prepared according to the method of Example 1-1.5.

18.4 The reaction raw material 1e was replaced with 18c, and (2-fluoro-6-(2H-1,2,3-triazol-2-yl)phenyl)(5-(4-methylfuro[3,2-d]pyrimidin-2-yl)-3,3a,4,6a-tetrahydrocyclopenta[c]pyrrol-2(1H)-yl)methanone (Compound 18) was prepared according to the method of Example 1-1.7. ¹H NMR (400 MHz, Methanol-*d*₄) δ 7.91 - 7.82 (m, 2H), 7.76 - 7.52 (m, 3H), 7.33 - 7.11 (m, 2H), 6.77 - 6.45 (m, 1H), 3.99 - 3.74 (m, 2H), 3.68 - 3.31 (m, 3H), 3.26 -2.96 (m, 3H), 2.38 (s, 3H). LCMS (ES, m/z): 431 [M+H]⁺.

### Example 19: Preparation of (2-fluoro-6-(2H-1,2,3-triazol-2-yl)phenyl)(5-(4-methyl-6,7-dihydrofuro[3,2-d]pyrimidin-2-yl)-3,3a,4,6a-tetrahydrocyclopenta[c]pyrrol-2(1H)-yl)methanone (19)

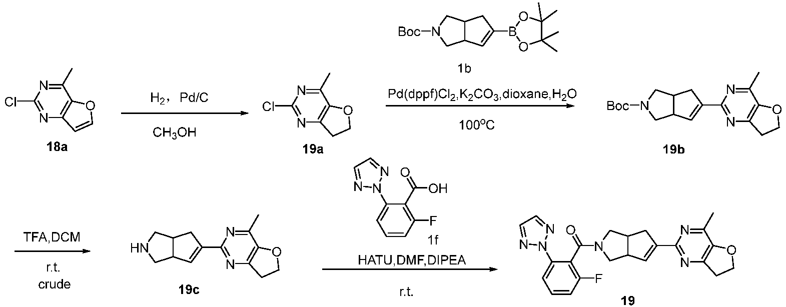

19.1 18a (2 g, 11.86 mmol), Pd/c (20%, 400 mg) and 10 mL of MeOH were added to a pressure-resistant reactor, and the reaction solution was hydrogenated at 50 °C under 5 psi hydrogen pressure for 6 hours. After the reaction was completed, it was filtered through diatomaceous earth and concentrated under reduced pressure. Column chromatography was used for separation and purification, and PE/EA (10:1) was used as the eluent to obtain 430 mg of 2-chloro-4-methyl-6,7-dihydrofuro[3,2-d]pyrimidine (Compound 19a).

19.2 The reaction raw material 1c was replaced with 19a, and tert-butyl 5-(4-methyl-6,7-dihydrofuro[3,2-d]pyrimidin-2-yl)-3,3a,4,6a-tetrahydrocyclopenta[c]pyrrole-2(1H) -carboxylate (Compound 19b) was prepared according to the method of Example 1-1.4.

19.3 The reaction raw material 1d was replaced with 19b, and 5-(4-methyl-6,7-dihydrofuro[3,2-d]pyrimidin-2-yl)-1,2,3,3a,4,6a-hexahydrocyclopenta[c]pyrrole (Compound 19c) was prepared according to the method of Example 1-1.5.

19.4 The reaction raw material 1e was replaced with 19c, and (2-fluoro-6-(2H-1,2,3-triazol-2-yl)phenyl)(5-(4-methyl-6,7-dihydrofuro[3,2-d]pyrimidin-2-yl)-3,3 a, 4,6a-tetrahydrocyclopenta[c]pyrrol-2(1H)-yl)methanone (Compound 19) was prepared according to the method of Example 1-1.7. ¹H NMR (400 MHz, Chloroform-d) δ 8.12 - 7.78 (m, 2H), 7.73 (s, 1H), 7.47 (tdd, J = 8.3, 5.9, 2.1 Hz, 1H), 7.14 (tdd, J = 8.4, 5.4, 1.0 Hz, 1H), 6.77 - 6.45 (m, 1H), 4.53 (t, J = 8.8 Hz, 2H), 4.12 - 3.76 (m, 2H), 3.73 - 3.41 (m, 5H), 3.32 - 3.13 (m, 3H), 2.21 (s, 3H). LCMS (ES, m/z): 433 [M+H]⁺.

### Example 20: Preparation of (2-fluoro-6-(2H-1,2,3-triazol-2-yl)phenyl)(5-(4-methyl-5,7-dihydrofuro[3,4-d]pyrimidin-2-yl)-3,3a,4,6a-tetrahydrocyclopenta[c]pyrrol-2(1H)-yl)methanone (20)

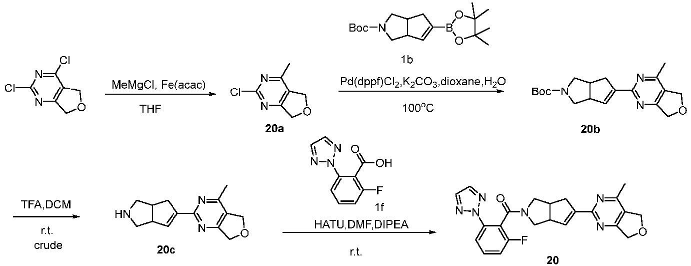

20.1 The reaction raw material 2,4,6-trichloro-5-fluoropyrimidine was replaced with 2,4-dichloro-5,7-dihydrofuro[3,4-d]pyrimidine, and 2-chloro-4-methyl-5,7-dihydrofuro[3,4-d]pyrimidine (Compound 20a) was prepared according to the method of Example 1-1.3.

20.2 The reaction raw material 1c was replaced with 20a, and tert-butyl 5-(4-methyl-5,7-dihydrofuro[3,4-d]pyrimidin-2-yl)-3,3a,4,6a-tetrahydrocyclopenta[c]pyrrole-2(1H) -carboxylate (Compound 20b) was prepared according to the method of Example 1-1.4.

20.3 The reaction raw material 1d was replaced with 20b, and 5-(4-methyl-5,7-dihydrofuro[3,4-d]pyrimidin-2-yl)-1,2,3,3a,4,6a-hexahydrocyclopenta[c]pyrrole (Compound 20c) was prepared according to the method of Example 1-1.5.

20.4 The reaction raw material 1e was replaced with 20c, and (2-fluoro-6-(2H-1,2,3-triazol-2-yl)phenyl)(5-(4-methyl-5,7-dihydrofuro[3,4-d]pyrimidin-2-yl)-3,3a, 4,6a-tetrahydrocyclopenta[c]pyrrol-2(1H)-yl)methanone (Compound 20) was prepared according to the method of Example 1-1.7. ¹H NMR (400 MHz, Methanol-*d*₄) δ 7.96 (s, 1H), 7.87 (td, *J =* 16.3, 15.7, 7.4 Hz, 1H), 7.74 - 7.57 (m, 2H), 7.35 - 7.22 (m, 1H), 6.90 - 6.59 (m, 1H), 5.19 - 5.13 (m, 2H), 4.98 (dt, *J =* 10.2, 2.9 Hz, 2H), 3.99 - 3.77 (m, 2H), 3.62 (tdd, *J =* 11.5, 8.4, 5.2 Hz, 1H), 3.51 - 3.39 (m, 1H), 3.24 - 2.87 (m, 3H), 2.74 - 2.57 (m, 1H), 2.48 - 2.42 (m, 3H). LCMS (ES, m/z): 433 [M+H]⁺.

### Example 21: Preparation of (2-fluoro-6-(2H-1,2,3-triazol-2-yl)phenyl)(5-(4-methylthieno[3,2-d]pyrimidin-2-yl)-3,3a,4,6a-tetrahydrocyclopenta[c]pyrrol-2(1H)-yl)methanone (21)

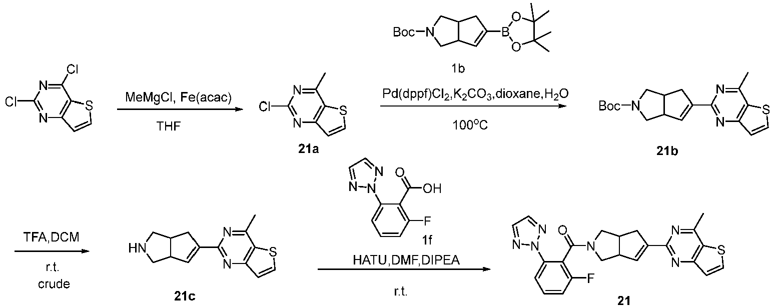

21.1 The reaction raw material 2,4,6-trichloro-5-fluoropyrimidine was replaced with 2,4-dichlorothieno[3,2-d]pyrimidine, and 2-chloro-4-methylthieno[3,2-d]pyrimidine (Compound 21a) was prepared according to the method of Example 1-1.3.

21.2 The reaction raw material 1c was replaced with 21a, and tert-butyl 5-(4-methylthieno[3,2-d]pyrimidin-2-yl)-3,3a,4,6a-tetrahydrocyclopenta[c]pyrrole-2(1H)-carboxyla te (Compound 21b) was prepared according to the method of Example 1-1.4.

21.3 The reaction raw material 1d was replaced with 21b, and 5-(4-methylthieno[3,2-d]pyrimidin-2-yl)-1,2,3,3a,4,6a-hexahydrocyclopenta[c]pyrrole (Compound 21c) was prepared according to the method of Example 1-1.5.

21.4 The reaction raw material 1e was replaced with 21c, and (2-fluoro-6-(2H-1,2,3-triazol-2-yl)phenyl)(5-(4-methylthieno[3,2-d]pyrimidin-2-yl)-3,3a,4,6a-tetrahydrocyclopenta[c]pyrrol-2(1H)-yl)methanone (Compound 21) was prepared according to the method of Example 1-1.7. ¹H NMR (400 MHz, Methanol-*d*₄) δ 7.96 - 7.86 (m, 2H), 7.83 - 7.61 (m, 3H), 7.32 - 7.14 (m, 2H), 6.71 - 6.48 (m, 1H), 3.91 - 3.76 (m, 2H), 3.63 - 3.28 (m, 3H), 3.23 -2.92 (m, 3H), 2.33 (s, 3H). LCMS (ES, m/z): 431 [M+H]⁺.

### Example 22: 5-(5-fluoro-4,6-dimethylpyrimidin-2-yl)-3,3a,4,6a-tetrahydrocyclopenta[c]pyrrol-2(1H)-yl)(6-methoxy-4-(2H-1,2,3-triazol-2-yl)pyridin-3-yl)methanone(22)

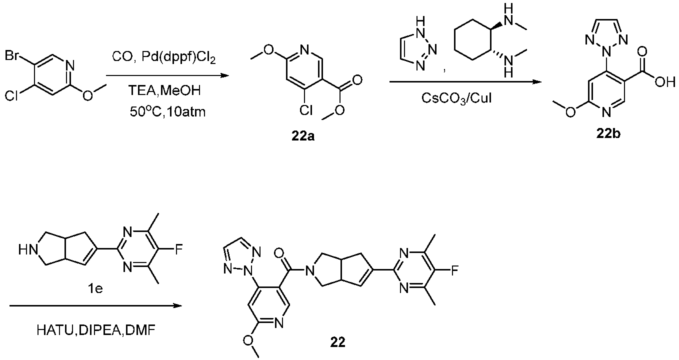

22.1 5-Bromo-4-chloro-2-methoxypyridine (500 mg, 2.247 mmol), TEA (682.3 mg, 6.741 mmol) and Pd(dppf)Cl₂ were added to methanol (5 mL). The reaction solution was added to a pressure resistant reactor, CO was introduced to a pressure of 10 atm, and the temperature was raised to 50 °C for overnight reaction. After the reaction was completed, the reaction solution was cooled to room temperature and extracted with EtOAc (3 x 20 mL). The organic phases were combined, washed with saturated brine (2 x 10 mL), dried over anhydrous Na₂SO₄, filtered, and the filtrate was concentrated under reduced pressure, purified by column chromatography and eluted with PE/EA (5:1) to obtain 183 mg of methyl 4-chloro-6-methoxypyridine-3-carboxylate (Compound 22a).

22.2 The reaction raw material 2-fluoro-6-iodobenzoic acid was replaced with 22a, and 6-methoxy-4-(2H-1,2,3-triazol-2-yl)nicotinic acid (Compound 22b) was prepared according to the method of Example 1-1.6.

22.3 The reaction raw material 1f was replaced with 22b, and 5-(5-fluoro-4,6-dimethylpyrimidin-2-yl)-3,3a,4,6a-tetrahydrocyclopenta[c]pyrrol-2(1H)-yl)(6-methoxy-4-(2H-1,2,3-triazol-2-yl)pyridin-3-yl)methanone (Compound 22) was prepared according to the method of Example 1-1.7. ¹H NMR (400 MHz, Methanol-*d*₄) δ 8.20 (d, *J =* 7.4 Hz, 1H), 7.89 (s, 2H), 7.39 (d, *J =* 11.5 Hz, 1H), 6.89 - 6.40 (m, 1H), 4.01 (d, *J =* 6.8 Hz, 3H), 3.96 - 3.79 (m, 2H), 3.69 - 3.45 (m, 3H), 3.18 - 2.87 (m, 3H), 2.46 (d, *J* = 6.6 Hz, 6H). LCMS (ES, m/z): 436 [M+H]⁺.

### Example 23: Preparation of (5-(5-fluoro-4,6-dimethylpyrimidin-2-yl)-3,3a,4,6a-tetrahydrocyclopenta[c]pyrrol-2(1H)-yl)(2-methoxy-5-(2H-1,2,3-triazol-2-yl)pyridin-4-yl)methanone (23)

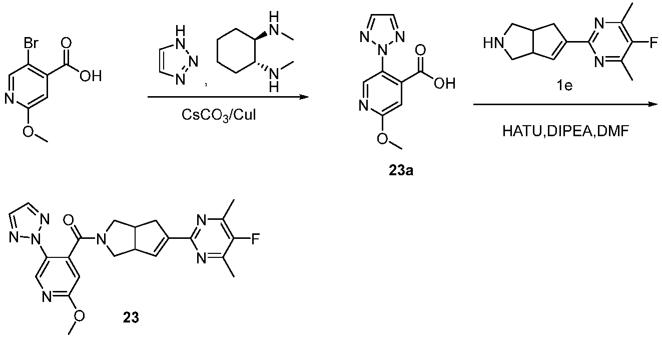

23.1 The reaction raw material 2-fluoro-6-iodobenzoic acid was replaced with 5-bromo-2-methoxyisonicotinic acid, and 2-methoxy-5-(2H-1,2,3-triazol-2-yl)isonicotinic acid (Compound 23a) was prepared according to the method of Example 1-1.6.

23.2 The reaction raw material 1f was replaced with 23a, and (5-(5-fluoro-4,6-dimethylpyrimidin-2-yl)-3,3a,4,6a-tetrahydrocyclopenta[c]pyrrol-2(1H)-yl)(2-methoxy-5-(2H-1,2,3-triazol-2-yl)pyridin-4-yl)methanone (Compound 23) was prepared according to the method of Example 1-1.7. ¹H NMR (400 MHz, Chloroform-*d*) δ 8.75 (d, *J* = 5.0 Hz, 1H), 7.70 (d, *J* = 13.0 Hz, 2H), 6.76 - 6.48 (m, 2H), 4.01 - 3.91 (m, 4H), 3.83 - 3.78 (m, 1H), 3.66 - 3.27 (m, 3H), 3.10 - 2.57 (m, 4H), 2.48 (dd, *J =* 5.5, 2.7 Hz, 6H). LCMS (ES, m/z): 436 [M+H]⁺.

### Example 24: Preparation of (3-(2H-1,2,3-triazol-2-yl)pyridin-2-yl)(5-(5-fluoro-4-methoxy-6-methylpyrimidin-2-yl)-3,3a,4, 6a-tetrahydrocyclopenta[c]pyrrol-2(1H)-yl)methanone (24)

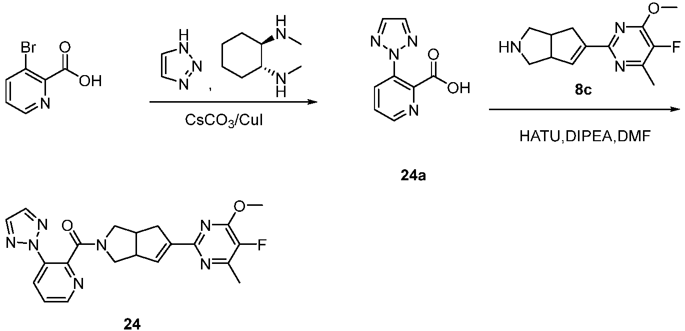

24.1 The reaction raw material 2-fluoro-6-iodobenzoic acid was replaced with 3-bromopicolinic acid, and 3-(2H-1,2,3-triazol-2-yl)picolinic acid (Compound 24a) was prepared according to the method of Example 1-1.6.

24.2 The reaction raw material 1f was replaced with 24a, and 1e was replaced with 8c, and (3-(2H-1,2,3-triazol-2-yl)pyridin-2-yl)(5-(5-fluoro-4-methoxy-6-methylpyrimidin-2-yl)-3,3a,4,6a-tetrahydrocyclopenta[c]pyrrol-2(1H)-yl)methanone (Compound 24) was prepared according to the method of Example 1-1.7. ¹H NMR (400 MHz, Chloroform-*d*) δ 8.76 - 8.54 (m, 1H), 8.33 - 8.24 (m, 1H), 7.86 (dd, *J* = 8.5, 3.2 Hz, 1H), 7.53 - 7.29 (m, 2H), 6.62 (d, *J =* 91.9 Hz, 1H), 4.04 (s, 3H), 3.98 - 3.85 (m, 1H), 3.73 - 3.32 (m, 3H), 3.19 - 2.83 (m, 3H), 2.58 (d, *J =* 16.7 Hz, 1H), 2.43 (dd, *J* = 11.0, 3.0 Hz, 3H). LCMS (ES, m/z): 422 [M+H]⁺.

### Example 25: Preparation of (5-(5-fluoro-4,6-dimethylpyrimidin-2-yl)-3,3a,4,6a-tetrahydrocyclopenta[c]pyrrol-2(1H)-yl)(6-methoxy-3-(2H-1,2,3-triazol-2-yl)pyridin-2-yl)methanone (25)

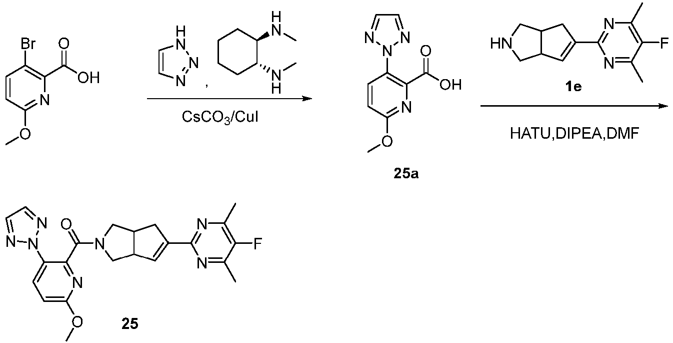

25.1 The reaction raw material 2-fluoro-6-iodobenzoic acid was replaced with 3-bromo-6-methoxypicolinic acid, and 6-methoxy-3-(2H-1,2,3-triazol-2-yl)picolinic acid (Compound 25a) was prepared according to the method of Example 1-1.6.

25.2 The reaction raw material 1f was replaced with 25a, and (5-(5-fluoro-4,6-dimethylpyrimidin-2-yl)-3,3a,4,6a-tetrahydrocyclopenta[c]pyrrol-2(1H)-yl)(6-methoxy-3-(2H-1,2,3-triazol-2-yl)pyridin-2-yl)methanone (Compound 25) was prepared according to the method of Example 1-1.7. ¹H NMR (400 MHz, Chloroform-*d*) δ 8.09 (dd, *J =* 8.7, 4.2 Hz, 1H), 7.79 (t, *J =* 3.5 Hz, 1H), 7.31 (dd, *J =* 10.7, 3.2 Hz, 1H), 7.18 - 7.11 (m, 1H), 6.89 - 6.84 (m, 1H), 4.16 - 3.94 (m, 4H), 3.89 - 3.40 (m, 4H), 3.23 - 2.88 (m, 3H), 2.61 (d, *J =* 8.4 Hz, 6H). LCMS (ES, m/z): 436 [M+H]⁺.

### Example 26: (5-fluoro-3-(2H-1,2,3-triazol-2-yl)pyridin-2-yl)(5-(5-fluoro-4-methoxy-6-methylpyrimidin-2-yl) -3,3a,4,6a-tetrahydrocyclopenta[c]pyrrol-2(1H)-yl)methanone (26)

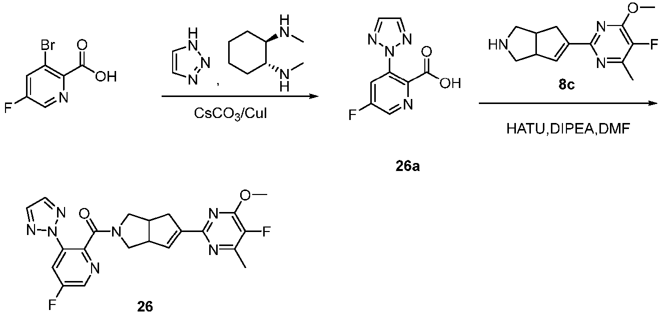

26.1 The reaction raw material 2-fluoro-6-iodobenzoic acid was replaced with 3-bromo-5-fluoropicolinic acid, and 5-fluoro-3-(2H-1,2,3-triazol-2-yl)picolinic acid (Compound 26a) was prepared according to the method of Example 1-1.6.

26.2 The reaction raw material 1f was replaced with 26a, and 1e was replaced with 8c, and (5-fluoro-3-(2H-1,2,3-triazol-2-yl)pyridin-2-yl)(5-(5-fluoro-4-methoxy-6-methylpyrimidin-2-yl)-3, 3a,4,6a-tetrahydrocyclopenta[c]pyrrol-2(1H)-yl)methanone (Compound 26) was prepared according to the method of Example 1-1.7. ¹H NMR (400 MHz, Methanol-*d*₄) δ 8.17 (dd, *J =* 8.6, 2.7 Hz, 1H), 7.96 - 7.92 (m, 1H), 7.81 (dd, *J =* 10.2, 3.2 Hz, 1H), 7.61 (dd, *J* = 3.2*,* 1.3 Hz, 1H), 6.84 - 6.37 (m, 1H), 4.04 (d, *J =* 3.8 Hz, 3H), 3.98 - 3.64 (m, 1H), 3.62 - 3.33 (m, 3H), 3.28 - 2.70 (m, 2H), 2.41 (dd, *J =* 10.0, 3.0 Hz, 3H). LCMS (ES, m/z): 440 [M+H]⁺.

### Example 27: (2-(2H-1,2,3-triazol-2-yl)phenyl)(5-(5-fluoro-4-methoxy-6-methylpyrimidin-2-yl)-3,3a,4,6a-tetrahydrocyclopenta[c]pyrrol-2(1H)-yl)methanone (27)

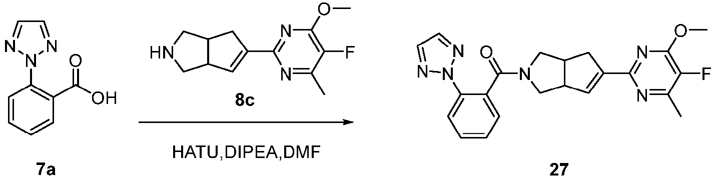

27.1 The reaction raw material 1f was replaced with 7a, and 1e was replaced with 8c, and (2-(2H-1,2,3-triazol-2-yl)phenyl)(5-(5-fluoro-4-methoxy-6-methylpyrimidin-2-yl)-3,3a,4,6a-tetrahydrocyclopenta[c]pyrrol-2(1H)-yl)methanone (Compound 27) was prepared according to the method of Example 1-1.7. ¹H NMR (400 MHz, Chloroform-*d*) δ 7.93 - 7.86 (m, 1H), 7.79 - 7.69 (m, 2H), 7.53 - 7.48 (m, 1H), 7.43 - 7.38 (m, 2H), 6.69 - 6.61 (m, 1H), 4.08 (s, 3H), 3.95 - 3.82 (m, 1H), 3.73 - 3.32 (m, 3H), 3.21 - 2.52 (m, 4H), 2.46 (d, *J =* 3.0 Hz, 3H). LCMS (ES, m/z): 421 [M+H]⁺.

### Example 28: Preparation of (5-(5-fluoro-4,6-dimethylpyrimidin-2-yl)-3,3a,4,6a-tetrahydrocyclopenta[c]pyrrol-2(1H)-yl)(2-fluoro-6-(4-methyl-2H-1,2,3-triazol-2-yl)phenyl)methanone (28)

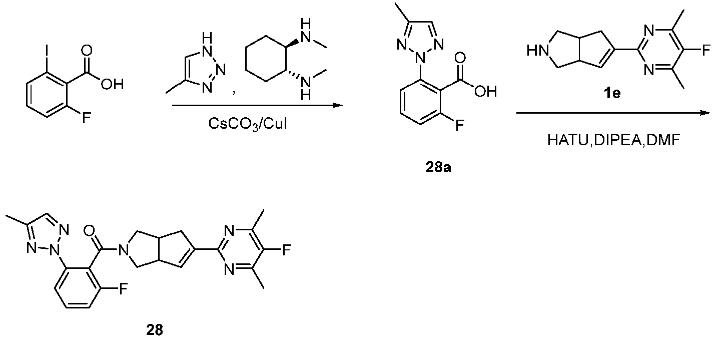

28.1 The reaction raw material 1,2,3-triazole was replaced with 4-methyl-1H-1,2,3-triazole, and 2-fluoro-6-(4-methyl-2H-1,2,3-triazol-2-yl)benzoic acid (Compound 28a) was prepared according to the method of Example 1-1.6.

28.2 The reaction raw material 1f was replaced with 28a, and (5-(5-fluoro-4,6-dimethylpyrimidin-2-yl)-3,3a,4,6a-tetrahydrocyclopenta[c]pyrrol-2(1H)-yl)(2-fluoro-6-(4-methyl-2H-1,2,3-triazol-2-yl)phenyl)methanone (Compound 28) was prepared according to the method of Example 1-1.7. ¹H NMR (400 MHz, Chloroform-*d*) δ 7.81 - 7.71 (m, 1H), 7.46 - 7.41 (m, 1H), 7.33 (s, 1H), 7.13 - 7.01 (m, 1H), 6.89 - 6.82 (m, 1H), 4.19 - 3.79 (m, 2H), 3.75 - 3.30 (m, 3H), 3.21 - 2.88 (m, 3H), 2.76 - 2.58 (m, 1H), 2.47 - 2.44 (m, 6H), 2.40 - 2.14 (m, 2H). LCMS (ES, m/z): 437 [M+H]⁺.

### Example 29: Preparation of (5-(5-fluoro-4,6-dimethylpyrimidin-2-yl)-3,3a,4,6a-tetrahydrocyclopenta[c]pyrrol-2(1H)-yl)(2-fluoro-6-(1H-pyrazol-3-yl)phenyl)methanone (29)

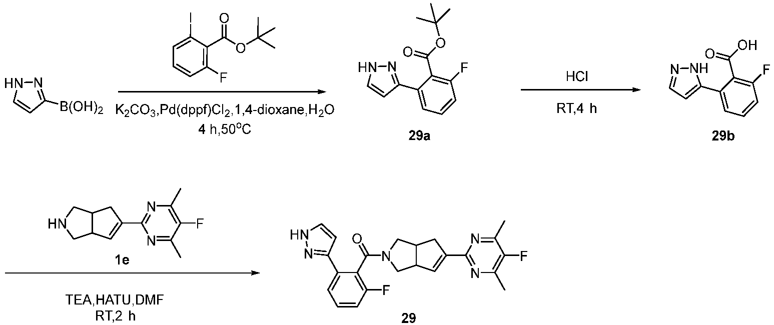

29.1 At room temperature, tert-butyl 2-fluoro-6-iodobenzoate (950 mg, 2.949 mmol) and 2H-pyrazol-3-yl boronic acid (396.0 mg, 3.539 mmol) were added to a mixed solution of 1.4-dioxane (6 mL) and water (1 mL), and K₂CO₃ (815.2 mg, 5.898 mmol) and Pd(dppf)Cl₂ (215.8 mg, 0.295 mmol) were added in batches. Under nitrogen protection, the temperature was raised to 50 °C and the reaction was carried out for 4 hours. The reaction solution was concentrated under reduced pressure, separated and purified by column chromatography, and eluted with PE/EA (10: 1) to obtain 210 mg of tert-butyl 2-fluoro-6-(2H-pyrazol-3-yl)benzoate (Compound 29a).

29.2 29a (87 mg, 0.332 mmol) was added to a 1,4-dioxane solution (5 mL) of HCl, reacted at room temperature for 4 hours, concentrated under reduced pressure, separated and purified by column chromatography, and eluted with PE/EA (3:1) to obtain 62 mg of 2-fluoro-6-(1H-pyrazol-3-yl)benzoic acid (Compound 29b).

29.3 The reaction raw material 1f was replaced with 29b, and (5-(5-fluoro-4, 6-dimethylpyrimidin-2-yl)-3,3 a,4,6a-tetrahydrocyclopenta[c]pyrrol-2(1H)-yl)(2-fluoro-6-(1H-pyrazol-3-yl)phenyl)methanone (Compound 29) was prepared according to the method of Example 1-1.7. ¹H NMR (400 MHz, Chloroform-*d*) δ 7.61 (d, *J* = 2.2 Hz, 1H), 7.54 - 7.27 (m, 3H), 7.17 - 6.98 (m, 1H), 6.76 - 6.38 (m, 2H), 4.03 - 3.76 (m, 1H), 3.67 - 2.96 (m, 5H), 2.95 - 2.64 (m, 2H), 2.63 - 2.37 (m, 6H). LCMS (ES, m/z): 422 [M+H]⁺.

### Example 30: (4-fluoro-2-(2H-1,2,3-triazol-2-yl)phenyl)(5-(5-fluoro-4,6-bis(methyl-d₃)pyrimidin-2-yl)-3,3a,4, 6a-tetrahydrocyclopenta[c]pyrrol-2(1H)-yl)methanone (30)

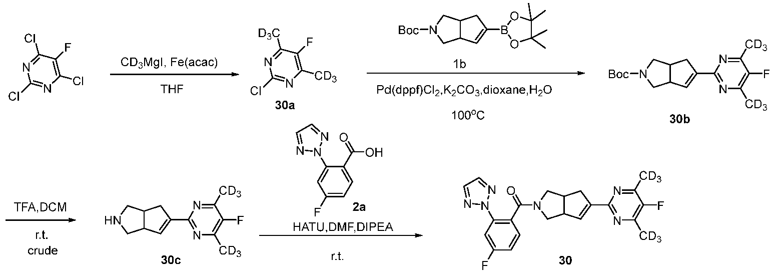

30.1 The reaction raw material methylmagnesium chloride was replaced with methyl-d₃-magnesium iodide, and 2-chloro-5-fluoro-4,6-bis(methyl-d₃)pyrimidine (Compound 30a) was prepared according to the method of Example 1-1.3.

30.2 The reaction raw material 1c was replaced with 30a, and tert-butyl 5-(5-fluoro-4,6-bis(methyl-d₃)pyrimidin-2-yl)-3,3a,4,6a-tetrahydrocyclopenta[c]pyrrole-2(1H) carboxylate (Compound 30b) was prepared according to the method of Example 1-1.4.

30.3 The reaction raw material 1d was replaced with 30b, and 5-(5-fluoro-4,6-bis(methyl-d₃)pyrimidin-2-yl)-1,2,3,3a,4,6a-hexahydrocyclopenta[c]pyrrole (Compound 30c) was prepared according to the method of Example 1-1.5.

30.4 The reaction raw material 1e was replaced with 30c, and 1f was replaced with 2a, and (4-fluoro-2-(2H-1,2,3-triazol-2-yl)phenyl)(5-(5-fluoro-4,6-bis(methyl-d₃)pyrimidin-2-yl)-3,3a,4,6a-tetrahydrocyclopenta[c]pyrrol-2(1H)-yl)methanone (Compound 30) was prepared according to the method of Example 1-1.7. ¹H NMR (400 MHz, DMSO-*d*₆) δ 8.15 - 7.92 (m, 2H), 7.74 - 7.70 (m, 1H), 7.55 - 7.44 (m, 1H), 7.43 - 7.35 (m, 1H), 6.58 (d, *J =* 80.4 Hz, 1H), 3.80 - 3.45 (m, 3H), 3.26 - 2.90 (m, 3H), 2.84 - 2.59 (m, 2H). LCMS (ES, m/z): 429 [M+H]⁺.

### Example 31: Preparation of (5-(5-fluoro-4,6-dimethylpyrimidin-2-yl)-3,3a,4,6a-tetrahydrocyclopenta[c]pyrrol-2(1H)-yl)(2-(methoxy-d₃)-5-(2H-1,2,3-triazol-2-yl)pyridin-4-yl)methanone(31)

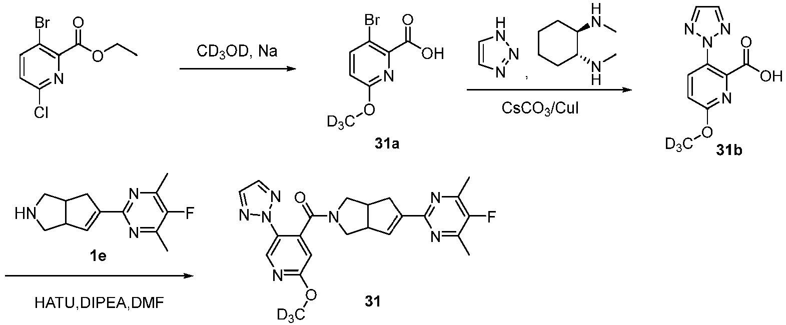

31.1 Sodium (1.3 g, 56.52 mmol) was added to deuterated methanol-d₄ (30 mL), stirred at room temperature for 10 minutes, ethyl 3-bromo-6-chloropicolinate (1.5 g, 5.67 mmol) was added thereto, and the temperature was raised to 60 °C and the reaction was carried out for 6 hours. After the reaction was completed, the reaction solution was concentrated under pressure, adjusted to pH 5-6 with citric acid solution, added with 100 mL of water, and extracted with EtOAc (3 x 20 mL). The organic phases were combined and dried over anhydrous sodium sulfate, filtered, and the filtrate was concentrated under reduced pressure to obtain 0.8 g of 3-bromo-6-(methoxy-d₃)picolinic acid (Compound 31a).

31.2 The reaction raw material 2-fluoro-6-iodobenzoic acid was replaced with 31a, and 6-(methoxy-d₃)-3-(2H-1,2,3-triazol-2-yl)picolinic acid (Compound 31b) was prepared according to the method of Example 1-1.6.

31.3 The reaction raw material 1f was replaced with 31b, and (5-(5-fluoro-4, 6-dimethylpyrimidin-2-yl)-3,3 a,4,6a-tetrahydrocyclopenta[c]pyrrol-2(1H)-yl)(2-(methoxy-d₃)-5-(2H-1,2,3-triazol-2-yl)pyridin-4-yl)methanone (Compound 31) was prepared according to the method of Example 1-1.7. ¹H NMR (400 MHz, Chloroform-*d*) δ 8.74 (d, *J =* 5.0 Hz, 1H), 7.72 (d, *J =* 13.0 Hz, 2H), 6.76 - 6.48 (m, 2H), 3.87 - 3.74 (m, 2H), 3.64 - 3.22 (m, 3H), 3.10 - 2.57 (m, 4H), 2.46 (dd, *J =* 5.5, 2.7 Hz, 6H). LCMS (ES, m/z): 439 [M+H]⁺.

### Example 32: Preparation of (5-(5-fluoro-4,6-bis(methyl-d₃)pyrimidin-2-yl)-3,3a,4,6a-tetrahydrocyclopenta[c]pyrrol-2(1H)-yl)(2-methoxy-5-(2H-1,2,3-triazol-2-yl)pyridin-4-yl)methanone (32)

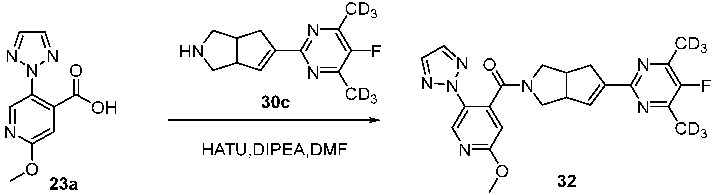

32.1 The reaction raw material 1f was replaced with 23a, and 1e was replaced with 30c, and (5-(5-fluoro-4,6-bis(methyl-d₃)pyrimidin-2-yl)-3,3a,4,6a-tetrahydrocyclopenta[c]pyrrol-2(1H)-yl)(2 -methoxy-5-(2H-1,2,3-triazol-2-yl)pyridin-4-yl)methanone (Compound 31) was prepared according to the method of Example 1-1.7. ¹H NMR (400 MHz, Chloroform-*d*) δ 8.78 (d, *J =* 5.0 Hz, 1H), 7.72 (d, *J =* 13.0 Hz, 2H), 6.74 - 6.42 (m, 2H), 4.06 - 3.86 (m, 4H), 3.83 - 3.78 (m, 1H), 3.66 - 3.27 (m, 3H), 3.10 - 2.57 (m, 4H). LCMS (ES, m/z): 442 [M+H]⁺.

### Example 33: (3-(2H-1,2,3-triazol-2-yl)pyridin-2-yl)(5-(5-fluoro-4-(methoxy-d₃)-6-methylpyrimidin-2-yl)-3, 3a,4,6a-tetrahydrocyclopenta[c]pyrrol-2(1H)-yl)methanone (33)

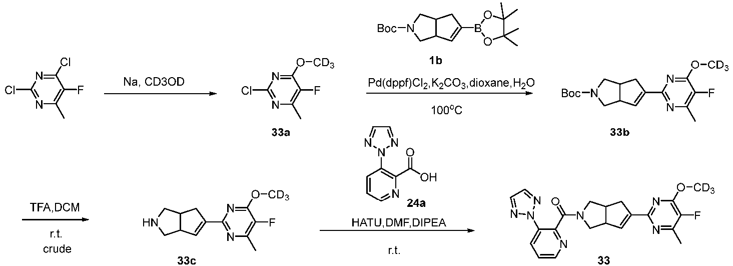

33.1 Sodium (1.5 g, 65.21 mmol) was added to deuterated methanol-d₄ (50 mL), stirred at room temperature for 10 minutes, 2,4-dichloro-5-fluoro-6-methylpyrimidine (1 g, 5.56 mmol) was added, the temperature was raised to 60 °C and the reaction was carried out for 8 hours. After the reaction was completed, the reaction solution was concentrated under pressure, adjusted to pH 5-6 with citric acid solution, added with 100 mL of water, and extracted with EtOAc (3 x 20 mL). The organic phases were combined and dried over anhydrous sodium sulfate, filtered, and the filtrate was concentrated under reduced pressure to obtain 0.76 g of 2-chloro-5-fluoro-4-(methoxy-d₃)-6-methylpyrimidine (Compound 33a).

33.2 The reaction raw material 1c was replaced with 33a, and tert-butyl 5-(5-fluoro-4-(methoxy-d₃)-6-methylpyrimidin-2-yl)-3,3a,4,6a-tetrahydrocyclopenta[c]pyrrole-2 (1H)carboxylate (Compound 33b) was prepared according to the method of Example 1-1.4.

33.3 The reaction raw material 1d was replaced with 33b, and 5-(5-fluoro-4-(methoxy-d₃)-6-methylpyrimidin-2-yl)-1,2,3,3a,4,6a-hexahydrocyclopenta[c]pyrrole (Compound 33c) was prepared according to the method of Example 1-1.5.

33.4 The reaction raw material 1e was replaced with 33c, and 1f was replaced with 24a, and (3-(2H-1,2,3-triazol-2-yl)pyridin-2-yl)(5-(5-fluoro-4-(methoxy-d₃)-6-methylpyrimidin-2-yl)-3,3a,4, 6a-tetrahydrocyclopenta[c]pyrrol-2(1H)-yl)methanone (Compound 33) was prepared according to the method of Example 1-1.7. ¹H NMR (400 MHz, Chloroform-*d*) δ 8.74 - 8.51 (m, 1H), 8.35 - 8.23 (m, 1H), 7.90 (dd, *J =* 8.5, 3.2 Hz, 1H), 7.54 - 7.28 (m, 2H), 6.62 (d, *J =* 91.9 Hz, 1H), 3.94 - 3.41 (m, 4H), 3.19 - 2.83 (m, 3H), 2.58 (d, *J =* 16.7 Hz, 1H), 2.41 (dd, *J =* 11.0, 3.0 Hz, 3H). LCMS (ES, m/z): 425 [M+H]⁺.

### Example 34: Preparation of (5-(5-fluoro-4,6-dimethylpyrimidin-2-yl)-3,3a,4,6a-tetrahydrocyclopenta[c]pyrrol-2(1H)-yl)(6-(methoxy-d₃)-4-(2H-1,2,3-triazol-2-yl)pyridin-3-yl)methanone (34)

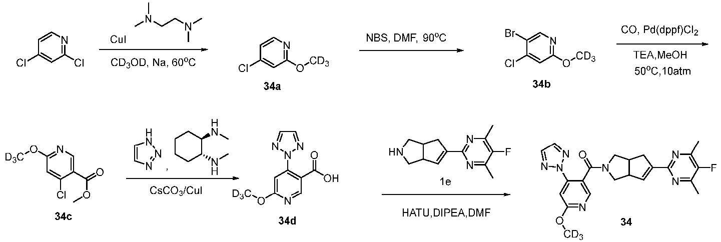

34.1 Sodium (1.5 g, 65.21 mmol) was added to deuterated methanol-d₄ (50 mL), stirred at room temperature for 10 minutes, 2,4-dichloropyridine (1.0 g, 6.76 mmol), CuI (0.2 g, 1.05 mmol), N,N,N',N'-tetramethylethylenediamine (0.7 g, 6.03 mmol) were added to the solution, and the temperature was raised to 60 °C for overnight reaction under nitrogen protection. After the reaction was completed, the reaction solution was concentrated under pressure, added with water, and extracted with EtOAc (3 x 30 mL). The organic phases were combined, dried over anhydrous sodium sulfate, filtered, and the filtrate was concentrated, separated and purified by column chromatography, and eluted with PE:EA=20:1 to obtain 0.6 g of 4-chloro-2-(methoxy-d₃)pyridine (Compound 34a).

34.2 34a (0.6 g, 4.08 mmol) and NBS (0.75 g, 4.21 mmol) were added to DMF (10 mL), and the temperature was raised to 90 °C and the reaction was carried out for 8 hours. After the reaction was completed, the reaction solution was diluted with water and extracted with EtOAc (3 x 20 mL). The organic phase was washed with saturated brine, dried over anhydrous sodium sulfate, filtered, and concentrated under reduced pressure to obtain 0.3 g of 5-bromo-4-chloro-2-(methoxy-d₃)pyridine (Compound 34b).

34.3 The reaction raw material 5-bromo-4-chloro-2-methoxypyridine was replaced with 34b, and methyl 4-chloro-6-(methoxy-d₃)nicotinate (Compound 34c) was prepared according to the method of Example 22-22.1.

34.4 The reaction raw material 2-fluoro-6-iodobenzoic acid was replaced with 34c, and 6-(methoxy-d₃)-4-(2H-1,2,3-triazol-2-yl)nicotinic acid (Compound 34d) was prepared according to the method of Example 1-1.6.

34.5 The reaction raw material 1f was replaced with 34d, and (5-(5-fluoro-4, 6-dimethylpyrimidin-2-yl)-3,3 a,4,6a-tetrahydrocyclopenta[c]pyrrol-2(1H)-yl)(6-(methoxy-d₃)-4-(2H-1,2,3-triazol-2-yl)pyridin-3-yl)methanone (Compound 34) was prepared according to the method of Example 1-1.7. ¹H NMR (400 MHz, Methanol-*d*₄) δ 8.21 (d, *J =* 7.4 Hz, 1H), 7.86 (s, 2H), 7.36 (d, *J =* 11.5 Hz, 1H), 6.84 - 6.38 (m, 1H), 3.93 - 3.77 (m, 2H), 3.69 - 3.45 (m, 3H), 3.20 - 2.82 (m, 3H), 2.44 (d, *J =* 6.6 Hz, 6H). LCMS (ES, m/z): 439 [M+H]⁺.

### Example 35: Preparation of (5-(5-fluoro-4,6-bis(methyl-d₃)pyrimidin-2-yl)-3,3a,4,6a-tetrahydrocyclopenta[c]pyrrol-2(1H)-yl)(6-methoxy-4-(2H-1,2,3-triazol-2-yl)pyridin-3-yl)methanone (35)

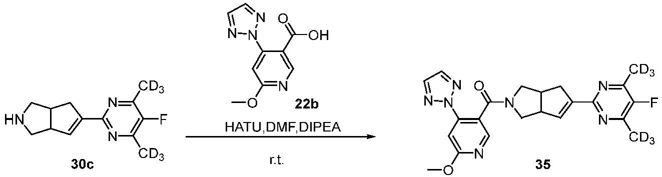

35.1 The reaction raw material 1f was replaced with 22b, and 1e was replaced with 30c, and (5-(5-fluoro-4,6-bis(methyl-d₃)pyrimidin-2-yl)-3,3a,4,6a-tetrahydrocyclopenta[c]pyrrol-2(1H)-yl)(6 -methoxy-4-(2H-1,2,3-triazol-2-yl)pyridin-3-yl)methanone (Compound 35) was prepared according to the method of Example 1-1.7. ¹H NMR (400 MHz, Methanol-*d*₄) δ 8.21 (d, *J =* 7.4 Hz, 1H), 7.83 (s, 2H), 7.37 (d, *J =* 11.5 Hz, 1H), 6.90 - 6.39 (m, 1H), 4.05 (d, *J =* 6.8 Hz, 3H), 3.98 - 3.76 (m, 2H), 3.63 - 3.41 (m, 3H), 3.16 - 2.81 (m, 3H). LCMS (ES, m/z): 442 [M+H]⁺.

### Example 36: Preparation of (5-(5-fluoro-4,6-dimethylpyrimidin-2-yl)-3,3a,4,6a-tetrahydrocyclopenta[c]pyrrol-2(1H)-yl)(4-(methoxy-d₃)-2-(2H-1,2,3-triazol-2-yl)phenyl)methanone (36)

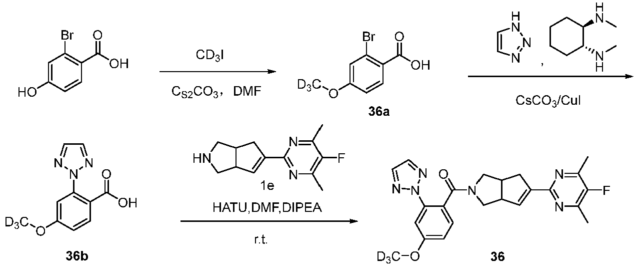

36.1 The raw material bromocyclopropane was replaced with deuterated iodomethane, and 2-bromo-4-(methoxy-d₃)benzoic acid (Compound 36a) was prepared according to the method of Example 13-13.1.

36.2 The raw material 2-fluoro-6-iodobenzoic acid was replaced with 36a, and prepare 4-(methoxy-d₃)-2-(2H-1,2,3-triazol-2-yl)benzoic acid (Compound 36b) according to the method of Example 1-1.6.

36.3 The raw material 1f was replaced with 36b, and (5-(5-fluoro-4,6-dimethylpyrimidin-2-yl)-3,3a,4,6a-tetrahydrocyclopenta[c]pyrrol-2(1H)-yl)(4-(methoxy-d₃)-2-(2H-1,2,3-triazol-2-yl)phenyl)methanone (Compound 36) was prepared according to the method of Example 1-1.7. ¹H NMR (400 MHz, DMSO-*d*₆) δ 7.91 (d, *J =* 26.3 Hz, 2H), 7.41 - 7.34 (m, 2H), 7.09 - 7.02 (m, 1H), 6.55 (d, *J =* 78.0 Hz, 1H), 3.72 - 3.38 (m, 3H), 3.31 - 3.02 (m, 2H), 2.97 - 2.52 (m, 3H), 2.43 (s, 6H). LCMS (ES, m/z): 438 [M+H]⁺.

### Example 37: Preparation of (5-(5-fluoro-4-(2-hydroxypropan-2-yl)-6-methylpyrimidin-2-yl)-3,3a,4,6a-tetrahydrocyclopenta[c]pyrrol-2(1H)-yl)(2-fluoro-6-(2H-1,2,3-triazol-2-yl)phenyl)methanone (37)

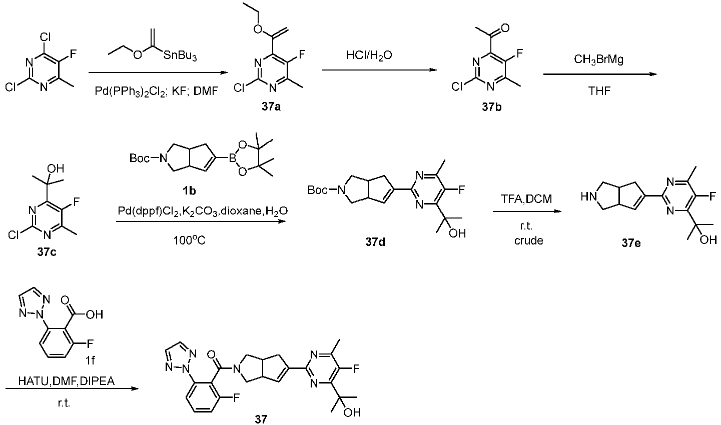

37.1 Under nitrogen protection, 2,4-dichloro-5-fluoro-6-methylpyrimidine (6.5 g, 35.9 mmol) was added to anhydrous DMF (15 mL) solution, and tributyl-(1-ethoxy-ethenyl)-stannane (14.2 g, 39.3 mmol) and dichlorobis(triphenylphosphine)palladium(II) (500 mg, 0.71 mmol) were added. The mixture was reacted at 100 °C for 10 hours, cooled to room temperature, a saturated solution of potassium fluoride was added and the mixture was stirred at room temperature for 1 hour. After the reaction was completed, the reaction solution was filtered through diatomaceous earth and extracted with ethyl acetate (3 x 50 mL), the organic phase was washed with water, dried over anhydrous sodium sulfate, filtered, and the filtrate was concentrated under reduced pressure. Column chromatography for separation and purification (PE:EA=10:1) was used to obtain 2-chloro-4-(1-ethoxyethenyl)-5-fluoro-6-methylpyrimidine (6.8 g) (Compound 37a).

37.2 37a (6.0 g, 27.8 mmol) was dissolved in THF (10 mL), and 3 N HCl solution (15 mL) was added. The reaction was allowed to react at room temperature for 1 hour. When the reaction was complete, the reaction solution was adjusted to pH 7-8 with saturated NaHCO₃, and extracted with ethyl acetate (3 x 50 mL). The organic phase was washed with water, dried over anhydrous sodium sulfate, filtered, and the filtrate was concentrated to obtain 1-(2-chloro-5-fluoro-6-methylpyrimidin-4-yl)ethan-1-one (5.1 g) (Compound 37b). The crude product was directly subjected to the next step without purification.

37.3 The reaction raw material methyl 2-chloro-6-methylpyrimidine-4-carboxylate was replaced with 37b, and 2-(2-chloro-5-fluoro-6-methylpyrimidin-4-yl)propan-2-ol (Compound 37c) was prepared according to the method of Example 30-30.1.

37.4 The reaction raw material 1c was replaced with 37c, and tert-butyl 5-[5-fluoro-4-(2-hydroxypropyl)-6-methylpyrimidin-2-yl]-3,3 a,4, 6a-tetrahydrocyclopenta[c]pyrrole -2(1H)-carboxylate (Compound 37d) was prepared according to the method of Example 1-1.4.

37.5 The reaction raw material 1d was replaced with 37d, and 5-[5-fluoro-4-(2-hydroxypropyl)-6-methylpyrimidin-2-yl]-1,2,3,3 a,4, 6a-hexahydrocyclopenta[c] pyrrole (Compound 37e) was prepared according to the method of Example 1-1.5.

37.6 The reaction raw material 1e was replaced with 37e, and (5-(5-fluoro-4-(2-hydroxypropan-2-yl)-6-methylpyrimidin-2-yl)-3,3 a,4, 6a-tetrahydrocyclopenta[c] pyrrol-2(1H)-yl)(2-fluoro-6-(2H-1,2,3-triazol-2-yl)phenyl)methanone (Compound 37) was prepared according to the method of Example 1-1.7. ¹H NMR (400 MHz, Chloroform-*d*) δ 7.89 - 7.71 (m, 2H), 7.63 - 7.48 (m, 2H), 7.35 - 7.18 (m, 1H), 6.69 - 6.42 (m, 1H), 3.99 - 3.74 (m, 2H), 3.66 - 3.61 (m, 2H), 3.49 - 3.02 (m, 3H), 3.00 - 2.82 (m, 1H), 2.38 (d, *J =* 2.9 Hz, 3H), 1.53 (s, 6H). LCMS (ES, m/z): 467 [M+H]⁺.

### Example 38: Preparation of (2-fluoro-6-(2H-1,2,3-triazol-2-yl)phenyl)(5-(5-(fluoromethyl)-4,6-dimethylpyrimidin-2-yl)-3, 3a,4,6a-tetrahydrocyclopenta[c]pyrrol-2(1H)-yl)methanone (38)

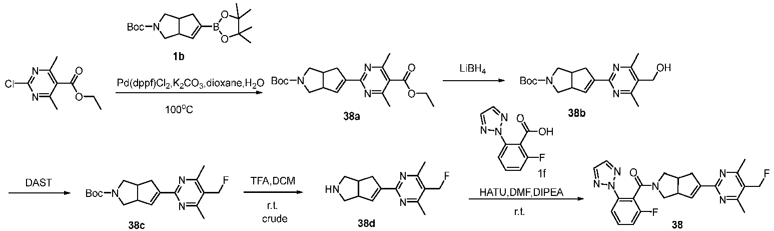

38.1 The reaction raw material 1c was replaced with ethyl 2-chloro-4,6-dimethylpyrimidine-5-carboxylate, and tert-butyl 5-(5-(ethoxycarbonyl)-4,6-dimethylpyrimidin-2-yl)-3,3a,4,6a-tetrahydrocyclopenta[c]pyrrole-2 (1H) carboxylate (Compound 38a) was prepared according to the method of Example 1-1.4.

38.2 38a (0.5 g, 1.29 mmol) was added to 5 mL of methanol solution, and LiBH₄ (0.56 g, 2.57 mmol) was added. The reaction was allowed to react at room temperature for 6 hours. After the reaction was completed, water was added to quench the reaction, and the reaction solution was extracted with ethyl acetate (3 x 50 mL), dried over anhydrous sodium sulfate, filtered, and the filtrate was concentrated under reduced pressure, and purified and separated by column chromatography (PE:EA=5: 1) to obtain 0.21 g of tert-butyl 5-(5-(hydroxymethyl)-4, 6-dimethylpyrimidin-2-yl)-3,3 a,4, 6a-tetrahydrocyclopenta[c]pyrrole-2(1H) carboxylate (Compound 38b).

38.3 38b (0.21 g, 0.61 mmol) was added to dichloromethane (5 mL), and DAST (0.2 g, 1.24 mmol) was added at room temperature. The reaction was allowed to react for 3 h at room temperature, and water was added to quench the reaction, and the reaction solution was extracted with dichloromethane (3 x 50 mL). The organic phases were combined, dried over anhydrous sodium sulfate, filtered, and the filtrate was concentrated under pressure, and purified and separated by column chromatography (PE:EA=10:1) to obtain 0.12 g of tert-butyl 5-(5-(fluoromethyl)-4,6-dimethylpyrimidin-2-yl)-3,3 a,4, 6a-tetrahydrocyclopenta[c]pyrrole-2(1H) carboxylate (Compound 38c).

38.4 The reaction raw material 1d was replaced with 38c, and 5-(5-(fluoromethyl)-4,6-dimethylpyrimidin-2-yl)-1,2,3,3 a,4,6a-hexahydrocyclopenta[c]pyrrole (Compound 38d) was prepared according to the method of Example 1-1.5.

38.5 The reaction raw material 1e was replaced with 38d, and (2-fluoro-6-(2H-1,2,3-triazol-2-yl)phenyl)(5-(5-(fluoromethyl)-4,6-dimethylpyrimidin-2-yl)-3,3a,4, 6a-tetrahydrocyclopenta[c]pyrrol-2(1H)-yl)methanone (Compound 38) was prepared according to the method of Example 1-1.7. ¹H NMR (400 MHz, Methanol-*d*₄) δ 7.96 - 7.82 (m, 2H), 7.72 - 7.56 (m, 2H), 7.33 - 7.23 (m, 1H), 6.88 - 6.63 (m, 1H), 5.65 - 5.50 (m, 2H), 3.99 - 3.39 (m, 5H), 3.25 - 2.88 (m, 3H), 2.73 - 2.57 (m, 6H). LCMS (ES, m/z): 437 [M+H]⁺.

### Example 39: Preparation of (2-fluoro-6-(2H-1,2,3-triazol-2-yl)phenyl)(5-(3-methoxy-6-methylpyrazin-2-yl)-3,3a,4,6a-tetrahydrocyclopenta[c]pyrrol-2(1H)-yl)methanone (39)

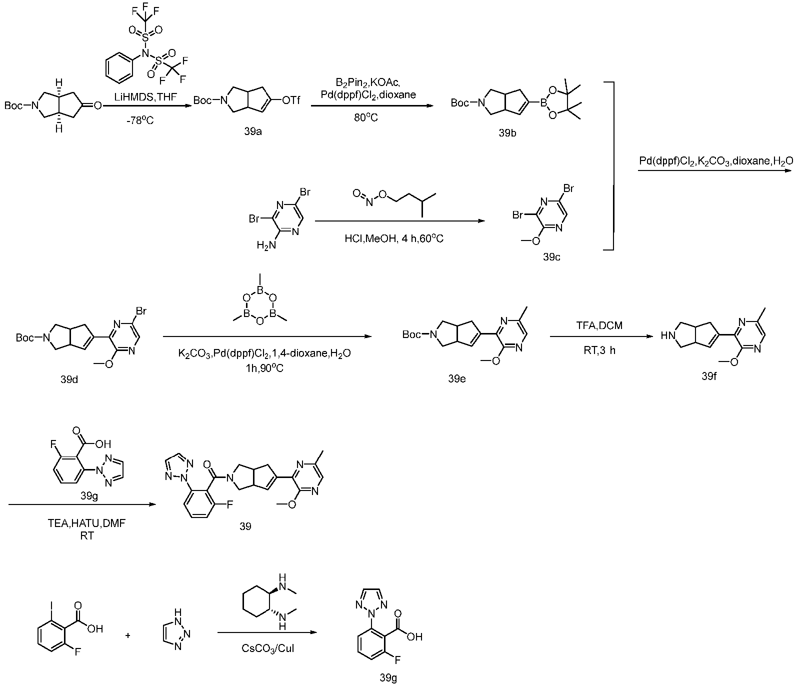

39.1 A solution of tert-butyl cis-5-oxo-hexahydrocyclopenta[c]pyrrole-2-carboxylate (5 g, 22.19 mmol) in THF (20 mL) was treated with LiHMDS (5.2 g, 110.95 mmol) at -78 °C for 2 hours, followed by the dropwise addition of N-phenylbis(trifluoromethanesulfonyl)imide (9.51 g, 26.63 mmol) at -78 °C. The resulting mixture was stirred overnight at room temperature under nitrogen atmosphere. The reaction was quenched by the addition of water/ice (10 mL) at room temperature. The resulting mixture was extracted with EtOAc (3 x 50 mL). The combined organic layers were washed with brine (3 x 20 mL) and dried over anhydrous Na₂SO₄. After filtration, the filtrate was concentrated under reduced pressure to obtain a residue, which was purified by silica gel column chromatography and eluted with PE/EA (1:1) to obtain tert-butyl 5-[[(trifluoromethyl)sulfonyl]oxy]-3,3a,4,6a-tetrahydrocyclopenta[c]pyrrole-2(1H)-carboxylate (Compound 39a) (4.8 g).

39.2 39a (4.5 g, 12.59 mmol), bis(pinacolato)diboron (4.8 g, 18.90 mmol), KOAc (3.5 g, 25.36 mmol) and Pd(dppf)Cl₂ (0.9 g, 1.24 mmol) were added to a solution of dioxane (20 mL), heated to 80 °C, and stirred overnight under nitrogen atmosphere. The mixture was cooled to room temperature. The resulting mixture was diluted with water (20 ml). The resulting mixture was extracted with EtOAc (3 x 50 mL). The combined organic layers were washed with brine (2 x 20 ml) and dried over anhydrous Na₂SO₄. After filtration, the filtrate was concentrated under reduced pressure to obtain 3.6 g of crude tert-butyl 5-(4,4,5,5-tetramethyl-1,3,2-dioxaborolan-2-yl)-1H,3H,3aH,6H,6aH-cyclopenta[c]pyrrole-2-carboxylate (Compound 39b). The crude product was used directly in the next step without further purification.

39.3 A solution of 3,5-dibromopyrazin-2-amine (4 g, 15.82 mmol) and isoamyl nitrite (5.56 g, 47.45 mmol) and HCl (1.16 mL, 31.79 mmol) in MeOH (80 mL) was stirred at 60 °C for 4 hours under nitrogen protection. After cooling to room temperature, the resulting mixture was concentrated under reduced pressure, and extracted with EtOAc (3 x 50 mL). The combined organic layers were washed with saturated brine (3 x 20 mL) and dried over anhydrous Na₂SO₄. After filtration, the filtrate was concentrated under reduced pressure. Column chromatography for separation and purification (PE/EA=7:1) was used to obtain 3.1 g of 3,5-dibromo-2-methoxypyrazine (Compound 39c).

39.4 A solution of 39c (2 g, 7.47 mmol), 39b (2.7 g, 8.21 mmol) and K₂CO₃ (5.58 g, 39.72 mmol, 3 equivalents) and Pd(dppf)Cl₂ (546.2 mg, 0.75 mmol) in 1,4-dioxane (40 mL) and H₂O (6 mL) was stirred at 95 °C for 4 hours under nitrogen atmosphere. The mixture was allowed to cool to room temperature. The resulting mixture was extracted with EtOAc (3 x 30 mL). The combined organic layers were washed with brine (2 x 15 mL) and dried over anhydrous Na₂SO₄. After filtration, the filtrate was concentrated under reduced pressure. The residue was purified by silica gel column chromatography and eluted with PE/EA (3:1) to obtain 1.8 g of tert-butyl 5-(6-bromo-3-methoxypyrazin-2-yl)-3,3a,4,6a-tetrahydrocyclopenta[c]pyrrole-2(1H)-carboxylate (Compound 39d).

39.5 39d (1.8 g, 4.54 mmol), trimethylcyclotriboroxane (684.2 mg, 5.45 mmol), K₂CO₃ (1.88 g, 13.63 mmol), and Pd(dppf)Cl₂ (332.4 mg, 0.45 mmol) were added to 1,4-dioxane (40 mL) and H₂O (6 mL), and the temperature was raised to 90 °C and the reaction was carried out for 1 hour under nitrogen protection. After the reaction was completed, the resulting mixture was cooled to room temperature and extracted with EtOAc (3 x 30 mL). The combined organic layers were washed with saturated brine (3 x 20 mL) and dried over anhydrous Na₂SO₄. After filtration, the filtrate was concentrated under reduced pressure. Column chromatography was used for separation and purification, and PE/EA (6:1) was used as the eluent to obtain 720 mg of tert-butyl 5-(3-methoxy-6-methylpyrazin-2-yl)-3,3a,4,6a-tetrahydrocyclopenta[c]pyrrole-2(1H)-carboxylate (Compound 39e).

39.6 39e (720 mg, 2.17 mmol) was added to a solution of DCM (4 mL) and TFA (16 mL), and stirred at room temperature for 3 hours under nitrogen protection. After the reaction was completed, the reaction solution was concentrated under pressure. The resulting mixture was extracted with EtOAc (3 x 15 mL). The combined organic layers were washed with saturated brine (2 x 9 mL) and dried over anhydrous Na₂SO₄. After filtration, the filtrate was concentrated under reduced pressure. Column chromatography was used for separation and purification, and PE/EA (1:2) was used as the eluent to obtain 460 mg of 5-(3-methoxy-6-methylpyrazin-2-yl)-1,2,3,3a,4,6a-hexahydrocyclopenta[c]pyrrole (Compound 39f).

39.7 2-Fluoro-6-iodobenzoic acid (10.0 g, 37.59 mmol), 1,2,3-triazole (5.2 g, 75.29 mmol), cuprous iodide (0.35 g, 1.2 mmol), cesium carbonate (24.5 g, 75.15 mmol), (1R,2R)-N,N-dimethyl-1,2-diaminocyclohexane (1.1 g, 7.73 mmol), and 1,4-dioxane (100 mL) were placed in a 250 mL round-bottom flask and stirred at 85 °C overnight under nitrogen protection. After the reaction was completed, the reaction solution was cooled to room temperature, 50 mL of tert-butyl methyl ether and 50 mL of water were added, and stirring was continued for half an hour before separation. After discarding the organic phase, the aqueous phase was adjusted to an acidic pH with 2N hydrochloric acid, and then extracted with ethyl acetate (50 mL × 3). The organic phases were combined, dried over anhydrous sodium sulfate, concentrated, and separated and purified by column chromatography (petroleum ether/ethyl acetate = 1:1) to obtain 4.6 g of 2-fluoro-6-(2H-1,2,3-triazol-2-yl)benzoic acid (Compound 39g).

39.8 39g (100 mg, 0.48 mmol) and 39f (126.1 mg, 0.545 mmol) were added to DMF (2 mL), and triethylamine (137.9 mg, 1.36 mmol) and HATU (259.0 mg, 0.68 mmol) were added in batches under room temperature with stirring. The mixture was stirred at room temperature for 2 hours. After the reaction was completed, the reaction solution was concentrated under reduced pressure and separated and purified by column chromatography (petroleum ether/ethyl acetate = 3:1) to obtain 28.1 mg of (2-fluoro-6-(2H-1,2,3-triazol-2-yl)phenyl)(5-(3-methoxy-6-methylpyrazin-2-yl)-3,3a,4,6a-tetrahydrocyclopenta[c]pyrrol-2(1H)-yl)methanone (Compound 39). ¹H NMR (400 MHz, Chloroform-*d*) δ 7.88 - 7.74 (m, 3H), 7.64 - 7.41 (m, 2H), 7.22 - 7.03 (m, 1H), 6.89 - 6.11 (m, 1H), 4.11 - 3.93 (m, 4H), 3.92 - 3.81 (m, 1H), 3.78 - 3.28 (m, 3H), 3.22 - 2.65 (m, 3H), 2.51 - 2.38 (m, 3H). LCMS (ES, m/z): 421 [M+H]⁺.

### Example 40: Preparation of (5-methoxy-2-(2H-1,2,3-triazol-2-yl)phenyl)(5-(3-methoxy-6-methylpyrazin-2-yl)-3,3a,4,6a-tetrahydrocyclopenta[c]pyrrol-2(1H)-yl)methanone (40)

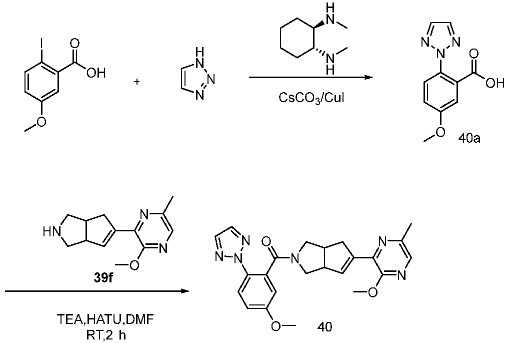

40.1 The reaction raw material 4-fluoro-6-iodobenzoic acid was replaced with 2-iodo-5-methoxybenzoic acid, and 5-methoxy-2-(2H-1,2,3-triazol-2-yl)benzoic acid (Compound 40a) was prepared according to the method of Example 39-39.7.

40.2 The reaction raw material 39g was replaced with 40a, and (5-methoxy-2-(2H-1,2,3-triazol-2-yl)phenyl)(5-(3-methoxy-6-methylpyrazin-2-yl)-3,3a,4,6a-tetrahydrocyclopenta[c]pyrrol-2(1H)-yl)methanone (Compound 40) was prepared according to the method of Example 39-39.8. ¹H NMR (400 MHz, Chloroform-*d*) δ 7.95 - 7.77 (m, 2H), 7.73 - 7.46 (m, 2H), 7.10 - 6.96 (m, 1H), 6.96 - 6.85 (m, 1H), 6.82 - 6.10 (m, 1H), 4.07 - 3.96 (m, 3H), 3.95 - 3.75 (m, 5H), 3.69 - 3.52 (m, 1H), 3.52 - 3.38 (m, 1H), 3.37 - 3.14 (m, 1H), 3.13 - 2.80 (m, 2H), 2.70 (t, *J =* 18.5 Hz, 1H), 2.53 - 2.36 (m, 3H). LCMS (ES, m/z): 433 [M+H]⁺.

### Example 41: Preparation of (4-methoxy-2-(2H-1,2,3-triazol-2-yl)phenyl)(5-(3-methoxy-6-methylpyrazin-2-yl)-3,3a,4,6a-tetrahydrocyclopenta[c]pyrrol-2(1H)-yl)methanone (41)

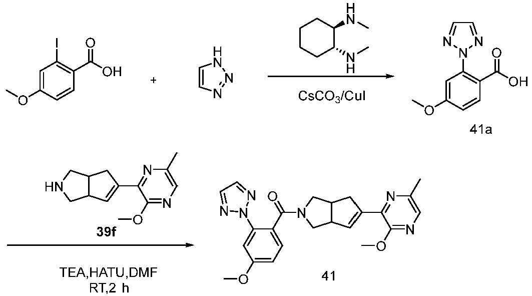

41.1 The reaction raw material 4-fluoro-6-iodobenzoic acid was replaced with 2-iodo-4-methoxybenzoic acid, and 4-methoxy-2-(2H-1,2,3-triazol-2-yl)benzoic acid (Compound 41a) was prepared according to the method of Example 39-39.7.

41.2 The reaction raw material 39g was replaced with 41a, and (4-methoxy-2-(2H-1,2,3-triazol-2-yl)phenyl)(5-(3-methoxy-6-methylpyrazin-2-yl)-3,3a,4,6a-tetrahydrocyclopenta[c]pyrrol-2(1H)-yl)methanone (Compound 41) was prepared according to the method of Example 39-39.8. ¹H NMR (400 MHz, DMSO-*d*₆) δ 7.94 - 7.89 (m, 3H), 7.39 - 7.33(m, 2H), 7.07 (t, *J =* 10.7 Hz, 1H), 6.66 (d, *J =* 84.6 Hz, 1H), 3.97 (d, *J* = 23.2 Hz, 3H), 3.86 (d, *J =* 7.6 Hz, 3H), 3.79 - 3.42 (m, 3H), 3.32 - 3.02 (m, 2H), 3.01 - 2.55 (m, 3H), 2.39 (d, *J =* 18.5 Hz, 3H). LCMS (ES, m/z): 433 [M+H]⁺.

### Example 42: Preparation of (2-methoxy-5-(2H-1,2,3-triazol-2-yl)pyridin-4-yl)(5-(3-methoxy-6-methylpyrazin-2-yl)-3,3a,4,6a-tetrahydrocyclopenta[c]pyrrol-2(1H)-yl)methanone (42)

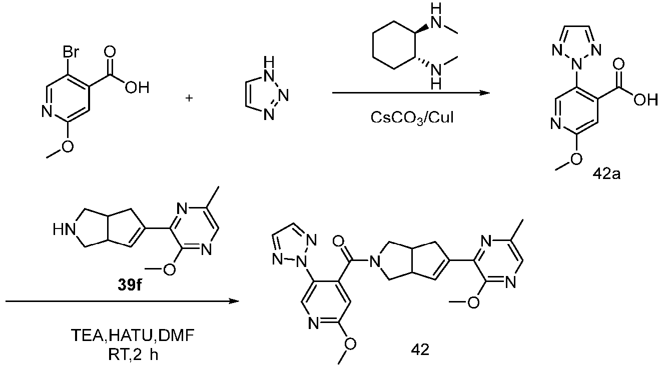

42.1 The reaction raw material 4-fluoro-6-iodobenzoic acid was replaced with 5-bromo-2-methoxyisonicotinic acid, and 2-methoxy-5-(2H-1,2,3-triazol-2-yl)isonicotinic acid (Compound 42a) was prepared according to the method of Example 39-39.7.

42.2 The reaction raw material 39g was replaced with 42a, and (2-methoxy-5-(2H-1,2,3-triazol-2-yl)pyridin-4-yl)(5-(3-methoxy-6-methylpyrazin-2-yl)-3,3a,4,6a-tetrahydrocyclopenta[c]pyrrol-2(1H)-yl)methanone (Compound 42) was prepared according to the method of Example 39-39.8. ¹H NMR (400 MHz, Chloroform-*d*) δ 8.75 (d, *J =* 11.5 Hz, 1H), 7.79 (d, *J =* 5.0 Hz, 1H), 7.66 (d, *J =* 17.0 Hz, 2H), 6.77 - 6.47 (m, 2H), 4.06 - 3.97 (m, 6H), 3.95 - 3.75 (m, 2H), 3.60 (d, *J =* 53.8 Hz, 1H), 3.50 - 3.24 (m, 2H), 3.14 - 2.89 (m, 3H), 2.51 - 2.39 (m, 3H). LCMS (ES, m/z): 434 [M+H]⁺.

### Example 43: Preparation of (2-fluoro-6-(pyrimidin-2-yl)phenyl)(5-(3-methoxy-6-methylpyrazin-2-yl)-3,3a,4,6a-tetrahydrocyclopenta[c]pyrrol-2(1H)-yl)methanone (43)

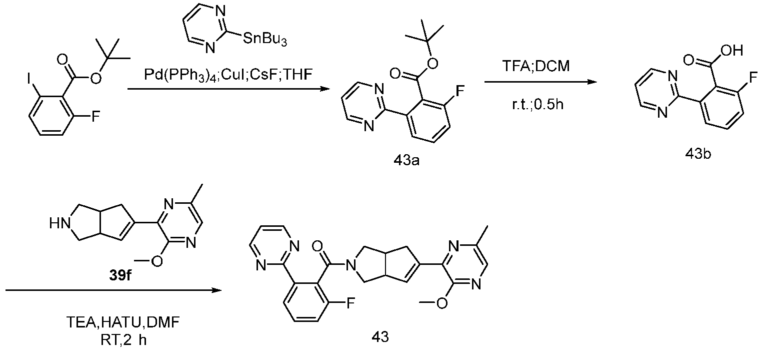

43.1 Tert-butyl 2-fluoro-6-iodobenzoate (200 mg, 0.62 mmol), cuprous iodide (11.82 mg, 0.06 mmol), and cesium fluoride (18.86 mg, 0.12 mmol) were added to tetrahydrofuran, and the mixture was stirred at room temperature for 15 minutes under nitrogen protection. Pd(PPh₃)₄ (43.05 mg, 0.04 mmol) was added at room temperature, and then 2-(tributyltin)pyrimidine (278.78 mg, 0.75 mmol) was added. The temperature was raised to 100 °C for overnight reaction. After the reaction was completed as detected by LCMS, column chromatography for separation and purification (PE/EA=2:1) was used to obtain 160 mg of tert-butyl 2-fluoro-6-(pyrimidin-2-yl)benzoate (Compound 43a).

43.2 43a (160 mg, 0.573 mmol) was added to 2 ml of dichloromethane, and trifluoroacetic acid (2 mL, 26.926 mmol) was added under nitrogen protection at room temperature. The mixture was stirred for 0.5 hours at room temperature. After the reaction was completed, the solvent was evaporated under reduced pressure to obtain 110 mg of 2-fluoro-6-(pyrimidin-2-yl)benzoic acid (Compound 43b).

43.3 The reaction raw material 39g was replaced with 43b, and (2-fluoro-6-(pyrimidin-2-yl)phenyl)(5-(3-methoxy-6-methylpyrazin-2-yl)-3,3a,4,6a-tetrahydrocyclopenta[c]pyrrol-2(1H)-yl)methanone (Compound 43) was prepared according to the method of Example 39-39.8. ¹H NMR (400 MHz, Chloroform-*d*) δ 8.77 - 8.54 (m, 2H),7.83 7.78 (m, 1H), 7.61 - 7.38 (m, 2H), 7.25 - 7.06 (m, 2H), 6.72 - 6.24 (m, 1H), 4.16 - 3.87 (m, 4H), 3.82 - 3.74 (m, 1H), 3.69 - 3.17 (m, 3H), 3.11 - 2.61 (m, 3H), 2.48 - 2.32 (m, 3H). LCMS (ES, m/z): 432 [M+H]⁺ ∘

### Example 44: Preparation of [4-fluoro-2-(2H-1,2,3-triazol-2-yl)phenyl][5-(3-methoxy-6-methylpyrazin-2-yl)-3,3a,4,6a-tetrahydrocyclopenta[c]pyrrol-2(1H)-yl]methanone (44)

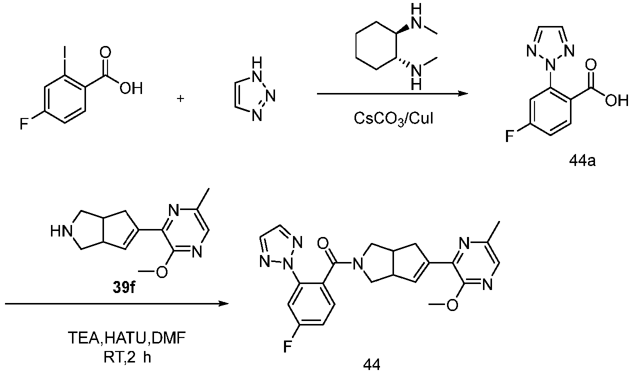

44.1 The reaction raw material 4-fluoro-6-iodobenzoic acid was replaced with 4-fluoro-2-iodobenzoic acid, and 4-fluoro-2-(2H-1,2,3-triazol-2-yl)benzoic acid (Compound 44a) was prepared according to the method of Example 39-39.7.

44.2 The reaction raw material 39g was replaced with 44a, and [4-fluoro-2-(2H-1,2,3-triazol-2-yl)phenyl][5-(3-methoxy-6-methylpyrazin-2-yl)-3,3a,4,6a-tetrahydrocyclopenta[c]pyrrol-2(1H)-yl]methanone (Compound 44) was prepared according to the method of Example 39-39.8. ¹H NMR (400 MHz, DMSO-*d*₆) δ 8.35 - 7.80 (m, 3H), 7.79 - 7.67 (m, 1H), 7.49 (s, 1H), 7.42 - 7.32 (m, 1H), 6.80 - 6.49 (m, 1H), 4.05 - 3.88 (m, 3H), 3.64 (br s, 2H), 3.55 - 3.34 (m, 1H), 3.12 (br s, 1H), 3.06 - 2.65 (m, 3H), 2.63 - 2.51 (m, 1H), 2.43 - 2.32 (m, 3H). LCMS (ES, m/z): 421 [M+H]⁺.

### Example 45: Preparation of [4-ethoxy-2-(2H-1,2,3-triazol-2-yl)phenyl][5-(3-methoxy-6-methylpyrazin-2-yl)-3,3a,4,6a-tetrahydrocyclopenta[c]pyrrol-2(1H)-yl]methanone (45)

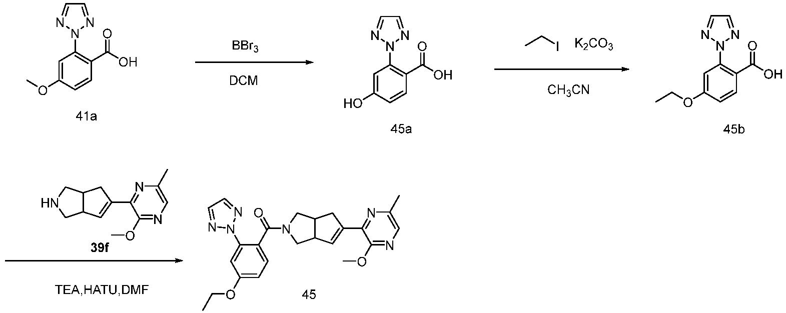

45.1 Compound 41a (300 mg, 1.37 mmol) was dissolved in 30 ml of dichloromethane solution, cooled in an ice-water bath, and boron tribromide dichloromethane solution (1.0 M, 3 mL) was added dropwise to the reaction solution under nitrogen protection. After the addition was complete, the mixture was allowed to react at room temperature for 3 hours, cooled in an ice-water bath, quenched with water, and extracted with dichloromethane. The organic phase was dried over anhydrous sodium sulfate, filtered, and the filtrate was concentrated under reduced pressure to obtain 230 mg of 4-hydroxy-2-(2H-1,2,3-triazol-2-yl)benzoic acid (Compound 45a). The crude product was directly subjected to the next step without purification.

45.2 Compound 45a (230 mg, 1.12 mmol), iodoethane (262 mg, 1.68 mmol) and potassium carbonate (464 mg, 3.36 mmol) were added to 30 mL of acetonitrile, and the temperature was raised to 60 °C and the reaction was carried out for 4 hours. After the reaction was completed, the temperature was lowered to room temperature, 1 M hydrochloric acid solution was added to adjust the pH to 4-5, the solvent was evaporated under pressure, and the mixture was extracted with dichloromethane. The organic phase was dried over anhydrous sodium sulfate, filtered, and the filtrate was concentrated under reduced pressure to obtain 4-ethoxy-2-(2H-1,2,3-triazol-2-yl)benzoic acid (Compound 45b). The crude product was directly subjected to the next step without purification.

45.3 The reaction raw material 39g was replaced with 45b, and [4-ethoxy-2-(2H-1,2,3-triazol-2-yl)phenyl][5-(3-methoxy-6-methylpyrazin-2-yl)-3,3a,4,6a-tetrahydrocyclopenta[c]pyrrol-2(1H)-yl]methanone (Compound 45) was prepared according to the method of Example 39-39.8. ¹H NMR (400 MHz, DMSO-*d*₆) δ 8.07 - 7.80 (m, 3H), 7.39 - 7.29 (m, 2H), 7.05 (br t, *J =* 9.9 Hz, 1H), 6.79 - 6.53 (m, 1H), 4.13 (s, 2H), 4.01 - 3.89 (m, 3H), 3.77 - 3.57 (m, 2H), 3.46 (br d, *J =* 5.6 Hz, 1H), 3.29 - 3.04 (m, 2H), 2.95 - 2.80 (m, 2H), 2.67 (br s, 1H), 2.39 (br d, *J =* 18.1 Hz, 3H), 1.40 - 1.34 (m, 3H). LCMS (ES, m/z): 447 [M+H]⁺.

### Example 46: Preparation of [5-(3-ethoxy-6-methylpyrazin-2-yl)-3,3a,4,6a-tetrahydrocyclopenta[c]pyrrol-2(1H)-yl][4-methoxy-2-(2H-1,2,3-triazol-2-yl)phenyl]methanone (46)

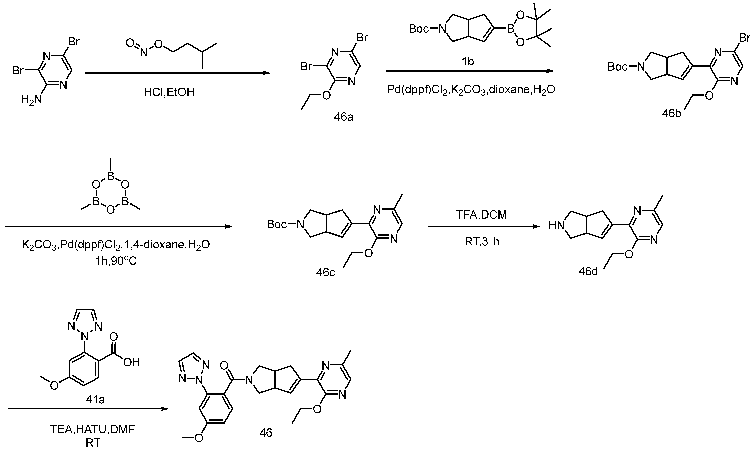

46.1 The reaction raw material methanol was replaced with ethanol, and 3,5-dibromo-2-ethoxypyrazine (Compound 46a) was prepared according to the method of Example 39-39.3.

46.2 The reaction raw material 39c was replaced with 46a, and tert-butyl 5-(6-bromo-3-ethoxypyrazin-2-yl)-3,3a,4,6a-tetrahydrocyclopenta[c]pyrrole-2(1H)-carboxylate (Compound 46b) was prepared according to the method of Example 39-39.4.

46.3 The reaction raw material 39d was replaced with 46b, and tert-butyl 5-(3-ethoxy-6-methylpyrazin-2-yl)-3,3a,4,6a-tetrahydrocyclopenta[c]pyrrole-2(1H)-carboxylate (Compound 46c) was prepared according to the method of Example 39-39.5.

46.4 The reaction raw material 39e was replaced with 46c, and 5-(3-ethoxy-6-methylpyrazin-2-yl)-1,2,3a,4,6a-hexahydrocyclopenta[c]pyrrole (Compound 46d) was prepared according to the method of Example 39-39.6.

46.5 The reaction raw material 39f was replaced with 46d, 39g was replaced with 41a, and [5-(3-ethoxy-6-methylpyrazin-2-yl)-3,3a,4,6a-tetrahydrocyclopenta[c]pyrrol-2(1H)-yl][4-methoxy-2-(2H-1,2,3-triazol-2-yl)phenyl]methanone (Compound 46) was prepared according to the method of Example 39-39.8. ¹H NMR (400 MHz, DMSO-*d*₆) δ 8.08 - 7.79 (m, 3H), 7.48 - 7.22 (m, 2H), 7.07 (td, *J =* 8.3, 4.1 Hz, 1H), 6.69 (d, *J =* 76.7 Hz, 1H), 4.48 - 4.34 (m, 2H), 3.86 (d, *J =* 6.5, 3H), 3.79 - 3.43 (m, 3H), 3.29 - 2.56 (m, 5H), 2.38 (d, *J =* 18.0 Hz, 3H), 1.48 - 1.29 (m, 3H). LCMS (ES, m/z): 447 [M+H]⁺.

### Example 47: Preparation of [5-(6-ethyl-3-methoxypyrazin-2-yl)-3,3a,4,6a-tetrahydrocyclopenta[c]pyrrol-2(1H)-yl][4-methoxy-2-(2H-1,2,3-triazol-2-yl)phenyl]methanone (47)

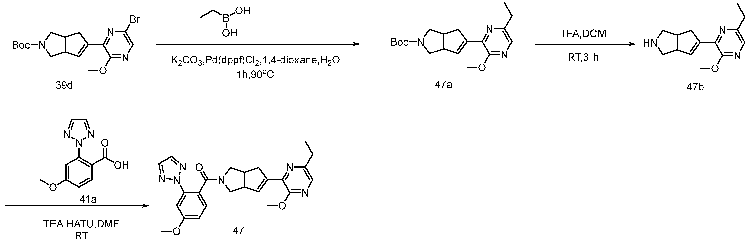

47.1 The reaction raw material trimethylcyclotriboroxane was replaced with ethylboronic acid, and tert-butyl 5-(6-ethyl-3-methoxypyrazin-2-yl)-3,3a,4,6a-tetrahydrocyclopenta[c]pyrrole-2(1H)-carboxylate (Compound 47a) was prepared according to the method of Example 39-39.5.

47.2 The reaction raw material 39e was replaced with 47a, and 5-(6-ethyl-3-methoxypyrazin-2-yl)-1,2,3a,4,6a-hexahydrocyclopenta[c]pyrrole (Compound 47b) was prepared according to the method of Example 39-39.6.

47.3 The reaction raw material 39f was replaced with 47b, 1g was replaced with 3a, and [5-(6-ethyl-3-methoxypyrazin-2-yl)-3,3a,4,6a-tetrahydrocyclopenta[c]pyrrol-2(1H)-yl][4-methoxy-2-(2H-1,2,3-triazol-2-yl)phenyl]methanone (Compound 47) was prepared according to the method of Example 39-39.8. ¹H NMR (400 MHz, DMSO) δ 7.91 (d, *J =* 30.0 Hz, 3H), 7.37 (d, *J =* 15.1 Hz, 2H), 7.18 - 6.95 (m, 1H), 6.66 (d, *J =* 83.7 Hz, 1H), 3.97 (d, *J =* 22.9 Hz, 3H), 3.86 (d, *J =* 8.1 Hz, 3H), 3.79 - 3.67 (m, 1H), 3.62 (s, 1H), 3.47 (s, 1H), 3.31 - 3.14 (m, 1H), 3.10 (d, *J =* 9.9 Hz, 1H), 2.99 (dd, *J =* 16.6, 6.9 Hz, 1H), 2.88 (dd, *J =* 23.9, 11.7 Hz, 2H), 2.73 (d, *J =* 7.4 Hz, 1H), 2.62 (dd, *J =* 22.3, 11.3 Hz, 1H), 1.21 (dt, *J =* 19.6, 7.4 Hz, 3H). LCMS (ES, m/z): 447 [M+H]⁺ ∘

### Example 48: Preparation of [4-methoxy-2-(pyrimidin-2-yl)phenyl][5-(3-methoxy-6-methylpyrazin-2-yl)-3,3a,4,6a-tetrahydrocyclopenta[c]pyrrol-2(1H)-yl]methanone (48)

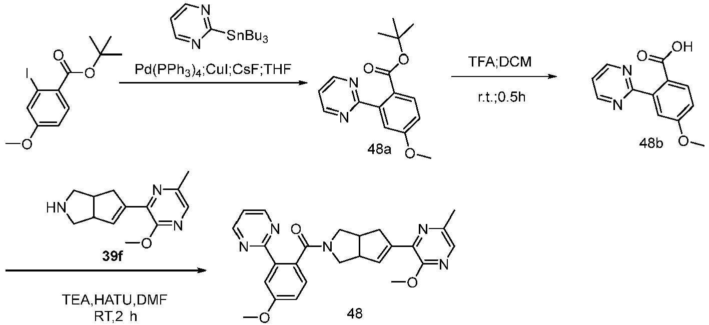

48.1 The reaction raw material tert-butyl 2-fluoro-6-iodobenzoate was replaced with tert-butyl 2-iodo-4-methoxybenzoate, and tert-butyl 4-methoxy-2-(pyrimidin-2-yl)benzoate (Compound 48a) was prepared according to the method of Example 43-43.1.

48.2 The reaction raw material 43a was replaced with 48a, and 4-methoxy-2-(pyrimidin-2-yl)benzoic acid (Compound 48b) was prepared according to the method of Example 43-43.2.

48.3 The reaction raw material 39g was replaced with 48b, and [4-methoxy-2-(pyrimidin-2-yl)phenyl][5-(3-methoxy-6-methylpyrazin-2-yl)-3,3a,4,6a-tetrahydrocyclopenta[c]pyrrol-2(1H)-yl]methanone (Compound 48) was prepared according to the method of Example 39-39.8. ¹H NMR (400 MHz, DMSO) δ 8.81 - 8.60 (m, 2H), 7.94 (d, *J =* 9.9 Hz, 1H), 7.65 (d, *J =* 14.7 Hz, 1H), 7.27 (dd, *J =* 19.5, 12.2 Hz, 2H), 7.10 (t, *J =* 9.4 Hz, 1H), 6.68 (d, *J* = 97.0 Hz, 1H), 3.97 (d, *J =* 36.2 Hz, 3H), 3.85 (d, *J =* 7.9 Hz, 3H), 3.75 (dd, *J =* 23.7, 13.0 Hz, 1H), 3.68 - 3.59 (m, 1H), 3.48 (s, 1H), 3.21 (d, *J =* 10.6 Hz, 1H), 3.08 - 2.92 (m, 1H), 2.90 - 2.70 (m, 2H), 2.63 (dd, *J* = 46.2, 11.3 Hz, 1H), 2.39 (d, *J* = 27.3 Hz, 3H). LCMS (ES, m/z): 444 [M+H]⁺ ∘

### Example 49: Preparation of [5-(3-methoxy-6-methylpyrazin-2-yl)-3,3a,4,6a-tetrahydrocyclopenta[c]pyrrol-2(1H)-yl][5-methyl-2-(2H-1,2,3-triazol-2-yl)phenyl]methanone (49)

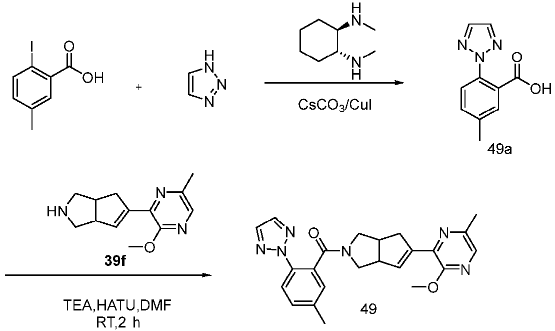

49.1 The reaction raw material 2-fluoro-6-iodobenzoic acid was replaced with 2-iodo-5-methylbenzoic acid, and 5-methyl-2-(2H-1,2,3-triazol-2-yl)benzoic acid (Compound 49a) was prepared according to the method of Example 43-43.1.

49.2 The reaction raw material 39g was replaced with 49a, and [5-(3-methoxy-6-methylpyrazin-2-yl)-3,3a,4,6a-tetrahydrocyclopenta[c]pyrrol-2(1H)-yl][5-methyl-2-(2H-1,2,3-triazol-2-yl)phenyl]methanone (Compound 49) was prepared according to the method of Example 39-39.8. ¹H NMR (400 MHz, DMSO-*d*₆) δ 7.93 (br d, *J =* 4.6 Hz, 3H), 7.74 (br dd, *J =* 8.3, 14.7 Hz, 1H), 7.40 (br t, *J =* 8.4 Hz, 1H), 7.26 - 7.17 (m, 1H), 6.87 - 6.47 (m, 1H), 4.02 - 3.92 (m, 3H), 3.62 (br s, 2H), 3.56 - 3.33 (m, 1H), 3.23 - 3.08 (m, 1H), 3.07 - 2.70 (m, 3H), 2.59 (br d, *J* = 15.7 Hz, 1H), 2.41 - 2.33 (m, 6H). LCMS (ES, m/z): 417 [M+H]⁺.

### Example 50: Preparation of [5-(3,6-dimethoxypyrazin-2-yl)-3,3a,4,6a-tetrahydrocyclopenta[c]pyrrol-2(1H)-yl][4-fluoro-2-( 2H-1,2,3-triazol-2-yl)phenyl]methanone (50)

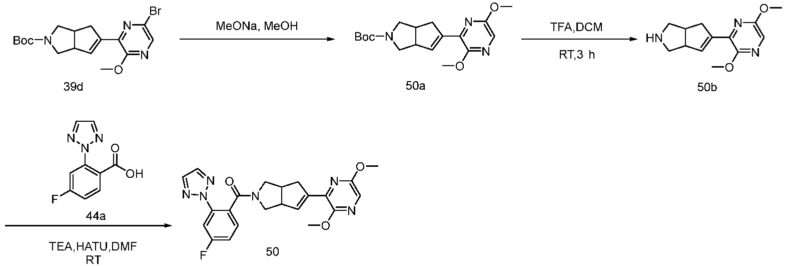

50.1 Compound 39d (300 mg, 0.76 mmol) and sodium methoxide (123 mg, 2.28 mmol) were added to 30 mL of methanol solution, and heated to 60 °C for reaction for 6 hours. After the reaction was completed, the solvent was evaporated under reduced pressure, and separated and purified by column chromatography (PE: EA = 5:1) to obtain 136 mg of tert-butyl 5-(3,6-dimethoxypyrazin-2-yl)-3,3a,4,6a-tetrahydrocyclopenta[c]pyrrole-2(1H)-carboxylate (Compound 50a).

50.2 The reaction raw material 39e was replaced with 50a, and 5-(3,6-dimethoxypyrazin-2-yl)-1,2,3a,4,6a-hexahydrocyclopenta[c]pyrrole (Compound 50b) was prepared according to the method of Example 39-39.6.

50.3 The reaction raw material 39f was replaced with 50b, and 39g was replaced with 44a, and [5-(3,6-dimethoxypyrazin-2-yl)-3,3a,4,6a-tetrahydrocyclopenta[c]pyrrol-2(1H)-yl][4-fluoro-2-(2H-1,2,3-triazol-2-yl)phenyl]methanone (Compound 50) was prepared according to the method of Example 39-39.8. ¹H NMR (400 MHz, DMSO) δ 8.29 - 7.82 (m, 3H), 7.76 (d, *J =* 4.8 Hz, 1H), 7.48 (dd, *J =* 17.4, 8.7 Hz, 1H), 7.39 (d, *J =* 8.1 Hz, 1H), 6.69 (d, *J* = 71.9 Hz, 1H), 3.96 (d, *J* = 21.2 Hz, 3H), 3.85 (d, *J =* 26.1 Hz, 3H), 3.79 - 3.60 (m, 2H), 3.52 (s, 1H), 3.21 (dd, *J =* 11.8, 5.9 Hz, 1H), 3.10 - 2.82 (m, 3H), 2.67 (t, *J =* 17.2 Hz, 1H). LCMS (ES, m/z): 437 [M+H]⁺.

### Example 51: Preparation of [5-(3,6-dimethylpyrazin-2-yl)-3,3a,4,6a-tetrahydrocyclopenta[c]pyrrol-2(1H)-yl][2-fluoro-6-(2 H-1,2,3-triazol-2-yl)phenyl]methanone (51)

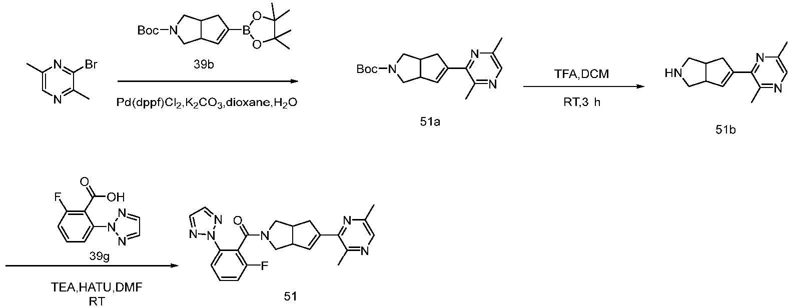

51.1 The reaction raw material 39c was replaced with 3-bromo-2,5-dimethylpyrazine, and tert-butyl 5-(3,6-dimethylpyrazin-2-yl)-3,3a,4,6a-tetrahydrocyclopenta[c]pyrrole-2(1H)-carboxylate (Compound 51a) was prepared according to the method of Example 39-39.4.

51.2 The reaction raw material 39e was replaced with 51a, and 5-(3,6-dimethylpyrazin-2-yl)-1,2,3a,4,6a-hexahydrocyclopenta[c]pyrrole (Compound 51b) was prepared according to the method of Example 39-39.6.

51.3 The reaction raw material 39f was replaced with 51b, and [5-(3,6-dimethylpyrazin-2-yl)-3,3a,4,6a-tetrahydrocyclopenta[c]pyrrole-2(1H)-yl][2-fluoro-6-(2H-1,2,3-triazol-2-yl)phenyl]methanone (Compound 51) was prepared according to the method of Example 39-39.8. ¹H NMR (400 MHz, DMSO-*d*₆) δ 8.32 - 8.22 (m, 1H), 8.15 (s, 1H), 7.89 (d, *J =* 6.1 Hz, 1H), 7.84 - 7.75 (m, 1H), 7.72 - 7.58 (m, 1H), 7.50 - 7.33 (m, 1H), 6.30 - 6.05 (m, 1H), 3.80 - 3.45 (m, 3H), 3.26 - 2.96 (m, 2H), 2.92 - 2.72 (m, 1H), 2.71 - 2.56 (m, 5H), 2.48 - 2.37 (m, 3H). LCMS (ES, m/z): 405 [M+H]⁺.

### Example 52: Preparation of [5-(3,6-dimethylpyrazin-2-yl)-3,3a,4,6a-tetrahydrocyclopenta[c]pyrrol-2(1H)-yl][4-methoxy-2-(2H-1,2,3-triazol-2-yl)phenyl]methanone (52)

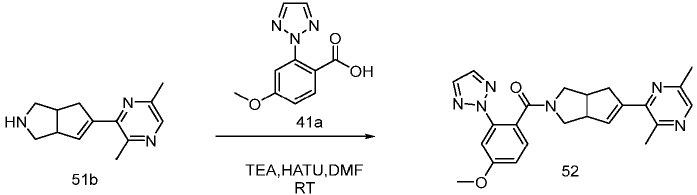

52.1 The reaction raw material 39f was replaced with 51b, 39g was replaced with 41a, and [5-(3,6-dimethylpyrazin-2-yl)-3,3a,4,6a-tetrahydrocyclopenta[c]pyrrol-2(1H)-yl][4-methoxy-2-(2H-1,2,3-triazol-2-yl)phenyl]methanone (Compound 52) was prepared according to the method of Example 39-39.8. ¹H NMR (400 MHz, DMSO) δ 8.26 (d, *J =* 5.0 Hz, 1H), 7.98 (d, *J =* 7.7 Hz, 2H), 7.38 (d, *J =* 7.6 Hz, 2H), 7.09 (t, *J* = 7.8 Hz, 1H), 6.12 (d, *J =* 76.3 Hz, 1H), 3.87 (d, *J =* 4.6 Hz, 3H), 3.81 - 3.58 (m, 2H), 3.49 (s, 1H), 3.28 (dd, *J =* 11.8, 5.9 Hz, 1H), 3.17 - 3.01 (m, 1H), 2.92 (ddd, *J* = 32.2, 15.1, 7.3 Hz, 2H), 2.80 - 2.65 (m, 1H), 2.56 (d, *J =* 18.5 Hz, 3H), 2.43 (d, *J =* 15.4 Hz, 3H). LCMS (ES, m/z): 417 [M+H]⁺.

### Example 53: Preparation of [5-(3,6-dimethoxypyrazin-2-yl)-3,3a,4,6a-tetrahydrocyclopenta[c]pyrrol-2(1H)-yl][4-methoxy-2-(2H-1,2,3-triazol-2-yl)phenyl]methanone (53)

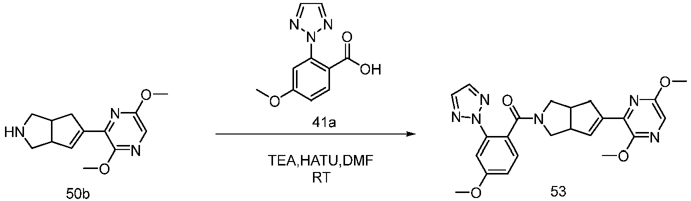

53.1 The reaction raw material 39f was replaced with 50b, 39g was replaced with 41a, and [5-(3,6-dimethoxypyrazin-2-yl)-3,3a,4,6a-tetrahydrocyclopenta[c]pyrrol-2(1H)-yl][4-methoxy-2-(2H-1,2,3-triazol-2-yl)phenyl]methanone (Compound 53) was prepared according to the method of Example 39-39.8. ¹H NMR (400 MHz, DMSO-*d*₆) δ 8.18 - 7.82 (m, 2H), 7.75 (br d, *J =* 3.9 Hz, 1H), 7.41 - 7.30 (m, 2H), 7.07 (br t, *J =* 9.5 Hz, 1H), 6.81 - 6.50 (m, 1H), 4.04 - 3.90 (m, 3H), 3.90 - 3.77 (m, 6H), 3.76 - 3.57 (m, 2H), 3.47 (br s, 1H), 3.29 - 3.02 (m, 2H), 2.85 (br d, *J =* 15.7 Hz, 2H), 2.59 (br d, *J =* 14.7 Hz, 1H). LCMS (ES, m/z): 449 [M+H]⁺.

### Example 54: Preparation of [5-(6-chloro-3-methylpyrazin-2-yl)-3,3a,4,6a-tetrahydrocyclopenta[c]pyrrol-2(1H)-yl][4-methoxy-2-(2H-1,2,3-triazol-2-yl)phenyl]methanone (54)

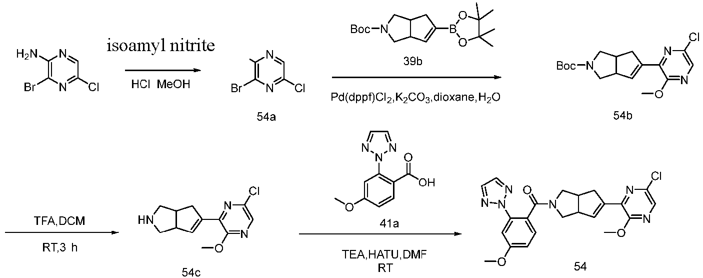

54.1 2-Amino-3-bromo-5-chloropyrazine (3 g, 14.4 mmol) was dissolved in 30 mL of methanol solution, tert-butyl nitrite (5.1 g, 43.2 mmol) was added, and then HCl methanol solution was added. The temperature was raised to 60 °C and the reaction was carried out for 6 hours. After the reaction was completed, water was added to quench the reaction, followed by extraction with ethyl acetate and separation of the layers. The organic phase was dried over anhydrous sodium sulfate, and filtered. The filtrate was concentrated under reduced pressure, and separated and purified by column chromatography (PE: EA=10:1) to obtain 2 g of 3-bromo-5-chloro-2-methoxypyrazine (Compound 54a).

54.2 The reaction raw material 39c was replaced with 54a, and tert-butyl 5-(6-chloro-3-methoxypyrazin-2-yl)-3,3a,4,6a-tetrahydrocyclopenta[c]pyrrole-2(1H)-carboxylate (Compound 54b) was prepared according to the method of Example 39-39.4.

54.2 The reaction raw material 39e was replaced with 54b, and 5-(6-chloro-3-methoxypyrazin-2-yl)-1,2,3a,4,6a-hexahydrocyclopenta[c]pyrrole (Compound 54c) was prepared according to the method of Example 39-39.6.

54.3 The reaction raw material 39f was replaced with 54c, 39g was replaced with 41a, and [5-(6-chloro-3-methylpyrazin-2-yl)-3,3a,4,6a-tetrahydrocyclopenta[c]pyrrol-2(1H)-yl][4-methoxy-2 -(2H-1,2,3-triazol-2-yl)phenyl]methanone (Compound 54) was prepared according to the method of Example 39-39.8. ¹H NMR (400 MHz, DMSO-*d*₆) δ 8.19 (d, *J =* 2.0 Hz, 1H), 8.09 - 7.76 (m, 2H), 7.40 - 7.29 (m, 2H), 7.06 (t, *J =* 9.4 Hz, 1H), 6.90 - 6.61 (m, 1H), 4.10 - 3.94 (m, 3H), 3.86 (d, *J =* 7.1 Hz, 3H), 3.62 (s, 2H), 3.58 - 3.37 (m, 1H), 3.33 - 3.03 (m, 2H), 2.93 (s, 1H), 2.86 - 2.66 (m, 2H). LCMS (ES, m/z): 453 [M+H]⁺.

### Example 55: Preparation of [5-(6-cyclopropyl-3-methoxypyrazin-2-yl)-3,3a,4,6a-tetrahydrocyclopenta[c]pyrrol-2(1H)-yl][4 -fluoro-2-(2H-1,2,3-triazol-2-yl)phenyl]methanone (55)

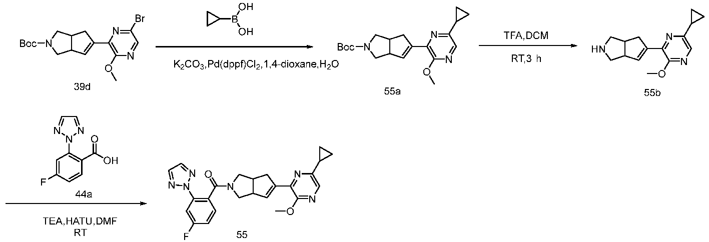

55.1 The reaction raw material trimethylcyclotriboroxane was replaced with cyclopropylboronic acid, and tert-butyl 5-(6-cyclopropyl-3-methoxypyrazin-2-yl)-3,3a,4,6a-tetrahydrocyclopenta[c]pyrrole-2(1H)-carboxylate (Compound 55a) was prepared according to the method of Example 39-39.5.

55.2 The reaction raw material 39e was replaced with 55a, and 5-(6-cyclopropyl-3-methoxypyrazin-2-yl)-1,2,3a,4,6a-hexahydrocyclopenta[c]pyrrole (Compound 55b) was prepared according to the method of Example 39-39.7.

55.3 The reaction raw material 39f was replaced with 55b, 39g was replaced with 44a, and [5-(6-cyclopropyl-3-methoxypyrazin-2-yl)-3,3a,4,6a-tetrahydrocyclopenta[c]pyrrol-2(1H)-yl][4-fluoro-2-(2H-1,2,3-triazol-2-yl)phenyl]methanone (Compound 55) was prepared according to the method of Example 39-39.8. ¹H NMR (400 MHz, DMSO) δ 8.00 (d, *J =* 6.2 Hz, 3H), 7.74 (dd, *J =* 12.8, 11.1 Hz, 1H), 7.50 (t, *J =* 6.6 Hz, 1H), 7.38 (dd, *J =* 20.5, 9.0 Hz, 1H), 6.65 (d, *J =* 84.9 Hz, 1H), 3.96 (d, *J =* 24.1 Hz, 3H), 3.87 - 3.71 (m, 1H), 3.64 (s, 1H), 3.48 (s, 1H), 3.31 - 3.05 (m, 2H), 3.05 - 2.89 (m, 1H), 2.80 (d, *J =* 13.9 Hz, 1H), 2.75 - 2.54 (m, 1H), 2.22 - 1.97 (m, 1H), 1.07 - 0.65 (m, 4H). LCMS (ES, m/z): 447 [M+H]⁺.

### Example 56: Preparation of [4-methoxy-2-(2H-1,2,3-triazol-2-yl)phenyl][5-[3-methoxy-6-(methyl-d3)pyrazin-2-yl]-3,3a,4,6a-tetrahydrocyclopentyl[c]pyrrol-2(1H)-yl]methanone (56)

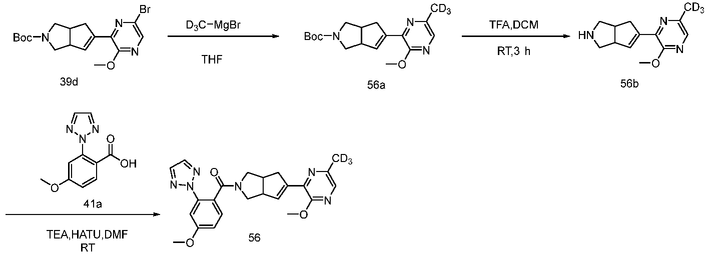

56.1 Compound 39d (300 mg, 0.755 mmol) and ferric triacetylacetonate (50 mg, 0.14 mmol) were dissolved in 20 mL of tetrahydrofuran solution and cooled in an ice bath. Methyl-D3-magnesium iodide tetrahydrofuran solution (1 M, 2 mL) was added dropwise. After the addition was complete, the temperature was raised to room temperature and the reaction was carried out for 6 hours. After the reaction was complete, the reaction solution was quenched with water, extracted with ethyl acetate and dried over anhydrous sodium sulfate. The solvent was evaporated under reduced pressure and separated and purified by column chromatography (PE:EA=5: 1) to obtain 120 mg of tert-butyl 5-(3-methoxy-6-(methyl-d3)pyrazin-2-yl)-3,3a,4,6a-tetrahydrocyclopenta[c]pyrrole-2(1H)-carboxylate (Compound 56a).

56.2 The reaction raw material 39e was replaced with 56a, and 5-(3-methoxy-6-(methyl-d3)pyrazin-2-yl)-1,2,3,3a,4,6a-hexahydrocyclopenta[c]pyrrole (Compound 56b) was prepared according to the method of Example 39-39.6.

56.3 The reaction raw material 39f was replaced with 56b, 39g was replaced with 41a, and [4-methoxy-2-(2H-1,2,3-triazol-2-yl)phenyl][5-[3-methoxy-6-(methyl-d3)pyrazin-2-yl]-3,3a,4,6a-tetrahydrocyclopenta[c]pyrrol-2(1H)-yl]methanone (Compound 56) was prepared according to the method of Example 39-39.8. ¹H NMR (400 MHz, DMSO-*d*₆) δ 8.27 - 7.65 (m, 3H), 7.42 - 7.26 (m, 2H), 7.06 (br t, *J* = 9.9 Hz, 1H), 6.79 - 6.50 (m, 1H), 4.01 - 3.90 (m, 3H), 3.86 (br d, *J =* 7.1 Hz, 3H), 3.61 (br s, 2H), 3.52 - 3.35 (m, 1H), 3.30 - 3.04 (m, 2H), 2.98 - 2.81 (m, 2H), 2.65 - 2.53 (m, 1H). LCMS (ES, m/z): 436 [M+H]⁺.

### Example 57: Preparation of [5-(3-methoxy-6-methylpyrazin-2-yl)-3,3a,4,6a-tetrahydrocyclopenta[c]pyrrol-2(1H)-yl][4-(methoxy-d3)-2-(2H-1,2,3-triazol-2-yl)phenyl]methanone (57)

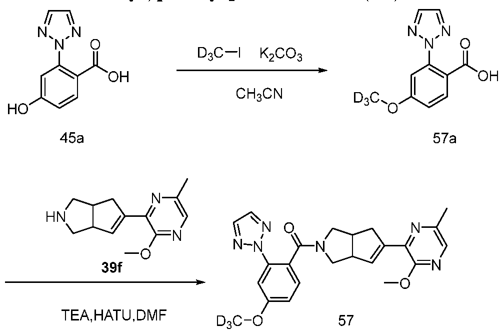

57.1 The raw material iodoethane was replaced with deuterated iodomethane, and 4-(methoxy-d3)-2-(2H-1,2,3-triazol-2-yl)benzoic acid (Compound 57a) was prepared according to the method of Example 45-45.2.

57.2 The raw material 39g was replaced with 57a, and [5-(3-methoxy-6-methylpyrazin-2-yl)-3,3a,4,6a-tetrahydrocyclopenta[c]pyrrol-2(1H)-yl][4-(methoxy-d3)-2-(2H-1,2,3-triazol-2-yl)phenyl]methanone (Compound 57) was prepared according to the method of Example 39-39.8. ¹H NMR (400 MHz, DMSO) δ 8.04 - 7.77 (m, 3H), 7.36 (d, *J =* 14.3 Hz, 2H), 7.06 (t, *J =* 9.8 Hz, 1H), 6.65 (d, *J =* 84.1 Hz, 1H), 3.96 (d, *J =* 23.1 Hz, 3H), 3.77 - 3.54 (m, 2H), 3.52 - 3.41 (m, 1H), 3.30 - 3.15 (m, 1H), 3.09 (d, *J* = 9.9 Hz, 1H), 2.92 (ddd, *J* = 39.8, 24.4, 11.2 Hz, 2H), 2.70 - 2.54 (m, 1H), 2.38 (d, *J =* 18.4 Hz, 3H). LCMS (ES, m/z): 436 [M+H]⁺.

### Example 58: Preparation of [4-methoxy-2-(2H-1,2,3-triazol-2-yl)phenyl][5-[3-(methoxy-d3)-6-methylpyrazin-2-yl]-3,3a,4,6a-tetrahydrocyclopentyl[c]pyrrol-2(1H)-yl]methanone (58)

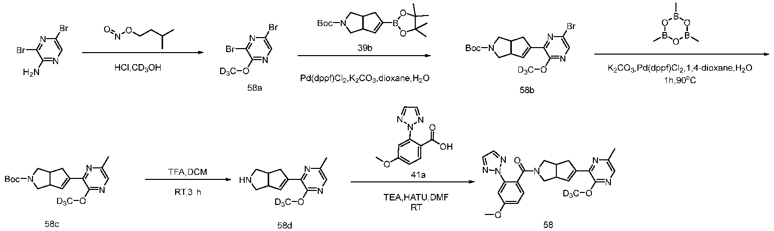

58.1 The reaction raw material methanol was replaced with deuterated methanol, and 3,5-dibromo-2-(methoxy-d3)pyrazine (Compound 58a) was prepared according to the method of Example 39-39.3.

58.2 The reaction raw material 39c was replaced with 58a, and tert-butyl 5-[6-bromo-3-(methoxy-d3)pyrazin-2-yl]-3,3a,4,6a-tetrahydrocyclopenta[c]pyrrole-2(1H)-carboxylate (Compound 58b) was prepared according to the method of Example 39-39.4.

58.3 The reaction raw material 39d was replaced with 58b, and tert-butyl 5-[3-(methoxy-d3)-6-methylpyrazin-2-yl]-3,3a,4,6a-tetrahydrocyclopenta[c]pyrrole-2(1H)-carboxylate (Compound 58c) was prepared according to the method of Example 39-39.5.

58.4 The reaction raw material 39e was replaced with 58c, and 5-[3-(methoxy-d3)-6-methylpyrazin-2-yl]-1,2,3,3a,4,6a-hexahydrocyclopenta[c]pyrrole (Compound 58d) was prepared according to the method of Example 39-39.6.

58.5 The reaction raw material 39g was replaced with 41a, 39f was replaced with 58d, and [4-methoxy-2-(2H-1,2,3-triazol-2-yl)phenyl][5-[3-(methoxy-d3)-6-methylpyrazin-2-yl]-3,3a,4,6a-tetrahydrocyclopentyl[c]pyrrol-2(1H)-yl]methanone (Compound 58) was prepared according to the method of Example 39-39.8. ¹H NMR (400 MHz, DMSO-*d*₆) δ 8.12 - 7.67 (m, 3H), 7.43 - 7.25 (m, 2H), 7.13 - 6.99 (m, 1H), 6.80 - 6.51 (m, 1H), 4.07 - 3.89 (m, 3H), 3.61 (s, 2H), 3.52 - 3.35 (m, 1H), 3.28 - 2.98 (m, 2H), 2.83 (d, *J* = 15.2 Hz, 2H), 2.67 - 2.53 (m, 1H), 2.38 (d, *J* = 18.3 Hz, 3H). LCMS (ES, m/z): 436 [M+H]⁺.

### Example 59: Preparation of [5-[6-(fluoromethyl)-3-methoxypyrazin-2-yl]-3,3a,4,6a-tetrahydrocyclopentyl[c]pyrrol-2(1H)-yl][4-methoxy-2-(2H-1,2,3-triazol-2-yl)phenyl]methanone (59)

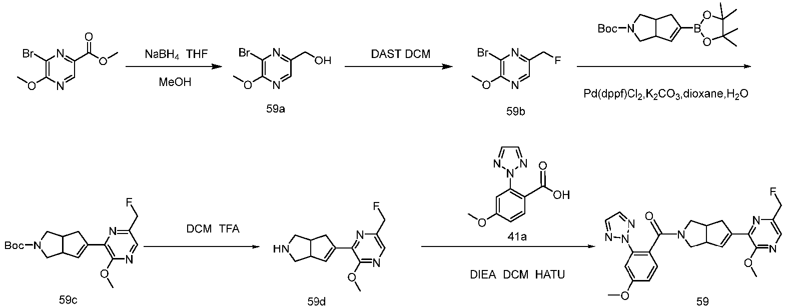

59.1 Methyl 6-bromo-5-methoxypicolinate (1.0 g, 4.06 mmol) was dissolved in 50 mL of tetrahydrofuran, and sodium borohydride (0.9 g, 23.79 mmol) was added. After the addition was completed, the temperature was raised to reflux, and 8 mL of methanol was added. The reaction was continued for 2 hours. After the reaction was completed, a saturated ammonium chloride solution was added to quench the reaction. The mixture was extracted with ethyl acetate, the organic phases were combined, dried over anhydrous sodium sulfate, filtered, and the filtrate was concentrated to obtain 800 mg of (6-bromo-5-methoxypyrazin-2-yl)methanol (Compound 59a). The crude product was directly used for the next step without purification.

59.2 Compound 59a (800 mg, 3.67 mmol) was dissolved in 10 mL of dichloromethane solution, cooled in an ice bath, and diethylaminosulfur trifluoride (0.78 g, 7.34 mmol) dissolved in 5 mL of dichloromethane was slowly added dropwise under nitrogen protection. After the addition was completed, the reaction was continued in an ice bath for 30 minutes. After the reaction was completed, a saturated sodium bicarbonate solution was added to quench the reaction, and the mixture was extracted with ethyl acetate. The organic phase was dried over anhydrous sodium sulfate, and the solvent was evaporated under reduced pressure. Column chromatography for separation and purification (PE:EA=10:1) was used to obtain 330 mg of 3-bromo-5-(fluoromethyl)-2-methoxypyrazine (Compound 59b).

59.3 The reaction raw material 39c was replaced with 59b, and tert-butyl 5-(6-(fluoromethyl)-3-methoxypyrazin-2-yl)-3,3a,4,6a-tetrahydrocyclopenta[c]pyrrole-2(1H)-carboxylate (Compound 59c) was prepared according to the method of Example 39-39.4.

59.4 The reaction raw material 39e was replaced with 59c, and 5-[6-(fluoromethyl)-3-methoxypyrazin-2-yl]-1,2,3,3a,4,6a-hexahydrocyclopenta[c]pyrrole (Compound 59d) was prepared according to the method of Example 39-39.6.

59.5 The reaction raw material 39g was replaced with 41a, 39f was replaced with 59d, and [5-[6-(fluoromethyl)-3-methoxypyrazin-2-yl]-3,3a,4,6a-tetrahydrocyclopentyl[c]pyrrol-2(1H)-yl][4-methoxy-2-(2H-1,2,3-triazol-2-yl)phenyl]methanone (Compound 59) was prepared according to the method of Example 39-39.8. ¹H NMR (400 MHz, DMSO-d₆) δ 8.26 - 8.21 (m, 1H), 8.02 - 7.76 (m, 2H), 7.40 - 7.31 (m, 2H), 7.11 - 7.02 (m, 1H), 6.88 - 6.61 (m, 1H), 5.56 - 5.32 (m, 2H), 4.09 - 3.99 (m, 3H), 3.86 (d, *J* = 8.1 Hz, 3H), 3.75 - 3.61 (m, 2H), 3.49 (br d, *J* = 1.0 Hz, 1H), 3.29 - 3.17 (m, 1H), 3.13 - 2.95 (m, 1H), 2.94 - 2.80 (m, 2H), 2.68 - 2.53 (m, 1H). LCMS (ES, m/z): 451 [M+H]⁺_{∘}

### Example 60: Preparation of [5-[6-(difluoromethyl)-3-methoxypyrazin-2-yl]-3,3a,4,6a-tetrahydrocyclopentyl[c]pyrrol-2(1H )-yl][4-fluoro-2-(2H-1,2,3-triazol-2-yl)phenyl]methanone (60)

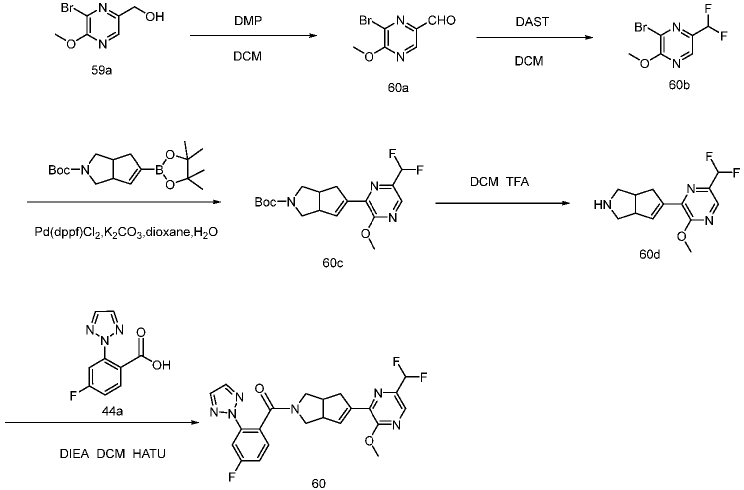

60.1 Compound 59a (0.6 g, 2.73 mmol) was dissolved in 20 mL of dichloromethane, and Dess-Martin periodinane (1.74 g, 4.11 mmol) was added. After the addition was complete, the mixture was reacted at room temperature for 2 hours. After the reaction was complete, a saturated sodium bicarbonate solution was added to quench the reaction. The mixture was extracted with dichloromethane, the organic phases were combined, dried over anhydrous sodium sulfate, filtered, the filtrate was concentrated, and separated and purified by column chromatography (PE: EA = 30:1) to obtain 285 mg of 6-bromo-5-methoxypyrazine-2-carbaldehyde (Compound 60a).

60.2 Compound 60a (285 mg, 1.31 mmol) was dissolved in 10 mL of dichloromethane solution, cooled in an ice bath, and diethylaminosulfur trifluoride (635 mg, 3.34 mmol) dissolved in 5 mL of dichloromethane was slowly added dropwise under nitrogen protection. After the addition was complete, the mixture was reacted at room temperature for 2 hours, and then the temperature was raised to reflux and the reaction was carried out overnight. After the reaction was completed, a saturated sodium bicarbonate solution was added to quench the reaction, and the mixture was extracted with ethyl acetate. The organic phase was dried over anhydrous sodium sulfate, the solvent was evaporated under reduced pressure, and column chromatography for separation and purification (PE:EA=10:1) was used to obtain 340 mg of 3-bromo-5-(difluoromethyl)-2-methoxypyrazine (Compound 60b).

60.3 The reaction raw material 39c was replaced with 60b, and tert-butyl 5-[6-(difluoromethyl)-3-methoxypyrazin-2-yl]-3,3a,4,6a-tetrahydrocyclopenta[c]pyrrole-2(1H)-carboxylate (Compound 60c) was prepared according to the method of Example 39-39.4.

60.4 The reaction raw material 39e was replaced with 60c, and 5-[6-(difluoromethyl)-3-methoxypyrazin-2-yl]-1,2,3,3 a,4,6a-hexahydrocyclopenta[c]pyrrole (Compound 60d) was prepared according to the method of Example 39-39.6.

60.5 The reaction raw material 39g was replaced with 44a, 39f was replaced with 60d, and [5-[6-(difluoromethyl)-3-methoxypyrazin-2-yl]-3,3a,4,6a-tetrahydrocyclopentyl[c]pyrrol-2(1H)-yl][ 4-fluoro-2-(2H-1,2,3-triazol-2-yl)phenyl]methanone (Compound 60) was prepared according to the method of Example 39-39.8. ¹H NMR (400 MHz, DMSO) δ 8.40 (s, 1H), 8.22 - 7.84 (m, 2H), 7.74 (dd, *J* = 15.1, 9.8 Hz, 1H), 7.51 (dd, *J* = 12.8, 6.2 Hz, 1H), 7.39 (dd, *J* = 19.8, 8.8 Hz, 1H), 7.10 (dd, *J* = 54.5, 21.9 Hz, 1H), 6.95 - 6.58 (m, 1H), 4.07 (d, *J* = 26.8 Hz, 3H), 3.90 - 3.70 (m, 1H), 3.67 (s, 1H), 3.53 (d, *J* = 1.5 Hz, 1H), 3.25 (dd, *J* = 12.2, 5.2 Hz, 1H), 3.15 (t, *J* = 10.6 Hz, 1H), 3.09 - 2.93 (m, 1H), 2.94 - 2.79 (m, 1H), 2.79 - 2.54 (m, 1H). LCMS (ES, m/z): 457 [M+H]⁺_{∘}

### Example 61: Preparation of [4-fluoro-2-(1H-pyrazol-1-yl)phenyl][5-(3-methoxy-6-methylpyrazin-2-yl)-3,3a,4,6a-tetrahydrocyclopenta[c]pyrrol-2(1H)-yl]methanone (61)

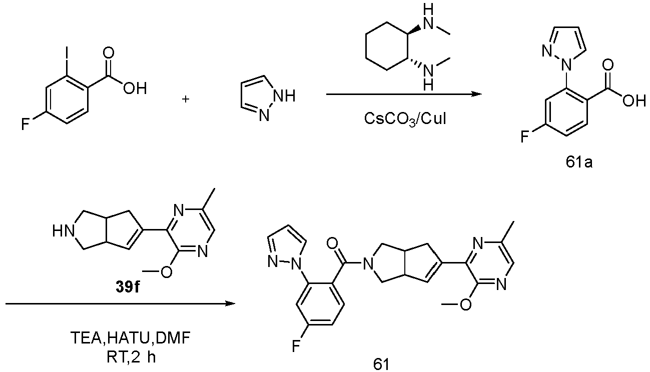

61.1 The reaction raw material 4-fluoro-6-iodobenzoic acid was replaced with 4-fluoro-2-iodobenzoic acid, and 1,2,3-triazole was replaced with 1H-pyrazole, and 4-fluoro-2-(1H-pyrazol-1-yl)benzoic acid (Compound 61a) was prepared according to the method of Example 39-39.7.

61.2 The reaction raw material 39g was replaced with 61a, and [4-fluoro-2-(1H-pyrazol-1-yl)phenyl][5-(3-methoxy-6-methylpyrazin-2-yl)-3,3a,4,6a-tetrahydrocyclopenta[c]pyrrol-2(1H)-yl]methanone (Compound 61) was prepared according to the method of Example 39-39.8. ¹H NMR (400 MHz, DMSO) δ 7.94 (d, *J* = 8.2 Hz, 1H), 7.84 (d, *J* = 4.9 Hz, 1H), 7.53 (t, *J* = 10.6 Hz, 2H), 7.45 - 7.33 (m, 1H), 7.28 (s, 1H), 6.86 (d, *J* = 28.0 Hz, 1H), 6.65 (d, *J* = 55.1 Hz, 1H), 3.96 (d, *J* = 15.8 Hz, 3H), 3.65 (dd, *J* = 18.1, 9.2 Hz, 2H), 3.26 - 3.09 (m, 2H), 3.07 - 2.89 (m, 1H), 2.87 - 2.66 (m, 2H), 2.59 (d, *J* = 16.1 Hz, 1H), 2.39 (d, *J* = 15.7 Hz, 3H). LCMS (ES, m/z): 420 [M+H]⁺_{∘}

### Example 62: Preparation of 3-[2-[4-fluoro-2-(2H-1,2,3-triazol-2-yl)benzoyl]-1,2,3,3a,4,6a-hexahydrocyclopenta[c]pyrrol-5-yl]-5-methylpyrazine-2-carbonitrile (62)

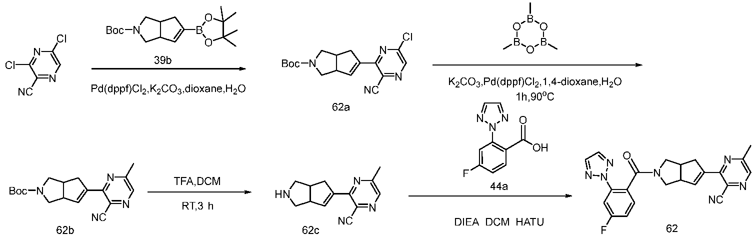

62.1 The reaction raw material 39c was replaced with 3,5-dichloropyrazine-2-carbonitrile, and tert-butyl 5-(6-chloro-3-cyanopyrazin-2-yl)-3,3a,4,6a-tetrahydrocyclopenta[c]pyrrole-2(1H)-carboxylate (Compound 62a) was prepared according to the method of Example 39-39.4.

62.2 The reaction raw material 39d was replaced with 62a, and tert-butyl 5-(3-cyano-6-methylpyrazin-2-yl)-3,3a,4,6a-tetrahydrocyclopenta[c]pyrrole-2(1H)-carboxylate (Compound 62b) was prepared according to the method of Example 39-39.5.

62.3 The reaction raw material 39d was replaced with 62b, and 3-(1,2,3a,4,6a-hexahydrocyclopenta[c]pyrrol-5-yl)-5-methylpyrazine-2-carbonitrile (Compound 62c) was prepared according to the method of Example 39-39.6.

62.4 The reaction raw material 39g was replaced with 44a, 39f was replaced with 62c, and 3-[2-[4-fluoro-2-(2H-1,2,3-triazol-2-yl)benzoyl]-1,2,3,3a,4,6a-hexahydrocyclopenta[c]pyrrol-5-yl]-5-methylpyrazine-2-carbonitrile (Compound 62) was prepared according to the method of Example 39-39.8. ¹H NMR (400 MHz, DMSO) δ 8.89 (d, *J* = 23.5 Hz, 1H), 7.98 (s, 2H), 7.73 (t, *J* = 10.8 Hz, 1H), 7.51 (s, 1H), 7.38 (dd, *J* = 16.8, 8.3 Hz, 1H), 6.85 (d, *J* = 91.0 Hz, 1H), 3.76 (t, *J* = 13.8 Hz, 1H), 3.67 (t, *J* = 12.2 Hz, 2H), 3.52 (s, 1H), 3.22 - 2.91 (m, 2H), 2.91 - 2.74 (m, 2H), 2.68 (d, *J* = 10.2 Hz, 3H). LCMS (ES, m/z): 416 [M+H]⁺_{∘}

### Example 63: Preparation of [4-methoxy-2-(2H-1,2,3-triazol-2-yl)phenyl][5-(3-methoxypyrazin-2-yl)-3,3a,4,6a-tetrahydrocyclopentyl[c]pyrrol-2(1H)-yl]methanone (63)

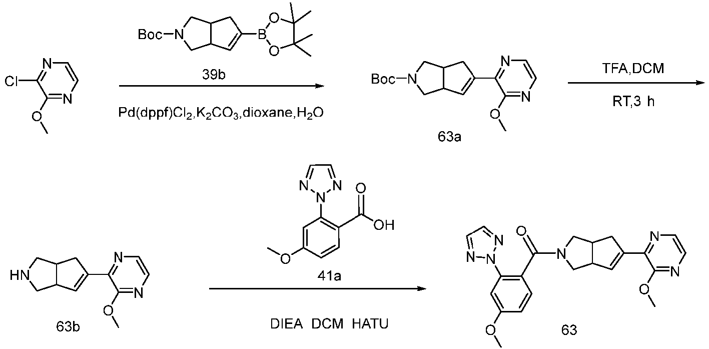

63.1 The reaction raw material 39c was replaced with 2-chloro-3-methoxypyrazine, and tert-butyl 5-(3-methoxypyrazin-2-yl)-3,3a,4,6a-tetrahydrocyclopenta[c]pyrrole-2(1H)-carboxylate (Compound 63a) was prepared according to the method of Example 39-39.4.

63.2 The reaction raw material 39d was replaced with 63a, and 5-(3-methoxypyrazin-2-yl)-1,2,3a,4,6a-hexahydrocyclopenta[c]pyrrole (Compound 63b) was prepared according to the method of Example 39-39.6.

63.3 The reaction raw material 39g was replaced with 41a, 39f was replaced with 63b, and [4-methoxy-2-(2H-1,2,3-triazol-2-yl)phenyl][5-(3-methoxypyrazin-2-yl)-3,3a,4,6a-tetrahydrocyclopentyl[c]pyrrol-2(1H)-yl]methanone (Compound 63) was prepared according to the method of Example 39-39.8. ¹H NMR (400 MHz, DMSO-d₆) δ 8.19 (d, *J* = 18.8 Hz, 1H), 8.07 (s, 1H), 8.04 - 7.68 (m, 2H), 7.41 - 7.28 (m, 2H), 7.07 (d, *J* = 10.8 Hz, 1H), 6.82 - 6.54 (m, 1H), 4.08 - 3.95 (m, 3H), 3.85 (d, *J* = 8.6 Hz, 3H), 3.76 - 3.59 (m, 2H), 3.47 (s, 1H), 3.31 - 3.04 (m, 2H), 2.84 (d, *J* = 15.2 Hz, 2H), 2.57 (d, *J* = 15.2 Hz, 1H). LCMS (ES, m/z): 419 [M+H]⁺_{∘}

### Example 64: Preparation of [5-[3-(1,1-difluoroethyl)pyrazin-2-yl]-3,3a,4,6a-tetrahydrocyclopentyl[c]pyrrol-2(1H)-yl][4-fluoro-2-(2H-1,2,3-triazol-2-yl)phenyl]methanone (64)

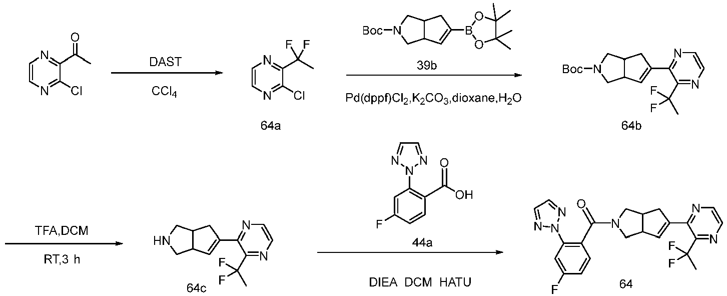

64.1 1-(3-Chloropyrazin-2-yl)ethanone (600 mg, 3.83 mmol) was dissolved in 10 mL of carbon tetrachloride, and diethylaminosulfur trifluoride (1.85 g, 11.49 mmol) was added. Under nitrogen protection, the temperature was raised to 90 °C and the reaction was carried out overnight. After the reaction was completed, the reaction solution was cooled to room temperature, then the reaction was quenched by adding saturated sodium bicarbonate solution, followed by extraction with dichloromethane. The organic phase was dried over anhydrous sodium sulfate, filtered. The filtrate was concentrated under reduced pressure, and separated and purified by column chromatography (PE:EA=5:1) to obtain 220 mg of 2-chloro-3-(1,1-difluoroethyl)pyrazine (Compound 64a).

64.2 The reaction raw material 39c was replaced with 64a, and tert-butyl 5-[3-(1,1-difluoroethyl)pyrazin-2-yl]-3,3a,4,6a-tetrahydrocyclopenta[c]pyrrole-2(1H)-carboxylate (Compound 64b) was prepared according to the method of Example 39-39.4.

64.3 The reaction raw material 39d was replaced with 64b, and 5-[3-(1,1-difluoroethyl)pyrazin-2-yl]-1,2,3,3a,4,6a-hexahydrocyclopenta[c]pyrrole (Compound 64c) was prepared according to the method of Example 39-39.6.

64.4 The reaction raw material 39g was replaced with 41a, 39f was replaced with 64c, and [5-[3-(1,1-difluoroethyl)pyrazin-2-yl]-3,3a,4,6a-tetrahydrocyclopentyl[c]pyrrol-2(1H)-yl][4-fluoro-2-(2H-1,2,3-triazol-2-yl)phenyl]methanone (Compound 64) was prepared according to the method of Example 39-39.8. ¹H NMR (400 MHz, DMSO) δ 8.80 (d, *J* = 11.3 Hz, 1H), 8.64 (d, *J* = 5.4 Hz, 1H), 8.09 (s, 2H), 7.77 (d, *J* = 9.7 Hz, 1H), 7.50 (t, *J* = 6.8 Hz, 1H), 7.42 (t, *J* = 8.1 Hz, 1H), 6.10 - 5.71 (m, 1H), 3.91 - 3.39 (m, 3H), 3.23 - 2.99 (m, 2H), 2.93 (d, *J* = 12.3 Hz, 1H), 2.77 - 2.62 (m, 1H), 2.48 - 2.29 (m, 1H), 2.07 (t, *J* = 19.3 Hz, 3H). LCMS (ES, m/z): 441 [M+H]⁺_{∘}

### Biological Test Examples

### Test Example 1: Orexin Target Function Test

### 1. Experimental purpose:

Using Cisbio HTRF IP-one kit to detect the changes in IP-one concentration of orexin receptor (OX1/OX2) signaling pathway by microplate reader, and calculating the IC₅₀ value of the compound to evaluate the antagonistic effect of the compound on OX1 and OX2 receptors.

### 2. Experimental materials:

Cell lines: CHO-K1-OX1 and CHO-K1-OX2 stable cell lines (Nanjingjinsirui Science & Technology Biology Corp.)
Cell culture conditions: F12+ 10% FBS+400 µg/ml G418
Reagents and consumables:
   F12 (Gibco, C₁1765500BT)
   FBS (Gibco, 10099-141C)
   Geneticin (G418) (Gibco, 11811031)
   PBS (meilunbio, MA0015)
   Trypsin (Gibco, 25200-072)
   Orexin A (MCE, HY-106224 )
   Orexin 2 receptor agonist (MCE, HY19320)
   96-well plate (cisbio, 66PL96025)
   IP-One-Gq kit (cisbio , 62IPAPEC)
   CO2 incubator (Thermo, 311)
   Centrifuge (Shanghai Anting, TGL-16C)
   Cell counter (Countstar, IC1000)
   Microplate reader (PerkinElmer, EnVision)

### 3. Experimental method:

(1) Preparation of the reaction buffer (1 x Stimulation buffer) required by the experiment: 5 x Stimulation buffer in Cisbio IP-one kit was diluted with ddH₂O in a ratio of 1:4 for later use.
(2) Preparation of the compound: The compound was diluted with DMSO to a 5 mM stock solution, then serially diluted 3.16-fold to generate 10 gradients, and then diluted with Stimulation buffer to the corresponding concentration (4 x) for later use.
(3) Cell preparation: CHO-K1-OX1 and CHO-K1-OX2 cells on the culture dish were digested with trypsin, washed with culture medium and collected in a 5 mL centrifuge tube. The cells were centrifuged at 1000 rpm for 5 minutes and the supernatant was discarded. 3 mL of PBS was added, and gently blown with a pipette to mix. After centrifugation at 1000 rpm for 5 minutes again, the supernatant was discarded. The cells were resuspended in 1 x Stimulation buffer, counted using a Countstar cell counter, and the cell density was adjusted to 1.71 x 10⁶ cells/mL for later use.
(4) Cell addition: The cell suspension was added to the assay plate, 7 µL/ well (i.e., about 12,000 cells/well).
(5) Compound addition: The diluted compounds with Stimulation buffer were added to the above assay plate, 3.5 µL/well.
(6) Reaction incubation: gentle shaking, the assay plate was incubated at 37°C for 30 minutes.
(7) Agonist addition of EC₈₀: 4× EC₈₀ Orexin A (OX1 receptor) agonist solution or 4× EC₈₀ Orexin 2 (OX1 receptor) agonist solution was added to the assay plate at 3.5 µL/well.
(8) Reaction incubation: After gentle shaking, the assay plate was incubated at 37°C for 30 minutes.
(9) Adding detection reagent: IP1-d2 and Anti-IP1 cryptate were diluted 1:20 with Lysis & detection buffer in Cisbio IP-one detection kit, and the diluted IP1-d2 and Anti-IP1 cryptate were added to the assay plate, 3 µL/well each. After shaking, the assay plate was left to stand at room temperature for 60 minutes.
(10) Experimental reading: The plate was read on the Envision, the readings at 665 nm and 615 nm channels were detected, and the ratio of 665 nm/615 nm readings was calculated.

### 4. Data analysis:

Based on the antagonistic effect values of the compound samples at different concentrations, the GraphPad Prism software was used to fit the orexin receptor antagonism curve of the compound samples and calculate the IC₅₀.

**Table 1**

| Compound Name | hOXIR IC₅₀ (nM) | hOX2R IC₅₀ (nM) | Selectivity |
|---|---|---|---|
| Seltorexant | 1102 | 26 | 42 |
| Compound 1 | 1131 | 14 | 80 |
| Compound 2 | 2300 | 18 | 128 |
| Compound 3 | 3920 | 23.2 | 169 |
| Compound 5 | 2800 | 16 | 171 |
| Compound 7 | 1650 | 12.3 | 134 |
| Compound 8 | 1243 | 12 | 100 |
| Compound 11 | 1950 | 27 | 72 |
| Compound 14 | 2750 | 24 | 114 |
| Compound 16 | 1880 | 20 | 94 |
| Compound 17 | 2880 | 22 | 131 |
| Compound 18 | 2310 | 23.4 | 99 |
| Compound 19 | 2480 | 18 | 138 |
| Compound 20 | 3170 | 24.5 | 129 |
| Compound 22 | 2780 | 15 | 185 |
| Compound 23 | 1960 | 14 | 140 |
| Compound 24 | 1600 | 21 | 76 |
| Compound 26 | 1140 | 17 | 67 |
| Compound 27 | 1680 | 20 | 84 |
| Compound 28 | 2310 | 19 | 122 |
| Compound 29 | 1280 | 20 | 64 |
| Compound 30 | 3920 | 21 | 186 |
| Compound 31 | 2830 | 18 | 157 |
| Compound 32 | 2460 | 20 | 123 |
| Compound 34 | 5000 | 24 | 208 |
| Compound 36 | 3100 | 18 | 172 |
| Compound 38 | 1800 | 22 | 82 |
| Compound 39 | 1200 | 20 | 60 |
| Compound 40 | 3600 | 30 | 120 |
| Compound 41 | 1653 | 19 | 87 |
| Compound 42 | 1300 | 24 | 54 |
| Compound 43 | 2100 | 28 | 75 |
| Compound 44 | 2400 | 18 | 133 |
| Compound 45 | 3000 | 25 | 125 |
| Compound 48 | 3250 | 21 | 154 |
| Compound 50 | 3610 | 27 | 134 |
| Compound 53 | 2860 | 32 | 89 |
| Compound 54 | 2600 | 22 | 118 |
| Compound 55 | 1900 | 28 | 67 |
| Compound 56 | 2300 | 16 | 144 |
| Compound 57 | 3160 | 18 | 176 |
| Compound 58 | 1980 | 24 | 83 |
| Compound 59 | 1800 | 17 | 106 |
| Compound 61 | 2100 | 14 | 150 |
| Compound 62 | 1650 | 18 | 92 |

The compound of the present application has good inhibitory activity on OX2 receptors, and the inhibitory effect of the compound on OX2 receptors is significantly better than that on OX1 receptors, with good selectivity.

### Test Example 2: Determination of pharmacokinetic parameters of the test substance in rat plasma

Healthy male SD rats aged 6-9 weeks were selected and randomly divided into two groups, with 3 rats in each group. One group was administered with the test compound at 1 mg/kg by intravenous injection, and the other group was administered with the test compound at 30 mg/kg by gavage. Whole blood was collected from the animals in the intravenous group and the gavage group before and 0.083, 0.25, 0.5, 1.0, 2.0, 4.0, 7.0, 10.0, and 24.0 hours after administration, and plasma samples were obtained by centrifugation.

LC-MS/MS method was used for quantitative analysis of all biological samples, and WinNonlinTMVersion7.0 (Pharsight, Mountain View, CA) pharmacokinetic software was used to calculate relevant pharmacokinetic parameters by non-compartmental model linear logarithmic trapezoidal method. AUC₀₋ₗₐₛₜ represents the area under the plasma concentration-time curve from zero time point to the last detectable concentration time point; PO represents oral; iv represents intravenous, Cₘₐₓ represents peak concentration, and F% represents oral bioavailability.

**Table 2**

| | PK parameters | | | | |
|---|---|---|---|---|---|
| | iv 1mg/kg | | PO 30mg/kg | | |
| | Cₘₐₓ(ng/mL) | AUC₀₋ₗₐₛₜ(h*ng/mL) | Cₘₐₓ(ng/mL) | AUC₀₋ₗₐₛₜ(h*ng/mL) | F (%) |
| Seltorexant | 1184 | 479 | 3173 | 3855 | 26.8 |
| Compound 2 | 1882 | 869 | 7482 | 15022 | 57.6 |
| Compound 5 | 1596 | 738 | 13778 | 18519 | 83.6 |
| Compound 8 | 3224.4 | 807.5 | 13849.1 | 16919.5 | 69.8 |
| Compound 12 | 3956.3 | 5305.8 | 60338.3 | 156391.3 | 98.3 |
| Compound 22 | 943 | 305 | 1943 | 2644 | 28.9 |
| Compound 25 | 1253.9 | 751.7 | 5627.7 | 8850.4 | 39.2 |
| Compound 37 | 980.2 | 626.1 | 9243.1 | 16774.2 | 89.3 |
| Compound 41 | 706.3 | 388.8 | 3977.1 | 5597.2 | 48 |
| Compound 45 | 2515.3 | 1558.3 | 19638.2 | 36045.4 | 77.1 |
| Compound 48 | 3306.2 | 886.3 | 9411.7 | 10060.6 | 37.8 |
| Compound 61 | 1915.9 | 737.1 | 5693.3 | 5397.4 | 24.4 |

In the pharmacokinetic evaluation experiment of rats, the compounds of the examples of the present application show good bioavailability after oral administration.

### Test Example 3: Autonomous Activity of Rats

Male SD rats aged 6-9 weeks were randomly divided into groups according to the principle of weight balance, with 8 rats in each group, and blank vehicle and 10, 30 and 50 mg/kg of the test compound were administered respectively. The animals were placed in the test box immediately after administration, and the activity distance of the animals within 60 minutes was recorded and analyzed using Top Scan Version 3.0. The total activity distance of the animals in each treatment group was compared with that in the blank vehicle group to determine whether the test product had a significant effect on the autonomous activity of the animals. The experimental data were expressed by mean ± standard deviation (Mean ± SD), and SPSS 21.0 statistical software was used for one-way analysis of variance. The Dunnett's test was used for pairwise comparison, and p<0.05 was represented as *.

**Table 3**

| Group | dose (mg/kg) | Activity distance (mm) | Inhibition rate(%) |
|---|---|---|---|
| vehicle (20% β-cyclodextrin) | - | 71034.44±19676.13 | - |
| Seltorexant | 10 | 58753.65±13055.81 | 17.29 |
| | 30 | 54020.96±5166.53* | 23.95* |
| | 50 | 31529.21±7233.98* | 55.61 * |
| Compound 1 | 10 | 59434.65±1478.32 | 16.33 |
| | 30 | 54433.35±3341.26* | 23.37* |
| | 50 | 28548.56±1238.05* | 59.81* |
| Compound 2 | 10 | 52097.9±18671.68* | 26.66* |
| | 30 | 46276.83±15906.44* | 34.85 * |
| | 50 | 37866.51±7149.48* | 46.69* |
| Compound 5 | 10 | 44396.01±2185.62* | 37.50* |
| | 30 | 32526.47±1464.45* | 45.79* |
| Compound 22 | 10 | 43970.97±9243.91 | 38.10* |
| | 30 | 37396.46±6850.26 | 47.35* |
| | 50 | 33132.59±9708.72 | 56.36* |
| Compound 20 | 10 | 60610.23±1657.26 | 14.67 |
| | 30 | 53815.36±1401.21* | 24.24* |
| | 50 | 48892.70±2164.16* | 31.17* |

In the autonomous activity experiment of rats, the compound of the present application can significantly reduce the autonomous activity distance of rats, and the lowest effective dose is equivalent to or better than Seltorexant.

### Test Example 3: Determination of blood-brain barrier permeability of the test substance in rats

Twelve male SD rats were randomly divided into 4 groups, with 3 rats in each group. They were fasted for 8 hours before the test and had free access to water. The rats were intragastrically administered with 30 mg/kg of the test substance solution, and 0.3 mL of blood was collected from the jugular venous plexus of the rats before and 0.25 hours after administration. The rats were then anesthetized, the chest cavity was opened, the right atrial appendage was cut, and the blood was rapidly perfused from the back of the left ventricle. When the outflowing liquid was transparent and bloodless, the perfusion could be stopped, and the brain tissue was removed, dried with filter paper, weighed, and homogenized with an appropriate amount of physiological saline (1:4, v/v) to prepare a brain tissue homogenate. The whole blood sample was centrifuged at 4000 r/min for 10 min to separate the plasma.

The established LC-MS/MS method is used to detect the concentration of the test substance in plasma and brain tissue homogenate. The brain-to-blood ratio (Kb/p) of the test substance can be obtained by dividing the drug concentration in brain tissue by the drug concentration in plasma. The larger the Kb/p, the better the blood-brain barrier permeability of the test substance.

**Table 4**

| | Seltorexant | Compound 39 | Compound 41 | Compound 42 | Compound 44 | Compound 46 |
|---|---|---|---|---|---|---|
| Brain(ng/g) | 516.3 | 285.4 | 989.5 | 192.6 | 231.3 | 210.5 |
| Plasma(ng/mL) | 2760.2 | 1023.5 | 2565.7 | 372.3 | 235.6 | 485.3 |
| Kb/p | 0.19 | 0.28 | 0.39 | 0.52 | 0.98 | 0.43 |

Conclusion: After the compound in the example of the present application is orally administered to rats, it can well penetrate the blood-brain barrier and has a higher brain-blood ratio.

### Test Example 4: Effect of Test Substance on Sleep in SD Rats

### Experimental procedure:

1. Surgical implantation of electrodes: The animals were placed in an environment with 14 h/10 h automatic alternation of light and dark for at least 4 days (lights off at 21:00, lights on at 7:00). On the day of the experiment, the animals were anesthetized with sodium pentobarbital (i.p., 60 mg/kg). After anesthesia, the brain was fixed with a stereotaxic instrument, the surgical area of the head was prepared, the skull was drilled and the electrodes were implanted.
2. Postoperative care: After the operation, the rats were carefully placed in a clean recovery cage in a lateral position to ensure that the airway was unobstructed. Light and dark were automatically alternating (lights off at 21:00, lights on at 7:00), the temperature was constant at 20-26 °C, and the relative humidity was 40-70%. The animals were cared for 3 days after the operation, intramuscularly injected with sodium penicillin, 80000 units/rat, and the experiments were performed after at least 7 days of recovery.
3. Dosage plan and indicator monitoring: Basic electroencephalogram and electromyography recordings were performed at least 7 days after postoperative recovery, and administration was started after the basic electroencephalogram and electromyography recordings were completed. Before administration, the animals were placed in the test cage to adapt for at least 24 h, and a single administration was performed 1 hour after the lights were turned off. The electroencephalogram and electromyography were recorded 12 h after administration.
4. Test index: The wake duration of rats in each hour of 12 hours after administration. The t-test was used for analysis, and P <0.05 indicated a significant difference. The experimental results are shown in the attached figures of the specification.

The results show that in the rat sleep effect test, the example compounds of the present application can significantly reduce the total wake duration and increase the total sleep duration at a lower dose. It is suggested that the compounds in the Examples of the present application have a good promoting effect on rat sleep.

Those skilled in the art will understand that many modifications and changes of the present application can be made without departing from its spirit and scope. The specific embodiments described herein are provided only by way of example and are not meant to be limited in any way. The true scope and spirit of the present application are shown by the appended claims, and the specification and examples are only exemplary.

## Claims

1. A compound represented by formula I or a pharmaceutically acceptable salt, a stereoisomer, or a tautomer thereof, 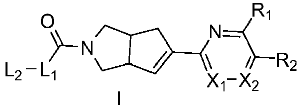 wherein
R₁ and R₂ are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, D, halogen, C₁-C₈ alkoxy, hydroxyl, nitro, and cyano;
or R₁ and R₂ together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, D, halogen, C₁-C₈ alkyl, C₁-C₈ alkoxy, hydroxyl, nitro, and cyano;
L₁ is selected from the group consisting of 6-14 membered aryl optionally substituted with one or more substituents R_{LA} and 5-14 membered heteroaryl optionally substituted with one or more substituents R_{LA}, preferably selected from the group consisting of 6-10 membered aryl optionally substituted with one or more substituents R_{LA} and 5-10 membered heteroaryl optionally substituted with one or more substituents R_{LA}, further preferably selected from the group consisting of phenyl optionally substituted with one or more substituents R_{LA} and 5-6 membered monocyclic heteroaryl optionally substituted with one or more substituents R_{LA}; the one or more substituents R_{LA} are independently selected from the group consisting of H, D, halogen, C₁-C₈ alkyl optionally substituted with one or more substituents R_{LB}, C₁-C₈ alkoxy optionally substituted with one or more substituents R_{LB}, cyano, C₂-C₈ alkynyl optionally substituted with one or more substituents R_{LB}, C₂-C₈ alkenyl optionally substituted with one or more substituents R_{LB}, hydroxyl, nitro, C₁-C₈ alkylthio optionally substituted with one or more substituents R_{LB}, C₃-C₈ cycloalkyl optionally substituted with one or more substituents R_{LB}, and OR₄, the one or more substituents R_{LB} are selected from the group consisting of H, D, halogen and hydroxyl;
R₄ is selected from the group consisting of C₃-C₈ cycloalkyl and 3-8 membered heterocyclyl, preferably C₃-C₆ cycloalkyl;
L₂ is selected from the group consisting of 6-14 membered aryl substituted with 0-4 Rb groups and 5-14 membered heteroaryl substituted with 0-4 Rb groups, preferably selected from the group consisting of 6-10 membered aryl substituted with 0-3 Rb groups and 5-10 membered monocyclic or bicyclic heteroaryl substituted with 0-3 Rb groups, further preferably selected from the group consisting of phenyl substituted with 0-3 Rb groups and 5-6 membered heteroaryl substituted with 0-3 Rb groups;
each Rb group is independently selected from the group consisting of H, D, optionally substituted C₁-C₈ alkyl, optionally substituted C₁-C₈ alkoxy, and halogen; and each substituent is independently selected from the group consisting of H, D, halogen and hydroxyl;
X₁ is selected from the group consisting of N and CR₁₀;
R₁₀ is selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, D, halogen, C₁-C₈ alkoxy, hydroxyl, nitro, and cyano, preferably selected from the group consisting of H, D, halogen and hydroxyl;
X₂ is selected from the group consisting of N and CR₁₁;
R₁₁ is selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano; and each substituent is independently selected from the group consisting of H, D, halogen, C₁-C₈ alkoxy, hydroxyl, nitro, and cyano;
and X₁ and X₂ are not N at the same time; and
when X₂ is CR₁₁ and X₁ is N, R₂ is not H or D.

2. The compound represented by formula I or a pharmaceutically acceptable salt, a stereoisomer, or a tautomer thereof according to claim 1, wherein the compound of formula I is represented by formula I-A, 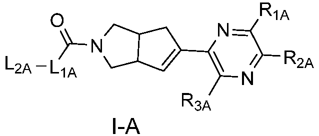
wherein R_{1A}, R_{2A}, and R_{3A} are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, halogen, C₁-C₈ alkoxy, hydroxyl, nitro, cyano, preferably selected from the group consisting of H, D, halogen and hydroxyl;
or R_{1A} and R_{2A} together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, halogen, C₁-C₈ alkyl, C₁-C₈ alkoxy, hydroxyl, nitro, and cyano;
L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; the one or more substituents are independently selected from the group consisting of H, D, halogen, optionally substituted C₁-C₈ alkyl, optionally substituted C₁-C₈ alkoxy, cyano, optionally substituted C₂-C₈ alkynyl, optionally substituted C₂-C₈ alkenyl, hydroxyl, nitro, optionally substituted C₁-C₈ alkylthio, optionally substituted C₃-C₈ cycloalkyl, and OR_{4A}, preferably independently selected from the group consisting of H, halogen, optionally substituted C₁-C₈ alkyl, optionally substituted C₁-C₈ alkoxy, hydroxyl, optionally substituted C₃-C₆ cycloalkyl, and OR_{4A}, further preferably independently selected from the group consisting of H, halogen, optionally substituted C₁-C₈ alkyl, optionally substituted C₁-C₈ alkoxy, optionally substituted C₃-C₆ cycloalkyl, and OR_{4A}, and each substituent is selected from the group consisting of H, D, halogen and hydroxyl, and the substituent is preferably selected from the group consisting of D, halogen, and hydroxyl;
R_{4A} is selected from the group consisting of C₃-C₈ cycloalkyl and 3-8 membered heterocyclyl, preferably C₃-C₆ cycloalkyl;
L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, further preferably selected from the group consisting of phenyl substituted with 0-3 Rbbb groups and 5-6 membered heteroaryl substituted with 0-3 Rbbb groups;
each Rbbb group is independently selected from the group consisting of H, D, optionally substituted C₁-C₈ alkyl, optionally substituted C₁-C₈ alkoxy, and halogen; and each substituent is independently selected from the group consisting of H, D, halogen and hydroxyl.

3. The compound represented by formula I-A or a pharmaceutically acceptable salt, a stereoisomer, or a tautomer thereof according to claim 2, wherein R_{1A}, R_{2A}, and R_{3A} are independently selected from the group consisting of H, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, halogen, C₁-C₈ alkoxy, hydroxyl, nitro, and cyano, preferably selected from the group consisting of H, halogen, and hydroxyl;
or R_{1A} and R_{2A} together with the carbon atom to which they are connected form a 3-8 membered heterocyclyl, a 5-8 membered heteroaryl, or a C₃-C₈ cycloalkyl optionally substituted with one or more substituents, and each substituent is independently selected from the group consisting of H, halogen, C₁-C₈ alkyl, C₁-C₈ alkoxy, hydroxyl, nitro, and cyano;
L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; the one or more substituents are independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}, preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₆ cycloalkyl, and OR_{4A}, further preferably independently selected from the group consisting of H, halogen, C₁-C₈ alkyl, C₁-C₈ alkoxy, C₁-C₈ haloalkyl, C₃-C₆ cycloalkyl, and OR_{4A};
R_{4A} is selected from the group consisting of C₃-C₈ cycloalkyl and 3-8 membered heterocyclyl, preferably C₃-C₆ cycloalkyl;
L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, further preferably selected from the group consisting of phenyl substituted with 0-3 Rbbb groups and 5-6 membered heteroaryl substituted with 0-3 Rbbb groups;
each Rbbb group is independently selected from the group consisting of H, optionally substituted C₁-C₈ alkyl, optionally substituted C₁-C₈ alkoxy, and halogen; and each substituent is independently selected from the group consisting of H, halogen, and hydroxyl.

4. The compound represented by formula I-A or a pharmaceutically acceptable salt, a stereoisomer, or a tautomer thereof according to claim 2 or 3, wherein it satisfies one or more of the following conditions:
(1) R_{1A}, R_{2A} and R_{3A} are independently selected from the group consisting of H, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of H, halogen, C₁-C₈ alkoxy, hydroxyl, nitro, and cyano, preferably selected from the group consisting of H, halogen, and hydroxyl; and the C₃-C₈ cycloalkyl is preferably selected from the group consisting of cyclopropyl, cyclobutyl, cyclopentyl, and cyclohexyl, more preferably cyclopropyl;
or R_{1A} and R_{2A} together with the carbon atom to which they are connected form a 3-8 membered heterocyclyl, a 5-8 membered heteroaryl, or a C₃-C₈ cycloalkyl optionally substituted with one or more substituents; the 3-8 membered heterocyclyl is preferably selected from the group consisting of tetrahydrofuryl, dihydrofuryl, tetrahydropyranyl, dihydropyranyl, tetrahydrothiopyranyl, dihydrothiopyranyl, tetrahydrothiophenyl, dihydrothiophenyl, azetidinyl, oxetanyl, thietanyl, piperidinyl and pyrrolidinyl, further preferably selected from the group consisting of tetrahydrofuryl, tetrahydropyranyl and dihydropyranyl; the 5-8 membered heteroaryl is preferably selected from the group consisting of furyl, pyranyl and thienyl; the C₃-C₈ cycloalkyl is preferably selected from the group consisting of cyclopropyl, cyclobutyl, cyclopentyl, cyclohexyl, cyclobutenyl, cyclopentenyl and cyclohexenyl, and further preferably selected from the group consisting of cyclopentyl, cyclohexyl, cyclopentenyl and cyclohexenyl; preferably, or R_{1A} and R_{2A} together with the carbon atom to which they are connected form 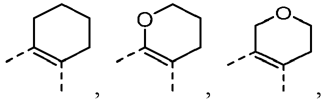 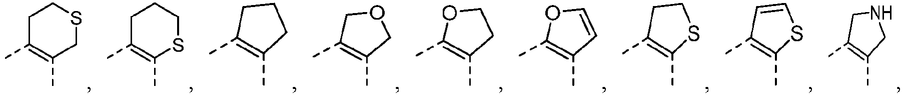 or 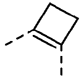 optionally substituted with one or more substituents, more preferably 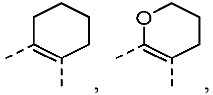 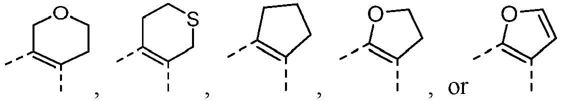 optionally substituted with one or more substituents, further preferably 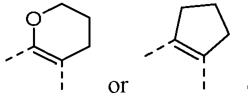 optionally substituted with one or more substituents, and the each substituent is independently selected from the group consisting of H, halogen, C₁-C₈ alkyl, C₁-C₈ alkoxy, hydroxyl, nitro, and cyano;
(2) L_{1A} is selected from the group consisting of phenyl, pyrimidinyl, pyridyl, pyrazinyl, thiazolyl, oxazolyl, imidazolyl, pyrazolyl, pyrrolyl, triazolyl, thiadiazolyl, thienyl, and furyl, preferably selected from the group consisting of phenyl, pyridyl, pyrazolyl, thiazolyl, and thienyl, further preferably phenyl, pyridyl, and thiazolyl, the above groups are optionally substituted with one or more substituents, the one or more substituents are independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}, preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₆ cycloalkyl, and OR_{4A}, further preferably independently selected from the group consisting of H, halogen, C₁-C₈ alkyl, C₁-C₈ alkoxy, C₁-C₈ haloalkyl, C₃-C₆ cycloalkyl, and OR_{4A};
R_{4A} is selected from the group consisting of C₃-C₈ cycloalkyl and 3-8 membered heterocyclyl, preferably C₃-C₆ cycloalkyl;
(3) L_{2A} is selected from the group consisting of phenyl, pyrimidinyl, pyridyl, pyrazinyl, oxazolyl, imidazolyl, pyrazolyl, pyrrolyl, 1,2,3-triazolyl, thienyl, furyl, and 1,3,4-triazolyl, preferably selected from the group consisting of phenyl, pyrimidinyl, pyridyl, pyrazolyl, thienyl, imidazolyl, and 1,2,3-triazolyl, further preferably phenyl, pyridyl, pyrazolyl, thienyl, and 1,2,3-triazolyl; wherein the above groups are optionally substituted with 0-4 Rbbb groups.

5. The compound represented by formula I-A or a pharmaceutically acceptable salt, a stereoisomer, or a tautomer thereof according to claim 2, wherein R_{1A}, R_{2A}, and R_{3A} are independently selected from the group consisting of H, D, C₁-C₈ alkyl, C₁-C₈ alkoxy, halogen, haloalkyl, deuterated C₁-C₈ alkyl, and deuterated C₁-C₈ alkoxy, preferably selected from the group consisting of H, D, C₁-C₈ alkyl, C₁-C₈ alkoxy, and deuterated C₁-C₈ alkyl;
L_{1A} is selected from the group consisting of 6-14 membered aryl optionally substituted with one or more substituents and 5-14 membered heteroaryl optionally substituted with one or more substituents, preferably selected from the group consisting of 6-10 membered aryl optionally substituted with one or more substituents and 5-10 membered heteroaryl optionally substituted with one or more substituents, further preferably selected from the group consisting of phenyl optionally substituted with one or more substituents and 5-6 membered monocyclic heteroaryl optionally substituted with one or more substituents; the one or more substituents are independently selected from the group consisting of H, D, C₁-C₈ alkyl, C₁-C₈ alkoxy, halogen, haloalkyl, deuterated C₁-C₈ alkyl, and deuterated C₁-C₈ alkoxy, preferably independently selected from the group consisting of H, D, C₁-C₈ alkyl, C₁-C₈ alkoxy, and deuterated C₁-C₈ alkyl;
L_{2A} is selected from the group consisting of 6-14 membered aryl substituted with 0-4 Rbbb groups and 5-14 membered heteroaryl substituted with 0-4 Rbbb groups, preferably selected from the group consisting of 5-10 membered aryl substituted with 0-3 Rbbb groups and 5-10 membered monocyclic or bicyclic heteroaryl substituted with 0-3 Rbbb groups, further preferably selected from the group consisting of phenyl substituted with 0-3 Rbbb groups and 5-6 membered heteroaryl substituted with 0-3 Rbbb groups;
each Rbbb group is independently selected from the group consisting of H, D, C₁-C₈ alkyl, C₁-C₈ alkoxy, halogen, haloalkyl, deuterated C₁-C₈ alkyl, and deuterated C₁-C₈ alkoxy; preferably selected from the group consisting of H, D, C₁-C₈ alkyl, C₁-C₈ alkoxy, deuterated C₁-C₈ alkyl, and deuterated C₁-C₈ alkoxy.

6. The compound represented by formula I-A or a pharmaceutically acceptable salt, a stereoisomer, or a tautomer thereof according to any one of claims 2-5, wherein the compound of formula I-A is represented by formula II-A: 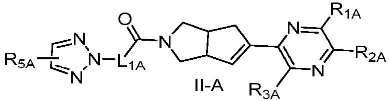 wherein R_{5A} is selected from the group consisting of H, optionally substituted C₁-C₈ alkyl, optionally substituted C₁-C₈ alkoxy, and halogen; and each substituent is independently selected from the group consisting of H, halogen, and hydroxyl.

7. The compound represented by formula I-A or a pharmaceutically acceptable salt, a stereoisomer, or a tautomer thereof according to any one of claims 2-6, wherein the compound of formula I-A is represented by formula III-A: 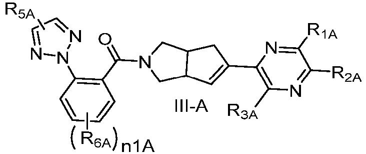
wherein R_{5A} is selected from the group consisting of H, optionally substituted C₁-C₈ alkyl, optionally substituted C₁-C₈ alkoxy, and halogen; and each substituent is independently selected from the group consisting of H, halogen, and hydroxyl;
n1A is an integer selected from 0-4, preferably 1;
R_{6A} is independently selected from the group consisting of H, halogen, C₁-C₈ alkyl, C₁-C₈ alkoxy, cyano, C₂-C₈ alkynyl, C₂-C₈ alkenyl, hydroxyl, nitro, C₁-C₈ hydroxyalkyl, C₁-C₈ hydroxyalkoxy, C₁-C₈ haloalkyl, C₁-C₈ haloalkoxy, C₁-C₈ alkylthio, C₃-C₈ cycloalkyl, and OR_{4A}; preferably independently selected from the group consisting of H, halogen, C₁-C₈ alkyl, C₁-C₈ alkoxy, hydroxyl, C₁-C₈ hydroxyalkyl, C₁-C₈ hydroxyalkoxy, C₁-C₈ haloalkyl, C₁-C₈ haloalkoxy, C₃-C₈ cycloalkyl, and OR_{4A}; further preferably independently selected from the group consisting of H, halogen, C₁-C₈ alkyl, C₁-C₈ alkoxy, C₁-C₈ haloalkyl, C₃-C₈ cycloalkyl, and OR_{4A};
R_{4A} is selected from the group consisting of C₃-C₈ cycloalkyl and 3-8 membered heterocyclyl, preferably C₃-C₆ cycloalkyl.

8. The compound represented by formula I or a pharmaceutically acceptable salt, a stereoisomer, or a tautomer thereof according to claim 1, wherein the compound of formula I is represented by formula I-B, 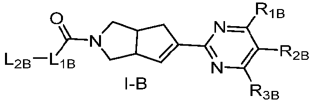
wherein R_{1B} and R_{3B} are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of halogen, D, C₁-C₈ alkoxy, hydroxyl, nitro, and cyano;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F;
or R_{1B} and R_{2B} together form a 3-8 membered heterocyclyl, a 5-8 membered heteroaryl, or a C₃-C₈ cycloalkyl optionally substituted with one or more substituents, and each substituent is independently selected from the group consisting of halogen, D, C₁-C₈ alkyl, C₁-C₈ alkoxy, hydroxyl, nitro, and cyano;
L_{1B} is selected from the group consisting of optionally substituted phenyl and optionally substituted pyridyl, each substituent is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
L_{2B} is 5-14 membered heteroaryl substituted with 0-4 Rbb groups, preferably 5-10 membered monocyclic or bicyclic heteroaryl substituted with 0-3 Rbb groups, and further preferably 5-6 membered heteroaryl substituted with 0-3 Rbb groups;
each Rbb group is independently selected from the group consisting of H, D, optionally substituted C₁-C₈ alkyl, optionally substituted C₁-C₈ alkoxy, and halogen, preferably selected from the group consisting of H and C₁-C₈ alkyl; and each substituent is independently selected from the group consisting of D and halogen.

9. The compound represented by formula I-B or a pharmaceutically acceptable salt, a stereoisomer, or a tautomer thereof according to claim 8, wherein it satisfies one or more of the following conditions:
(1) R_{1B} and R_{3B} are independently selected from the group consisting of H, D, optionally substituted C₁-C₈ alkyl, optionally substituted C₁-C₈ alkoxy, optionally substituted C₂-C₈ alkenyl, optionally substituted C₂-C₈ alkynyl, optionally substituted C₃-C₈ cycloalkyl, optionally substituted 3-8 membered heterocyclyl, halogen, hydroxyl, nitro, and cyano, preferably independently selected from the group consisting of H, optionally substituted C₁-C₈ alkyl, optionally substituted C₁-C₈ alkoxy, optionally substituted C₃-C₈ cycloalkyl, and halogen; and each substituent is independently selected from the group consisting of halogen, D, C₁-C₈ alkoxy, hydroxyl, nitro, and cyano; the 3-8 membered heterocyclyl is preferably selected from the group consisting of oxiranyl, azetidinyl, oxetanyl, thietanyl, tetrahydrofuryl, tetrahydrothiopyranyl, piperidinyl, and pyrrolidinyl; the C₃-C₈ cycloalkyl is preferably selected from the group consisting of cyclopropyl, cyclobutyl, cyclopentyl, and cyclohexyl, more preferably cyclopropyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F;
or R_{2B} and R_{1B} together form a 3-8 membered heterocyclyl, a 5-8 membered heteroaryl, or a C₃-C₈ cycloalkyl optionally substituted with one or more substituents; the 3-8 membered heterocyclyl is preferably selected from the group consisting of tetrahydrofuryl, dihydrofuryl, tetrahydropyranyl, dihydropyranyl, azetidinyl, oxetanyl, thietanyl, piperidinyl, and pyrrolidinyl, and further preferably selected from the group consisting of tetrahydrofuryl, tetrahydropyranyl, and dihydropyranyl; the 5-8 membered heteroaryl is preferably selected from the group consisting of furyl, pyranyl, and thienyl; the C₃-C₈ cycloalkyl is preferably selected from the group consisting of cyclopropyl, cyclobutyl, cyclopentyl, cyclohexyl, cyclopentenyl and cyclohexenyl, and further preferably selected from the group consisting of cyclopentyl, cyclohexyl, cyclopentenyl and cyclohexenyl; and each substituent is independently selected from the group consisting of halogen, D, C₁-C₈ alkyl, C₁-C₈ alkoxy, hydroxyl, nitro, and cyano;
(2) L_{1B} is selected from the group consisting of optionally substituted phenyl and optionally substituted pyridyl, and each substituent is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B} and C₁-C₈ alkylthio, preferably independently selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
(3) L_{2B} is selected from the group consisting of pyrimidinyl, pyridyl, pyrazinyl, thiazolyl, oxazolyl, imidazolyl, pyrazolyl, pyrrolyl, 1,2,3-triazolyl, thiadiazolyl, thienyl, furyl, and 1,3,4-triazolyl, preferably selected from the group consisting of pyrazolyl, imidazolyl, and 1,2,3-triazolyl, further preferably 1,2,3-triazolyl; wherein the above groups are optionally substituted with 0-4 Rbb groups.

10. The compound represented by formula I-B or a pharmaceutically acceptable salt, a stereoisomer, or a tautomer thereof according to any one of claims 8-9, wherein it satisfies one or more of the following conditions:
(1) L_{1B} is selected from the group consisting of 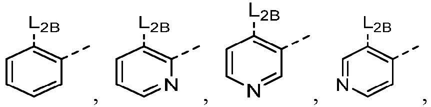 and 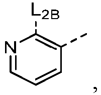 preferably selected from the group consisting of 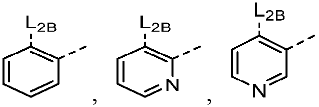 and 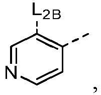 and further preferably selected from the group consisting of 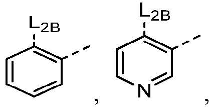 and 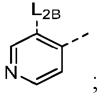 wherein the above groups are optionally substituted with one or more substituents, the one or more substituents are independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably independently selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
(2) R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

11. The compound represented by formula I-B or a pharmaceutically acceptable salt, a stereoisomer, or a tautomer thereof according to any one of claims 8-10, wherein the compound of formula I-B is represented by formula II-B: 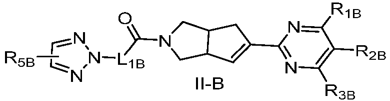
wherein R_{5B} is selected from the group consisting of H, D, optionally substituted C₁-C₈ alkyl, optionally substituted C₁-C₈ alkoxy, and halogen, and each substituent is independently selected from the group consisting of D and halogen;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

12. The compound or a pharmaceutically acceptable salt, a stereoisomer, or a tautomer thereof according to any one of claims 8-11, wherein the compound of formula I-B is represented by formula III-B: 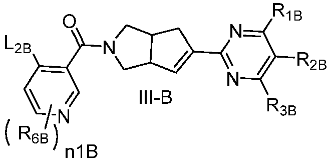
wherein n1B is an integer selected from 0-3;
R_{6B} is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

13. The compound or a pharmaceutically acceptable salt, a stereoisomer, or a tautomer thereof according to any one of claims 8-11, wherein the compound of formula I-B is represented by formula IV-B: 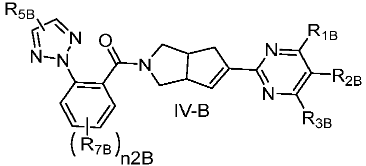
wherein R_{5B} is selected from the group consisting of H, D, optionally substituted C₁-C₈ alkyl, optionally substituted C₁-C₈ alkoxy, and halogen, and each substituent is independently selected from the group consisting of D and halogen;
n2B is an integer selected from 0-4, preferably 1;
R_{7B} is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio;
R_{4B} is selected from the group consisting of C₃-C₈ cycloalkyl and 3-8 membered heterocyclyl, preferably C₃-C₆ cycloalkyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, nitro, and cyano, preferably selected from the group consisting of C₁-C₈ alkyl, C₁-C₈ alkoxy, C₁-C₈ haloalkyl, halogen, further preferably C₁-C₈ alkyl, C₁-C₈ haloalkyl, and halogen, and even further preferably F.

14. The compound or a pharmaceutically acceptable salt, a stereoisomer, or a tautomer thereof according to any one of claims 8-12, wherein the compound of formula I-B is represented by formula V-B: 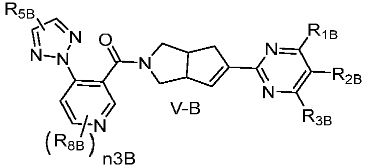
wherein R_{5B} is selected from the group consisting of H, D, optionally substituted C₁-C₈ alkyl, optionally substituted C₁-C₈ alkoxy, and halogen, and each substituent is independently selected from the group consisting of D and halogen;
n3B is an integer selected from 0-3, preferably 1,
R_{8B} is independently selected from the group consisting of H, D, C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₁-C₈ haloalkyl, C₁-C₈ haloalkoxy, hydroxyl, nitro, cyano, -OR_{4B}, and C₁-C₈ alkylthio, preferably selected from the group consisting of H, D, C₁-C₈ alkyl, C₁-C₈ alkoxy, halogen, C₁-C₈ deuterated alkyl, C₁-C₈ deuterated alkoxy, C₁-C₈ haloalkyl, -OR_{4B}, and C₁-C₈ alkylthio; R_{4B} is selected from the group consisting of C₃-C₈ cycloalkyl and 3-8 membered heterocyclyl, preferably C₃-C₆ cycloalkyl;
R_{2B} is selected from the group consisting of C₁-C₈ alkyl, C₁-C₈ alkoxy, halogen, C₁-C₈ deuterated alkyl, C₁-C₈ deuterated alkoxy, C₁-C₈ hydroxyalkyl, C₁-C₈ hydroxyalkoxy, C₂-C₈ alkenyl, C₂-C₈ alkynyl, C₁-C₈ haloalkyl, C₁-C₈ haloalkoxy, C₂-C₈ haloalkenyl, C₂-C₈ haloalkynyl, hydroxyl, and nitro, preferably selected from the group consisting of C₁-C₈ alkyl, C₁-C₈ alkoxy, C₁-C₈ haloalkyl, and halogen, further preferably selected from the group consisting of C₁-C₈ alkyl, C₁-C₈ haloalkyl, and halogen, and even further preferably F.

15. The compound or a pharmaceutically acceptable salt, a stereoisomer, or a tautomer thereof according to any one of claims 1-14, wherein it is selected from the group consisting of the following compounds:

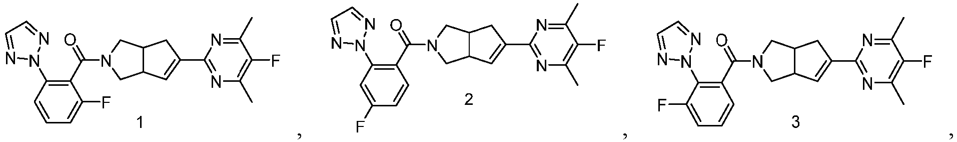

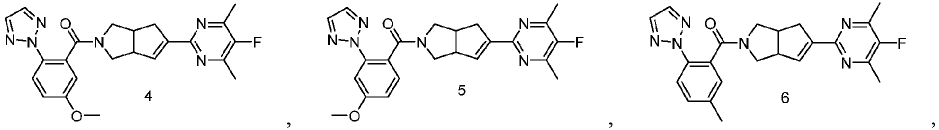

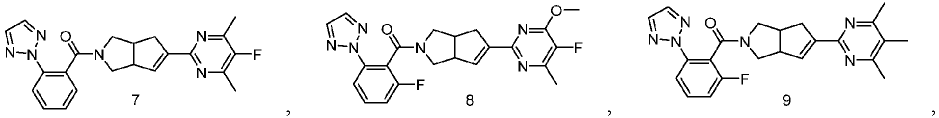

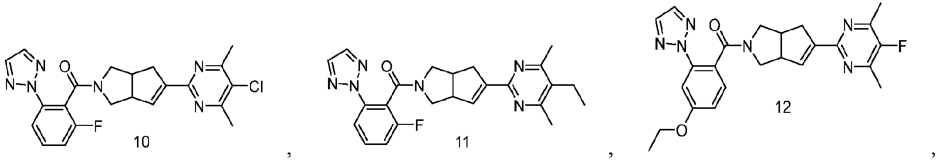

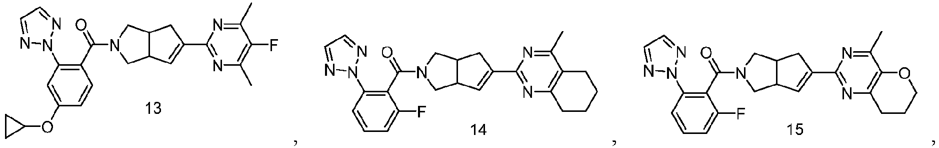

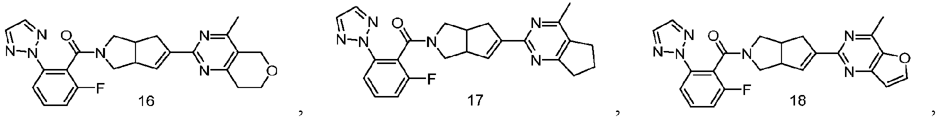

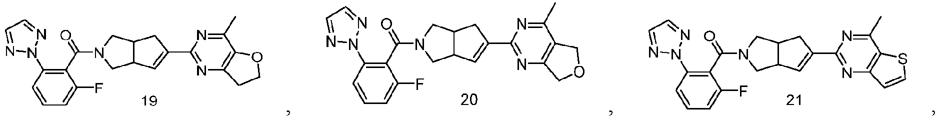

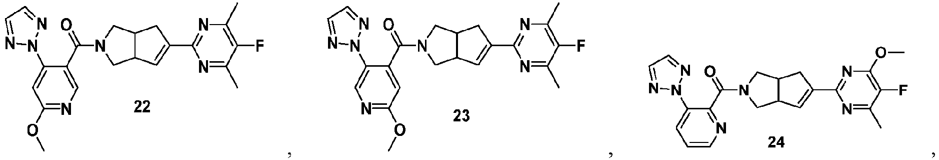

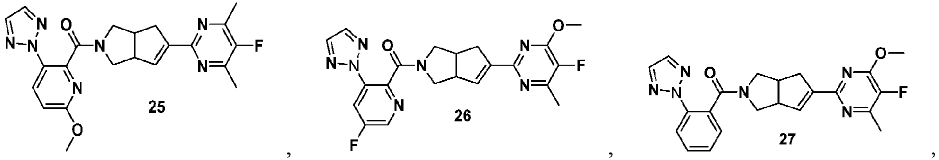

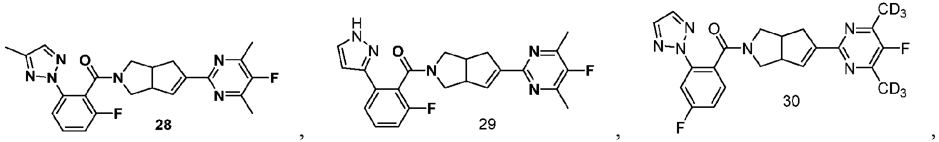

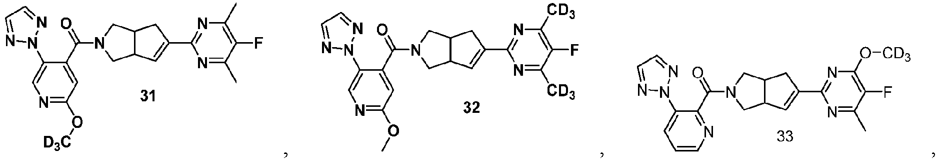

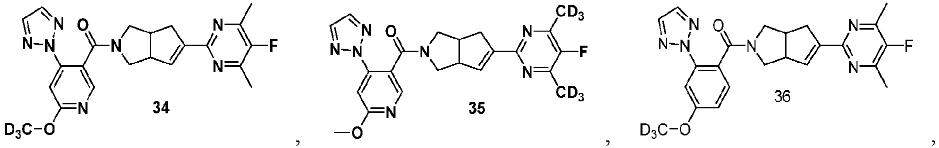

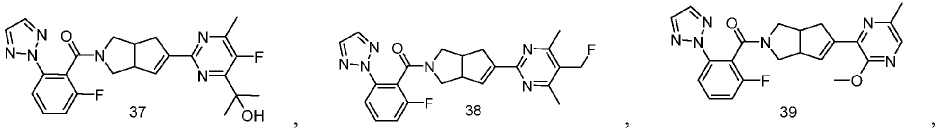

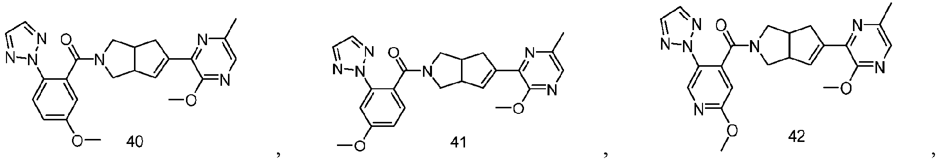

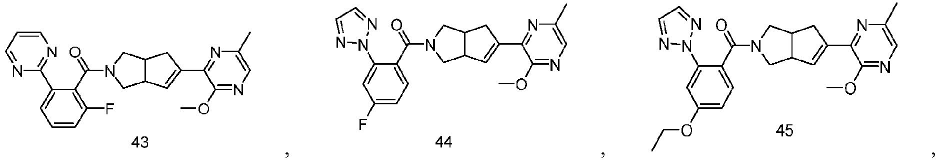

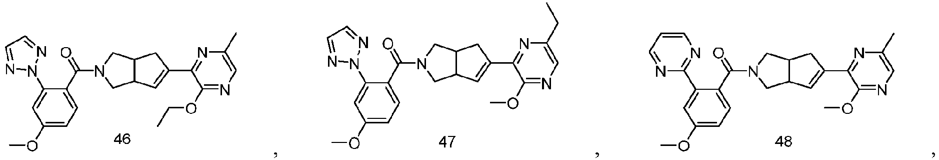

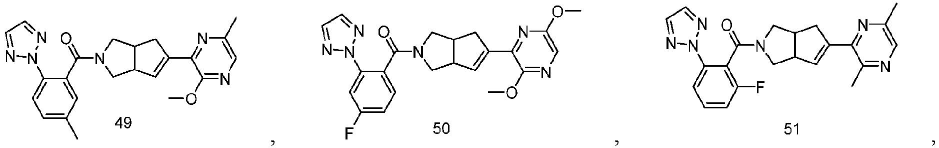

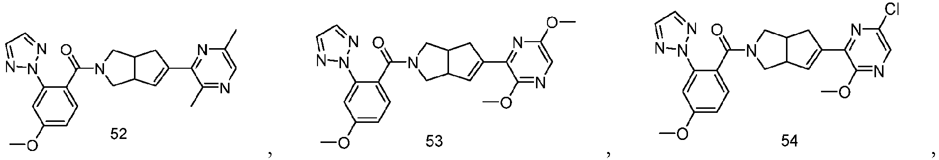

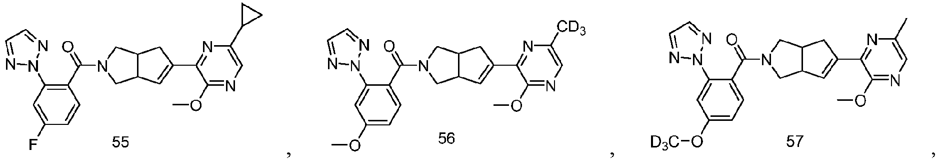

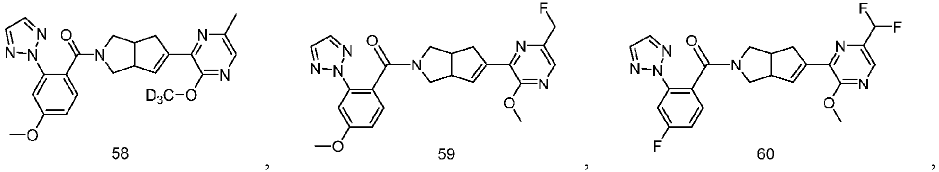

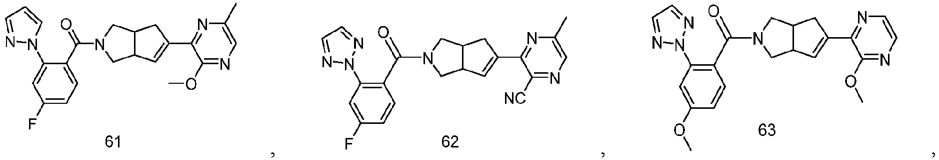

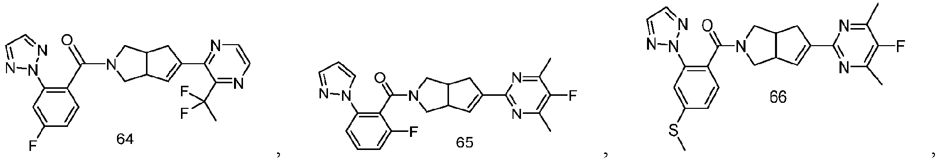

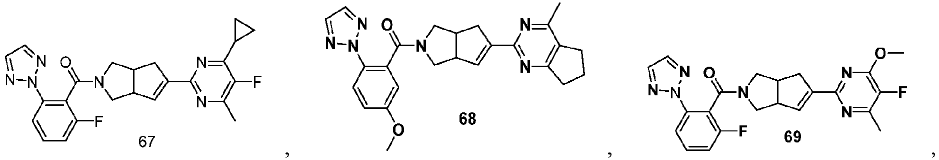

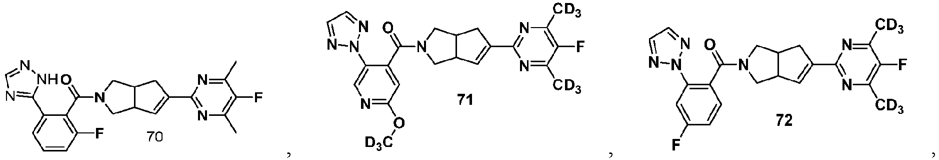

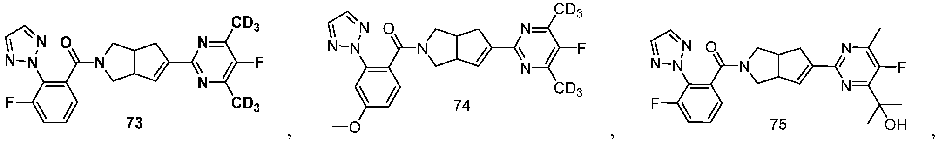

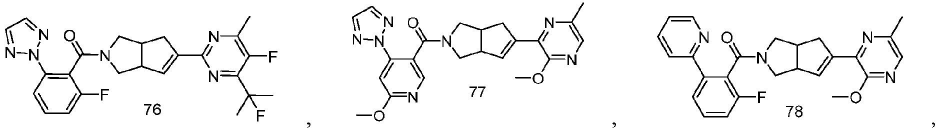

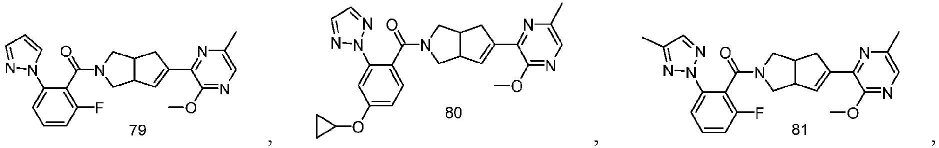

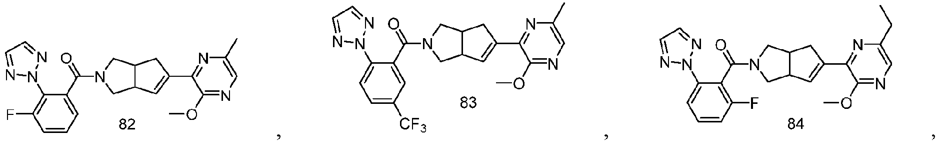

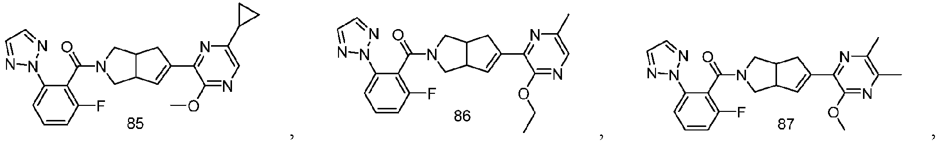

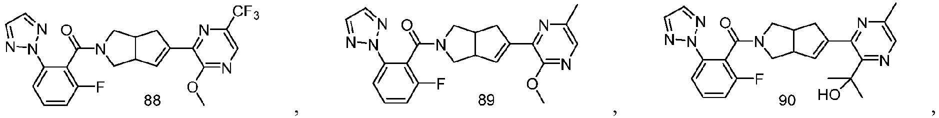

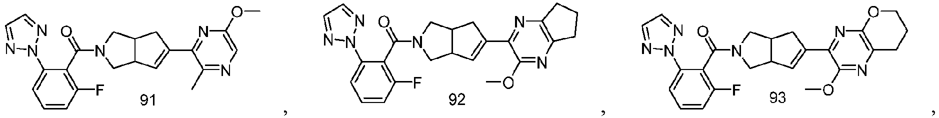

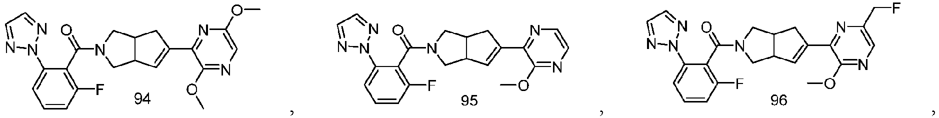

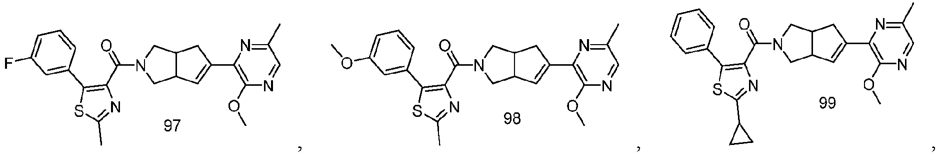

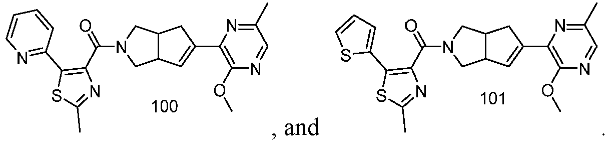

16. A pharmaceutical composition comprising a compound of formula I or a pharmaceutically acceptable salt, a stereoisomer, or a tautomer thereof according to any one of claims 1-15, and a pharmaceutically acceptable carrier.

17. Use of the compound or a pharmaceutically acceptable salt, a stereoisomer, or a tautomer thereof according to any one of claims 1-15, or the pharmaceutical composition according to claim 16 in the preparation of a medicament, wherein the medicament is used to prevent, treat, and/or alleviate a disease related to orexin receptors.

18. The use according to claim 17, wherein the disease related to orexin receptors is selected from the group consisting of: sleep disorder, anxiety disorder, panic disorder, obsessive-compulsive disorder, affective neuropathy, depressive neuropathy, anxiety neuropathy, mood disorder, panic attack disorder, behavioral disorder, emotional disturbance, post-traumatic stress disorder, psychosis, schizophrenia, manic depression, delirium, dementia, drug dependence, addiction, cognitive disorder, Parkinson's disease, movement disorder, eating disorder, headache, migraine, pain, insomnia, depression, Alzheimer's disease, and sleep apnea; preferably selected from the group consisting of: insomnia, depression, sleep disorder; further preferably selected from the group consisting of: major depressive disorder, primary and secondary insomnia or depression accompanied by insomnia.

19. A method for preparing the compound of formula I according to any one of claims 1-15, wherein it comprises the following steps: reacting a compound represented by formula I-d with a compound represented by formula I-f to obtain a target compound represented by formula I: 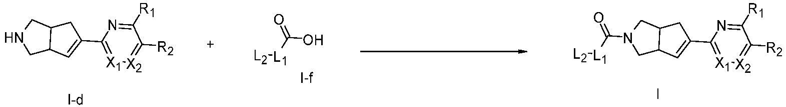 wherein R₁, R₂, X₁, X₂, L₁, and L₂ are defined as corresponding groups in any one of claims 1-15, and preferably the compound represented by formula I-d is a compound represented by formula I-dA and formula I-dB, 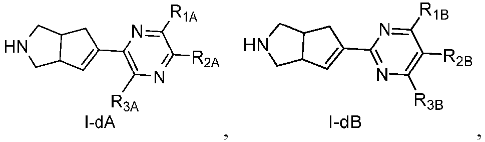 R_{1A}, R_{2A}, and R_{3A} are defined as corresponding groups in any one of claims 1-7 or claim 15; R_{1B}, R_{2B}, and R_{3B} are defined as corresponding groups in claim 1 or any one of claims 8-15.

20. A compound represented by formula I-d, 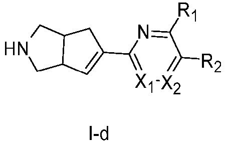 wherein R₁, R₂, X₁, and X₂ are defined as corresponding groups in any one of claims 1-15, and preferably the compound represented by formula I-d is a compound represented by formula I-dA and formula I-dB, 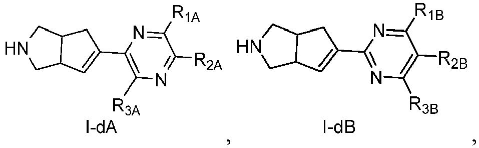 R_{1A}, R_{2A}, and R_{3A} are defined as corresponding groups in any one of claims 1-7 or claim 15; R_{1B}, R_{2B}, and R_{3B} are defined as corresponding groups in claim 1 or any one of claims 8-15.
